# EUROPEAN PATENT APPLICATION

(11) **EP 1 941 909 A1**
(43) Date of publication of application: **09.07.2008**
(21) Application number: 06822164.7
(22) Date of filing: 24.10.2006
(51) Int. Cl.: A61K 45/00, A61K 31/7105, A61K 39/395, A61P 13/08, A61P 13/10, A61P 35/00, A61P 35/02, A61P 35/04, A61P 43/00

(54) **PREVENTIVES/REMEDIES FOR CANCER**

(30) Priority: 24.10.2005 JP 2005308589; 22.02.2006 JP 2006045994
(71) Applicant: Takeda Pharmaceutical Company Limited, Osaka-shi, Osaka 541-0045 (JP)
(72) Inventor: ARAI, Toshimitsu, Osaka-shi, Osaka 5328686 (JP); TANIYAMA, Yoshio, Osaka-shi, Osaka 5328686 (JP); KOKUBO, Toshio, Osaka-shi, Osaka 5328686 (JP)
(74) Representative: Kalhammer, Georg
(86) International application number: PCT/JP2006/321184
(87) International publication number: WO 2007/049624

(57) **Abstract**

The present invention provides a drug containing a substance that inhibits a function of RET, such as an antibody against RET. In addition, the present invention provides a drug containing a substance that inhibits a function of GDNF, such as an antibody against GDNF. Moreover, the present invention provides a drug containing a substance that inhibits a function of GFRα1, such as an antibody against GFRα1. The drug of the present invention is useful as an agent for the prophylaxis or treatment of breast cancer and the like, an inhibitor of cancer cell growth and the like.

### [Sequence Listing]

SEQUENCE LISTING

<110> Takeda Pharmaceutical Company Limited

<120> PREVENTIVES/REMEDIES FOR CANCER

<130> 091005

<150> JP 2005-308589
<151> 2005-10-24

<150> JP 2006-045994
<151> 2006-02-22

<160> 32

<170> PatentIn version 3.2

<210> 1
<211> 1114
<212> PRT
<213> Homo sapiens

<400> 1

<210> 2
<211> 3342
<212> DNA
<213> Homo sapiens

<400> 2
atggcgaagg cgacgtccgg tgccgcgggg ctgcgtctgc tgttgctgct gctgctgccg 60

ctgctaggca aagtggcatt gggcctctac ttctcgaggg atgcttactg ggagaagctg 120

tatgtggacc aggcggccgg cacgcccttg ctgtacgtcc atgccctgcg ggacgcccct 180

gaggaggtgc ccagcttccg cctgggccag catctctacg gcacgtaccg cacacggctg 240

catgagaaca actggatctg catccaggag gacaccggcc tcctctacct taaccggagc 300

ctggaccata gctcctggga gaagctcagt gtccgcaacc gcggctttcc cctgctcacc 360

gtctacctca aggtcttcct gtcacccaca tcccttcgtg agggcgagtg ccagtggcca 420

ggctgtgccc gcgtatactt ctccttcttc aacacctcct ttccagcctg cagctccctc 480

aagccccggg agctctgctt cccagagaca aggccctcct tccgcattcg ggagaaccga 540

cccccaggca ccttccacca gttccgcctg ctgcctgtgc agttcttgtg ccccaacatc 600

agcgtggcct acaggctcct ggagggtgag ggtctgccct tccgctgcgc cccggacagc 660

ctggaggtga gcacgcgctg ggccctggac cgcgagcagc gggagaagta cgagctggtg 720

gccgtgtgca ccgtgcacgc cggcgcgcgc gaggaggtgg tgatggtgcc cttcccggtg 780

accgtgtacg acgaggacga ctcggcgccc accttccccg cgggcgtcga caccgccagc 840

gccgtggtgg agttcaagcg gaaggaggac accgtggtgg ccacgctgcg tgtcttcgat 900

gcagacgtgg tacctgcatc aggggagctg gtgaggcggt acacaagcac gctgctcccc 960

ggggacacct gggcccagca gaccttccgg gtggaacact ggcccaacga gacctcggtc 1020

caggccaacg gcagcttcgt gcgggcgacc gtacatgact ataggctggt tctcaaccgg 1080

aacctctcca tctcggagaa ccgcaccatg cagctggcgg tgctggtcaa tgactcagac 1140

ttccagggcc caggagcggg cgtcctcttg ctccacttca acgtgtcggt gctgccggtc 1200

agcctgcacc tgcccagtac ctactccctc tccgtgagca ggagggctcg ccgatttgcc 1260

cagatcggga aagtctgtgt ggaaaactgc caggcattca gtggcatcaa cgtccagtac 1320

aagctgcatt cctctggtgc caactgcagc acgctagggg tggtcacctc agccgaggac 1380

acctcgggga tcctgtttgt gaatgacacc aaggccctgc ggcggcccaa gtgtgccgaa 1440

cttcactaca tggtggtggc caccgaccag cagacctcta ggcaggccca ggcccagctg 1500

cttgtaacag tggaggggtc atatgtggcc gaggaggcgg gctgccccct gtcctgtgca 1560

gtcagcaaga gacggctgga gtgtgaggag tgtggcggcc tgggctcccc aacaggcagg 1620

tgtgagtgga ggcaaggaga tggcaaaggg atcaccagga acttctccac ctgctctccc 1680

agcaccaaga cctgccccga cggccactgc gatgttgtgg agacccaaga catcaacatt 1740

tgccctcagg actgcctccg gggcagcatt gttgggggac acgagcctgg ggagccccgg 1800

gggattaaag ctggctatgg cacctgcaac tgcttccctg aggaggagaa gtgcttctgc 1860

gagcccgaag acatccagga tccactgtgc gacgagctgt gccgcacggt gatcgcagcc 1920

gctgtcctct tctccttcat cgtctcggtg ctgctgtctg ccttctgcat ccactgctac 1980

cacaagtttg cccacaagcc acccatctcc tcagctgaga tgaccttccg gaggcccgcc 2040
caggccttcc cggtcagcta ctcctcttcc ggtgcccgcc ggccctcgct ggactccatg 2100
gagaaccagg tctccgtgga tgccttcaag atcctggagg atccaaagtg ggaattccct 2160
cggaagaact tggttcttgg aaaaactcta ggagaaggcg aatttggaaa agtggtcaag 2220
gcaacggcct tccatctgaa aggcagagca gggtacacca cggtggccgt gaagatgctg 2280
aaagagaacg cctccccgag tgagctgcga gacctgctgt cagagttcaa cgtcctgaag 2340
caggtcaacc acccacatgt catcaaattg tatggggcct gcagccagga tggcccgctc 2400
ctcctcatcg tggagtacgc caaatacggc tccctgcggg gcttcctccg cgagagccgc 2460
aaagtggggc ctggctacct gggcagtgga ggcagccgca actccagctc cctggaccac 2520
ccggatgagc gggccctcac catgggcgac ctcatctcat ttgcctggca gatctcacag 2580
gggatgcagt atctggccga gatgaagctc gttcatcggg acttggcagc cagaaacatc 2640
ctggtagctg aggggcggaa gatgaagatt tcggatttcg gcttgtcccg agatgtttat 2700
gaagaggatt cctacgtgaa gaggagccag ggtcggattc cagttaaatg gatggcaatt 2760
gaatcccttt ttgatcatat ctacaccacg caaagtgatg tatggtcttt tggtgtcctg 2820
ctgtgggaga tcgtgaccct agggggaaac ccctatcctg ggattcctcc tgagcggctc 2880
ttcaaccttc tgaagaccgg ccaccggatg gagaggccag acaactgcag cgaggagatg 2940
taccgcctga tgctgcaatg ctggaagcag gagccggaca aaaggccggt gtttgcggac 3000
atcagcaaag acctggagaa gatgatggtt aagaggagag actacttgga ccttgcggcg 3060
tccactccat ctgactccct gatttatgac gacggcctct cagaggagga gacaccgctg 3120
gtggactgta ataatgcccc cctccctcga gccctccctt ccacatggat tgaaaacaaa 3180
ctctatggca tgtcagaccc gaactggcct ggagagagtc ctgtaccact cacgagagct 3240
gatggcacta acactgggtt tccaagatat ccaaatgata gtgtatatgc taactggatg 3300
ctttcaccct cagcggcaaa attaatggac acgtttgata gt 3342

<210> 3
<211> 1072
<212> PRT
<213> Homo sapiens

<400> 3

Met Ala Lys Ala Thr Ser Gly Ala Ala Gly Leu Arg Leu Leu Leu Leu
1               5                   10                  15

Leu Leu Leu Pro Leu Leu Gly Lys Val Ala Leu Gly Leu Tyr Phe Ser
            20                  25                  30

Arg Asp Ala Tyr Trp Glu Lys Leu Tyr Val Asp Gln Ala Ala Gly Thr
        35                  40                  45

Pro Leu Leu Tyr Val His Ala Leu Arg Asp Ala Pro Glu Glu Val Pro
    50                  55                  60

Ser Phe Arg Leu Gly Gln His Leu Tyr Gly Thr Tyr Arg Thr Arg Leu
65                  70                  75                  80

His Glu Asn Asn Trp Ile Cys Ile Gln Glu Asp Thr Gly Leu Leu Tyr

                85                  90                  95

Leu Asn Arg Ser Leu Asp His Ser Ser Trp Glu Lys Leu Ser Val Arg
            100                 105                 110

Asn Arg Gly Phe Pro Leu Leu Thr Val Tyr Leu Lys Val Phe Leu Ser
        115                 120                 125

Pro Thr Ser Leu Arg Glu Gly Glu Cys Gln Trp Pro Gly Cys Ala Arg
    130                 135                 140

Val Tyr Phe Ser Phe Phe Asn Thr Ser Phe Pro Ala Cys Ser Ser Leu
145                 150                 155                 160

Lys Pro Arg Glu Leu Cys Phe Pro Glu Thr Arg Pro Ser Phe Arg Ile
                165                 170                 175

Arg Glu Asn Arg Pro Pro Gly Thr Phe His Gln Phe Arg Leu Leu Pro
            180                 185                 190

Val Gln Phe Leu Cys Pro Asn Ile Ser Val Ala Tyr Arg Leu Leu Glu
        195                 200                 205

Gly Glu Gly Leu Pro Phe Arg Cys Ala Pro Asp Ser Leu Glu Val Ser
    210                 215                 220

Thr Arg Trp Ala Leu Asp Arg Glu Gln Arg Glu Lys Tyr Glu Leu Val
225                 230                 235                 240

Ala Val Cys Thr Val His Ala Gly Ala Arg Glu Glu Val Val Met Val
                245                 250                 255

Pro Phe Pro Val Thr Val Tyr Asp Glu Asp Asp Ser Ala Pro Thr Phe
            260                 265                 270

Pro Ala Gly Val Asp Thr Ala Ser Ala Val Val Glu Phe Lys Arg Lys
        275                 280                 285

Glu Asp Thr Val Val Ala Thr Leu Arg Val Phe Asp Ala Asp Val Val
    290                 295                 300

Pro Ala Ser Gly Glu Leu Val Arg Arg Tyr Thr Ser Thr Leu Leu Pro
305                 310                 315                 320

Gly Asp Thr Trp Ala Gln Gln Thr Phe Arg Val Glu His Trp Pro Asn
                325                 330                 335

Glu Thr Ser Val Gln Ala Asn Gly Ser Phe Val Arg Ala Thr Val His
            340                 345                 350

Asp Tyr Arg Leu Val Leu Asn Arg Asn Leu Ser Ile Ser Glu Asn Arg
        355                 360                 365

Thr Met Gln Leu Ala Val Leu Val Asn Asp Ser Asp Phe Gln Gly Pro
    370                 375                 380

Gly Ala Gly Val Leu Leu Leu His Phe Asn Val Ser Val Leu Pro Val

385                 390                 395                 400

Ser Leu His Leu Pro Ser Thr Tyr Ser Leu Ser Val Ser Arg Arg Ala
                405                 410                 415

Arg Arg Phe Ala Gln Ile Gly Lys Val Cys Val Glu Asn Cys Gln Ala
            420                 425                 430

Phe Ser Gly Ile Asn Val Gln Tyr Lys Leu His Ser Ser Gly Ala Asn
        435                 440                 445

Cys Ser Thr Leu Gly Val Val Thr Ser Ala Glu Asp Thr Ser Gly Ile
    450                 455                 460

Leu Phe Val Asn Asp Thr Lys Ala Leu Arg Arg Pro Lys Cys Ala Glu
465                 470                 475                 480

Leu His Tyr Met Val Val Ala Thr Asp Gln Gln Thr Ser Arg Gln Ala
                485                 490                 495

Gln Ala Gln Leu Leu Val Thr Val Glu Gly Ser Tyr Val Ala Glu Glu
            500                 505                 510

Ala Gly Cys Pro Leu Ser Cys Ala Val Ser Lys Arg Arg Leu Glu Cys
        515                 520                 525

Glu Glu Cys Gly Gly Leu Gly Ser Pro Thr Gly Arg Cys Glu Trp Arg
    530                 535                 540

Gln Gly Asp Gly Lys Gly Ile Thr Arg Asn Phe Ser Thr Cys Ser Pro
545                 550                 555                 560

Ser Thr Lys Thr Cys Pro Asp Gly His Cys Asp Val Val Glu Thr Gln
                565                 570                 575

Asp Ile Asn Ile Cys Pro Gln Asp Cys Leu Arg Gly Ser Ile Val Gly
            580                 585                 590

Gly His Glu Pro Gly Glu Pro Arg Gly Ile Lys Ala Gly Tyr Gly Thr
        595                 600                 605

Cys Asn Cys Phe Pro Glu Glu Glu Lys Cys Phe Cys Glu Pro Glu Asp
    610                 615                 620

Ile Gln Asp Pro Leu Cys Asp Glu Leu Cys Arg Thr Val Ile Ala Ala
625                 630                 635                 640

Ala Val Leu Phe Ser Phe Ile Val Ser Val Leu Leu Ser Ala Phe Cys
                645                 650                 655

Ile His Cys Tyr His Lys Phe Ala His Lys Pro Pro Ile Ser Ser Ala
            660                 665                 670

Glu Met Thr Phe Arg Arg Pro Ala Gln Ala Phe Pro Val Ser Tyr Ser
        675                 680                 685

Ser Ser Gly Ala Arg Arg Pro Ser Leu Asp Ser Met Glu Asn Gln Val

690                 695                 700

Ser Val Asp Ala Phe Lys Ile Leu Glu Asp Pro Lys Trp Glu Phe Pro
705                 710                 715                 720

Arg Lys Asn Leu Val Leu Gly Lys Thr Leu Gly Glu Gly Glu Phe Gly
                725                 730                 735

Lys Val Val Lys Ala Thr Ala Phe His Leu Lys Gly Arg Ala Gly Tyr
            740                 745                 750

Thr Thr Val Ala Val Lys Met Leu Lys Glu Asn Ala Ser Pro Ser Glu
        755                 760                 765

Leu Arg Asp Leu Leu Ser Glu Phe Asn Val Leu Lys Gln Val Asn His
    770                 775                 780

Pro His Val Ile Lys Leu Tyr Gly Ala Cys Ser Gln Asp Gly Pro Leu
785                 790                 795                 800

Leu Leu Ile Val Glu Tyr Ala Lys Tyr Gly Ser Leu Arg Gly Phe Leu
                805                 810                 815

Arg Glu Ser Arg Lys Val Gly Pro Gly Tyr Leu Gly Ser Gly Gly Ser
            820                 825                 830

Arg Asn Ser Ser Ser Leu Asp His Pro Asp Glu Arg Ala Leu Thr Met
        835                 840                 845

Gly Asp Leu Ile Ser Phe Ala Trp Gln Ile Ser Gln Gly Met Gln Tyr
    850                 855                 860

Leu Ala Glu Met Lys Leu Val His Arg Asp Leu Ala Ala Arg Asn Ile
865                 870                 875                 880

Leu Val Ala Glu Gly Arg Lys Met Lys Ile Ser Asp Phe Gly Leu Ser
                885                 890                 895

Arg Asp Val Tyr Glu Glu Asp Ser Tyr Val Lys Arg Ser Gln Gly Arg
            900                 905                 910

Ile Pro Val Lys Trp Met Ala Ile Glu Ser Leu Phe Asp His Ile Tyr
        915                 920                 925

Thr Thr Gln Ser Asp Val Trp Ser Phe Gly Val Leu Leu Trp Glu Ile
    930                 935                 940

Val Thr Leu Gly Gly Asn Pro Tyr Pro Gly Ile Pro Pro Glu Arg Leu
945                 950                 955                 960

Phe Asn Leu Leu Lys Thr Gly His Arg Met Glu Arg Pro Asp Asn Cys
                965                 970                 975

Ser Glu Glu Met Tyr Arg Leu Met Leu Gln Cys Trp Lys Gln Glu Pro
            980                 985                 990

Asp Lys Arg Pro Val Phe Ala Asp Ile Ser Lys Asp Leu Glu Lys Met

995                 1000                1005

Met Val Lys Arg Arg Asp Tyr Leu Asp Leu Ala Ala Ser Thr Pro
    1010                1015                1020

Ser Asp Ser Leu Ile Tyr Asp Asp Gly Leu Ser Glu Glu Glu Thr
    1025                1030                1035

Pro Leu Val Asp Cys Asn Asn Ala Pro Leu Pro Arg Ala Leu Pro
    1040                1045                1050

Ser Thr Trp Ile Glu Asn Lys Leu Tyr Gly Arg Ile Ser His Ala
    1055                1060                1065

Phe Thr Arg Phe
    1070

<210> 4
<211> 3216
<212> DNA
<213> Homo sapiens

<400> 4
atggcgaagg cgacgtccgg tgccgcgggg ctgcgtctgc tgttgctgct gctgctgccg      60

ctgctaggca aagtggcatt gggcctctac ttctcgaggg atgcttactg ggagaagctg     120

tatgtggacc aggcggccgg cacgcccttg ctgtacgtcc atgccctgcg ggacgcccct     180

gaggaggtgc ccagcttccg cctgggccag catctctacg gcacgtaccg cacacggctg     240

catgagaaca actggatctg catccaggag gacaccggcc tcctctacct taaccggagc     300

ctggaccata gctcctggga gaagctcagt gtccgcaacc gcggctttcc cctgctcacc     360

gtctacctca aggtcttcct gtcacccaca tcccttcgtg agggcgagtg ccagtggcca     420

ggctgtgccc gcgtatactt ctccttcttc aacacctcct ttccagcctg cagctccctc     480

aagccccggg agctctgctt cccagagaca aggccctcct tccgcattcg ggagaaccga     540

cccccaggca ccttccacca gttccgcctg ctgcctgtgc agttcttgtg ccccaacatc     600

agcgtggcct acaggctcct ggagggtgag ggtctgccct tccgctgcgc cccggacagc     660

ctggaggtga gcacgcgctg ggccctggac cgcgagcagc gggagaagta cgagctggtg     720

gccgtgtgca ccgtgcacgc cggcgcgcgc gaggaggtgg tgatggtgcc cttcccggtg     780

accgtgtacg acgaggacga ctcggcgccc accttccccg cgggcgtcga caccgccagc     840

gccgtggtgg agttcaagcg gaaggaggac accgtggtgg ccacgctgcg tgtcttcgat     900

gcagacgtgg tacctgcatc aggggagctg gtgaggcggt acacaagcac gctgctcccc     960

ggggacacct gggcccagca gaccttccgg gtggaacact ggcccaacga gacctcggtc    1020

caggccaacg gcagcttcgt gcgggcgacc gtacatgact ataggctggt tctcaaccgg    1080

aacctctcca tctcggagaa ccgcaccatg cagctggcgg tgctggtcaa tgactcagac    1140

ttccagggcc caggagcggg cgtcctcttg ctccacttca acgtgtcggt gctgccggtc    1200

agcctgcacc tgcccagtac ctactccctc tccgtgagca ggagggctcg ccgatttgcc    1260

cagatcggga aagtctgtgt ggaaaactgc caggcattca gtggcatcaa cgtccagtac    1320

aagctgcatt cctctggtgc caactgcagc acgctagggg tggtcacctc agccgaggac    1380

acctcgggga tcctgtttgt gaatgacacc aaggccctgc ggcggcccaa gtgtgccgaa    1440

cttcactaca tggtggtggc caccgaccag cagacctcta ggcaggccca ggcccagctg 1500
cttgtaacag tggaggggtc atatgtggcc gaggaggcgg gctgccccct gtcctgtgca 1560
gtcagcaaga gacggctgga gtgtgaggag tgtggcggcc tgggctcccc aacaggcagg 1620
tgtgagtgga ggcaaggaga tggcaaaggg atcaccagga acttctccac ctgctctccc 1680
agcaccaaga cctgccccga cggccactgc gatgttgtgg agacccaaga catcaacatt 1740
tgccctcagg actgcctccg gggcagcatt gttgggggac acgagcctgg ggagccccgg 1800
gggattaaag ctggctatgg cacctgcaac tgcttccctg aggaggagaa gtgcttctgc 1860
gagcccgaag acatccagga tccactgtgc gacgagctgt gccgcacggt gatcgcagcc 1920
gctgtcctct tctccttcat cgtctcggtg ctgctgtctg ccttctgcat ccactgctac 1980
cacaagtttg cccacaagcc acccatctcc tcagctgaga tgaccttccg gaggcccgcc 2040
caggccttcc cggtcagcta ctcctcttcc ggtgcccgcc ggccctcgct ggactccatg 2100
gagaaccagg tctccgtgga tgccttcaag atcctggagg atccaaagtg ggaattccct 2160
cggaagaact tggttcttgg aaaaactcta ggagaaggcg aatttggaaa agtggtcaag 2220
gcaacggcct tccatctgaa aggcagagca gggtacacca cggtggccgt gaagatgctg 2280
aaagagaacg cctccccgag tgagctgcga gacctgctgt cagagttcaa cgtcctgaag 2340
caggtcaacc acccacatgt catcaaattg tatggggcct gcagccagga tggcccgctc 2400
ctcctcatcg tggagtacgc caaatacggc tccctgcggg gcttcctccg cgagagccgc 2460
aaagtggggc ctggctacct gggcagtgga ggcagccgca actccagctc cctggaccac 2520
ccggatgagc gggccctcac catgggcgac ctcatctcat ttgcctggca gatctcacag 2580
gggatgcagt atctggccga gatgaagctc gttcatcggg acttggcagc cagaaacatc 2640
ctggtagctg aggggcggaa gatgaagatt tcggatttcg gcttgtcccg agatgtttat 2700
gaagaggatt cctacgtgaa gaggagccag ggtcggattc cagttaaatg gatggcaatt 2760
gaatcccttt ttgatcatat ctacaccacg caaagtgatg tatggtcttt tggtgtcctg 2820
ctgtgggaga tcgtgaccct agggggaaac ccctatcctg ggattcctcc tgagcggctc 2880
ttcaaccttc tgaagaccgg ccaccggatg gagaggccag acaactgcag cgaggagatg 2940
taccgcctga tgctgcaatg ctggaagcag gagccggaca aaaggccggt gtttgcggac 3000
atcagcaaag acctggagaa gatgatggtt aagaggagag actacttgga ccttgcggcg 3060
tccactccat ctgactccct gatttatgac gacggcctct cagaggagga gacaccgctg 3120
gtggactgta ataatgcccc cctccctcga gccctccctt ccacatggat tgaaaacaaa 3180
ctctatggta gaatttccca tgcatttact agattc 3216

<210> 5
<211> 211
<212> PRT
<213> Homo sapiens

<400> 5

Met Lys Leu Trp Asp Val Val Ala Val Cys Leu Val Leu Leu His Thr
1               5                   10                  15

Ala Ser Ala Phe Pro Leu Pro Ala Gly Lys Arg Pro Pro Glu Ala Pro
            20                  25                  30

Ala Glu Asp Arg Ser Leu Gly Arg Arg Arg Ala Pro Phe Ala Leu Ser
        35                  40                  45

Ser Asp Ser Asn Met Pro Glu Asp Tyr Pro Asp Gln Phe Asp Asp Val
    50                  55                  60

Met Asp Phe Ile Gln Ala Thr Ile Lys Arg Leu Lys Arg Ser Pro Asp
65                  70                  75                  80

Lys Gln Met Ala Val Leu Pro Arg Arg Glu Arg Asn Arg Gln Ala Ala
                85                  90                  95

Ala Ala Asn Pro Glu Asn Ser Arg Gly Lys Gly Arg Arg Gly Gln Arg
            100                 105                 110

Gly Lys Asn Arg Gly Cys Val Leu Thr Ala Ile His Leu Asn Val Thr
        115                 120                 125

Asp Leu Gly Leu Gly Tyr Glu Thr Lys Glu Glu Leu Ile Phe Arg Tyr
    130                 135                 140

Cys Ser Gly Ser Cys Asp Ala Ala Glu Thr Thr Tyr Asp Lys Ile Leu
145                 150                 155                 160

Lys Asn Leu Ser Arg Asn Arg Arg Leu Val Ser Asp Lys Val Gly Gln
                165                 170                 175

Ala Cys Cys Arg Pro Ile Ala Phe Asp Asp Asp Leu Ser Phe Leu Asp
            180                 185                 190

Asp Asn Leu Val Tyr His Ile Leu Arg Lys His Ser Ala Lys Arg Cys
        195                 200                 205

Gly Cys Ile
    210

<210>  6
<211>  633
<212>  DNA
<213>  Homo sapiens

<400>  6
atgaagttat gggatgtcgt ggctgtctgc ctggtgctgc tccacaccgc gtccgccttc      60

ccgctgcccg ccggtaagag gcctcccgag gcgcccgccg aagaccgctc cctcggccgc     120

cgccgcgcgc ccttcgcgct gagcagtgac tcaaatatgc cagaggatta tcctgatcag     180

ttcgatgatg tcatggattt tattcaagcc accattaaaa gactgaaaag gtcaccagat     240

aaacaaatgg cagtgcttcc tagaagagag cggaatcggc aggctgcagc tgccaaccca     300

gagaattcca gaggaaaagg tcggagaggc cagaggggca aaaaccgggg ttgtgtctta     360

actgcaatac atttaaatgt cactgacttg ggtctgggct atgaaaccaa ggaggaactg     420

atttttaggt actgcagcgg ctcttgcgat gcagctgaga caacgtacga caaaatattg     480

aaaaacttat ccagaaatag aaggctggtg agtgacaaag tagggcaggc atgttgcaga     540

cccatcgcct ttgatgatga cctgtcgttt ttagatgata acctggttta ccatattcta     600

agaaagcatt ccgctaaaag gtgtggatgt atc                                  633

<210> 7
<211> 185
<212> PRT
<213> Homo sapiens

<400> 7

Met Lys Leu Trp Asp Val Val Ala Val Cys Leu Val Leu Leu His Thr
1               5                   10                  15

Ala Ser Ala Phe Pro Leu Pro Ala Ala Asn Met Pro Glu Asp Tyr Pro
            20                  25                  30

Asp Gln Phe Asp Asp Val Met Asp Phe Ile Gln Ala Thr Ile Lys Arg
        35                  40                  45

Leu Lys Arg Ser Pro Asp Lys Gln Met Ala Val Leu Pro Arg Arg Glu
    50                  55                  60

Arg Asn Arg Gln Ala Ala Ala Ala Asn Pro Glu Asn Ser Arg Gly Lys
65                  70                  75                  80

Gly Arg Arg Gly Gln Arg Gly Lys Asn Arg Gly Cys Val Leu Thr Ala
                85                  90                  95

Ile His Leu Asn Val Thr Asp Leu Gly Leu Gly Tyr Glu Thr Lys Glu
            100                 105                 110

Glu Leu Ile Phe Arg Tyr Cys Ser Gly Ser Cys Asp Ala Ala Glu Thr
        115                 120                 125

Thr Tyr Asp Lys Ile Leu Lys Asn Leu Ser Arg Asn Arg Arg Leu Val
    130                 135                 140

Ser Asp Lys Val Gly Gln Ala Cys Cys Arg Pro Ile Ala Phe Asp Asp
145                 150                 155                 160

Asp Leu Ser Phe Leu Asp Asp Asn Leu Val Tyr His Ile Leu Arg Lys
                165                 170                 175

His Ser Ala Lys Arg Cys Gly Cys Ile
            180                 185

<210> 8
<211> 555
<212> DNA
<213> Homo sapiens

<400> 8
atgaagttat gggatgtcgt ggctgtctgc ctggtgctgc tccacaccgc gtccgccttc      60

ccgctgcccg ccgcaaatat gccagaggat tatcctgatc agttcgatga tgtcatggat     120

tttattcaag ccaccattaa aagactgaaa aggtcaccag ataaacaaat ggcagtgctt     180

cctagaagag agcggaatcg gcaggctgca gctgccaacc cagagaattc cagaggaaaa     240

ggtcggagag gccagagggg caaaaaccgg ggttgtgtct taactgcaat acatttaaat     300

gtcactgact tgggtctggg ctatgaaacc aaggaggaac tgatttttag gtactgcagc     360

ggctcttgcg atgcagctga gacaacgtac gacaaaatat tgaaaaactt atccagaaat     420

agaaggctgg tgagtgacaa agtagggcag gcatgttgca gacccatcgc ctttgatgat     480

gacctgtcgt ttttagatga taacctggtt taccatattc taagaaagca ttccgctaaa     540

aggtgtggat gtatc                                                      555

<210>  9
<211>  133
<212>  PRT
<213>  Homo sapiens

<400>  9

Met Gly Cys Arg Gly Cys Leu Pro Gly Ala Ala Pro His Arg Val Arg
1               5                   10                  15

Leu Pro Ala Ala Asn Pro Glu Asn Ser Arg Gly Lys Gly Arg Arg Gly
            20                  25                  30

Gln Arg Gly Lys Asn Arg Gly Cys Val Leu Thr Ala Ile His Leu Asn
        35                  40                  45

Val Thr Asp Leu Gly Leu Gly Tyr Glu Thr Lys Glu Glu Leu Ile Phe
    50                  55                  60

Arg Tyr Cys Ser Gly Ser Cys Asp Ala Ala Glu Thr Thr Tyr Asp Lys
65                  70                  75                  80

Ile Leu Lys Asn Leu Ser Arg Asn Arg Arg Leu Val Ser Asp Lys Val
                85                  90                  95

Gly Gln Ala Cys Cys Arg Pro Ile Ala Phe Asp Asp Asp Leu Ser Phe
            100                 105                 110

Leu Asp Asp Asn Leu Val Tyr His Ile Leu Arg Lys His Ser Ala Lys
        115                 120                 125

Arg Cys Gly Cys Ile
    130

<210>  10
<211>  399
<212>  DNA
<213>  Homo sapiens

<400>  10
atgggatgtc gtggctgtct gcctggtgct gctccacacc gcgtccgcct tcccgctgcc      60

aacccagaga attccagagg aaaaggtcgg agaggccaga ggggcaaaaa ccggggttgt     120

gtcttaactg caatacattt aaatgtcact gacttgggtc tgggctatga aaccaaggag     180

gaactgattt ttaggtactg cagcggctct tgcgatgcag ctgagacaac gtacgacaaa     240

atattgaaaa acttatccag aaatagaagg ctggtgagtg acaaagtagg gcaggcatgt     300

tgcagaccca tcgcctttga tgatgacctg tcgtttttag atgataacct ggtttaccat     360

attctaagaa agcattccgc taaaaggtgt ggatgtatc                            399

<210>  11
<211>  465
<212>  PRT
<213>  Homo sapiens

<400> 11

Met Phe Leu Ala Thr Leu Tyr Phe Ala Leu Pro Leu Leu Asp Leu Leu
1               5                   10                  15

Leu Ser Ala Glu Val Ser Gly Gly Asp Arg Leu Asp Cys Val Lys Ala
            20                  25                  30

Ser Asp Gln Cys Leu Lys Glu Gln Ser Cys Ser Thr Lys Tyr Arg Thr
        35                  40                  45

Leu Arg Gln Cys Val Ala Gly Lys Glu Thr Asn Phe Ser Leu Ala Ser
    50                  55                  60

Gly Leu Glu Ala Lys Asp Glu Cys Arg Ser Ala Met Glu Ala Leu Lys
65                  70                  75                  80

Gln Lys Ser Leu Tyr Asn Cys Arg Cys Lys Arg Gly Met Lys Lys Glu
                85                  90                  95

Lys Asn Cys Leu Arg Ile Tyr Trp Ser Met Tyr Gln Ser Leu Gln Gly
            100                 105                 110

Asn Asp Leu Leu Glu Asp Ser Pro Tyr Glu Pro Val Asn Ser Arg Leu
        115                 120                 125

Ser Asp Ile Phe Arg Val Val Pro Phe Ile Ser Asp Val Phe Gln Gln
    130                 135                 140

Val Glu His Ile Pro Lys Gly Asn Asn Cys Leu Asp Ala Ala Lys Ala
145                 150                 155                 160

Cys Asn Leu Asp Asp Ile Cys Lys Lys Tyr Arg Ser Ala Tyr Ile Thr
                165                 170                 175

Pro Cys Thr Thr Ser Val Ser Asn Asp Val Cys Asn Arg Arg Lys Cys
            180                 185                 190

His Lys Ala Leu Arg Gln Phe Phe Asp Lys Val Pro Ala Lys His Ser
        195                 200                 205

Tyr Gly Met Leu Phe Cys Ser Cys Arg Asp Ile Ala Cys Thr Glu Arg
    210                 215                 220

Arg Arg Gln Thr Ile Val Pro Val Cys Ser Tyr Glu Glu Arg Glu Lys
225                 230                 235                 240

Pro Asn Cys Leu Asn Leu Gln Asp Ser Cys Lys Thr Asn Tyr Ile Cys
                245                 250                 255

Arg Ser Arg Leu Ala Asp Phe Phe Thr Asn Cys Gln Pro Glu Ser Arg
            260                 265                 270

Ser Val Ser Ser Cys Leu Lys Glu Asn Tyr Ala Asp Cys Leu Leu Ala
        275                 280                 285

Tyr Ser Gly Leu Ile Gly Thr Val Met Thr Pro Asn Tyr Ile Asp Ser

290                 295                 300

Ser Ser Leu Ser Val Ala Pro Trp Cys Asp Cys Ser Asn Ser Gly Asn
305                 310                 315                 320

Asp Leu Glu Glu Cys Leu Lys Phe Leu Asn Phe Phe Lys Asp Asn Thr
                325                 330                 335

Cys Leu Lys Asn Ala Ile Gln Ala Phe Gly Asn Gly Ser Asp Val Thr
            340                 345                 350

Val Trp Gln Pro Ala Phe Pro Val Gln Thr Thr Thr Ala Thr Thr Thr
        355                 360                 365

Thr Ala Leu Arg Val Lys Asn Lys Pro Leu Gly Pro Ala Gly Ser Glu
    370                 375                 380

Asn Glu Ile Pro Thr His Val Leu Pro Pro Cys Ala Asn Leu Gln Ala
385                 390                 395                 400

Gln Lys Leu Lys Ser Asn Val Ser Gly Asn Thr His Leu Cys Ile Ser
                405                 410                 415

Asn Gly Asn Tyr Glu Lys Glu Gly Leu Gly Ala Ser Ser His Ile Thr
            420                 425                 430

Thr Lys Ser Met Ala Ala Pro Pro Ser Cys Gly Leu Ser Pro Leu Leu
        435                 440                 445

Val Leu Val Val Thr Ala Leu Ser Thr Leu Leu Ser Leu Thr Glu Thr
    450                 455                 460

Ser
465

<210>  12
<211>  1395
<212>  DNA
<213>  Homo sapiens

<400>  12
atgttcctgg cgaccctgta cttcgcgctg ccgctcttgg acttgctcct gtcggccgaa      60

gtgagcggcg gagaccgcct ggattgcgtg aaagccagtg atcagtgcct gaaggagcag     120

agctgcagca ccaagtaccg cacgctaagg cagtgcgtgg cgggcaagga gaccaacttc     180

agcctggcat ccggcctgga ggccaaggat gagtgccgca gcgccatgga ggccctgaag     240

cagaagtcgc tctacaactg ccgctgcaag cggggtatga agaaggagaa gaactgcctg     300

cgcatttact ggagcatgta ccagagcctg cagggaaatg atctgctgga ggattcccca     360

tatgaaccag ttaacagcag attgtcagat atattccggg tggtcccatt catatcagat     420

gtttttcagc aagtggagca cattcccaaa gggaacaact gcctggatgc agcgaaggcc     480

tgcaacctcg acgacatttg caagaagtac aggtcggcgt acatcacccc gtgcaccacc     540

agcgtgtcca atgatgtctg caaccgccgc aagtgccaca aggccctccg gcagttcttt     600

gacaaggtcc cggccaagca cagctacgga atgctcttct gctcctgccg ggacatcgcc     660

tgcacagagc ggaggcgaca gaccatcgtg cctgtgtgct cctatgaaga gagggagaag     720

cccaactgtt tgaatttgca ggactcctgc aagacgaatt acatctgcag atctcgcctt 780
gcggattttt ttaccaactg ccagccagag tcaaggtctg tcagcagctg tctaaaggaa 840
aactacgctg actgcctcct cgcctactcg gggcttattg gcacagtcat gacccccaac 900
tacatagact ccagtagcct cagtgtggcc ccatggtgtg actgcagcaa cagtgggaac 960
gacctagaag agtgcttgaa atttttgaat ttcttcaagg acaatacatg tcttaaaaat 1020
gcaattcaag cctttggcaa tggctccgat gtgaccgtgt ggcagccagc cttcccagta 1080
cagaccacca ctgccactac caccactgcc ctccgggtta agaacaagcc cctggggcca 1140
gcagggtctg agaatgaaat tcccactcat gttttgccac cgtgtgcaaa tttacaggca 1200
cagaagctga aatccaatgt gtcgggcaat acacacctct gtatttccaa tggtaattat 1260
gaaaaagaag gtctcggtgc ttccagccac ataaccacaa aatcaatggc tgctcctcca 1320
agctgtggtc tgagcccact gctggtcctg gtggtaaccg ctctgtccac cctattatct 1380
ttaacagaaa catca 1395

<210> 13
<211> 460
<212> PRT
<213> Homo sapiens

<400> 13

Met Phe Leu Ala Thr Leu Tyr Phe Ala Leu Pro Leu Leu Asp Leu Leu
1               5                   10                  15

Leu Ser Ala Glu Val Ser Gly Gly Asp Arg Leu Asp Cys Val Lys Ala
            20                  25                  30

Ser Asp Gln Cys Leu Lys Glu Gln Ser Cys Ser Thr Lys Tyr Arg Thr
        35                  40                  45

Leu Arg Gln Cys Val Ala Gly Lys Glu Thr Asn Phe Ser Leu Ala Ser
    50                  55                  60

Gly Leu Glu Ala Lys Asp Glu Cys Arg Ser Ala Met Glu Ala Leu Lys
65                  70                  75                  80

Gln Lys Ser Leu Tyr Asn Cys Arg Cys Lys Arg Gly Met Lys Lys Glu
                85                  90                  95

Lys Asn Cys Leu Arg Ile Tyr Trp Ser Met Tyr Gln Ser Leu Gln Gly
            100                 105                 110

Asn Asp Leu Leu Glu Asp Ser Pro Tyr Glu Pro Val Asn Ser Arg Leu
        115                 120                 125

Ser Asp Ile Phe Arg Val Val Pro Phe Ile Ser Val Glu His Ile Pro
    130                 135                 140

Lys Gly Asn Asn Cys Leu Asp Ala Ala Lys Ala Cys Asn Leu Asp Asp
145                 150                 155                 160

Ile Cys Lys Lys Tyr Arg Ser Ala Tyr Ile Thr Pro Cys Thr Thr Ser
                165                 170                 175

Val Ser Asn Asp Val Cys Asn Arg Arg Lys Cys His Lys Ala Leu Arg
            180                 185                 190

Gln Phe Phe Asp Lys Val Pro Ala Lys His Ser Tyr Gly Met Leu Phe
        195                 200                 205

Cys Ser Cys Arg Asp Ile Ala Cys Thr Glu Arg Arg Arg Gln Thr Ile
    210                 215                 220

Val Pro Val Cys Ser Tyr Glu Glu Arg Glu Lys Pro Asn Cys Leu Asn
225                 230                 235                 240

Leu Gln Asp Ser Cys Lys Thr Asn Tyr Ile Cys Arg Ser Arg Leu Ala
                245                 250                 255

Asp Phe Phe Thr Asn Cys Gln Pro Glu Ser Arg Ser Val Ser Ser Cys
            260                 265                 270

Leu Lys Glu Asn Tyr Ala Asp Cys Leu Leu Ala Tyr Ser Gly Leu Ile
        275                 280                 285

Gly Thr Val Met Thr Pro Asn Tyr Ile Asp Ser Ser Ser Leu Ser Val
    290                 295                 300

Ala Pro Trp Cys Asp Cys Ser Asn Ser Gly Asn Asp Leu Glu Glu Cys
305                 310                 315                 320

Leu Lys Phe Leu Asn Phe Phe Lys Asp Asn Thr Cys Leu Lys Asn Ala
                325                 330                 335

Ile Gln Ala Phe Gly Asn Gly Ser Asp Val Thr Val Trp Gln Pro Ala
            340                 345                 350

Phe Pro Val Gln Thr Thr Thr Ala Thr Thr Thr Thr Ala Leu Arg Val
        355                 360                 365

Lys Asn Lys Pro Leu Gly Pro Ala Gly Ser Glu Asn Glu Ile Pro Thr
    370                 375                 380

His Val Leu Pro Pro Cys Ala Asn Leu Gln Ala Gln Lys Leu Lys Ser
385                 390                 395                 400

Asn Val Ser Gly Asn Thr His Leu Cys Ile Ser Asn Gly Asn Tyr Glu
                405                 410                 415

Lys Glu Gly Leu Gly Ala Ser Ser His Ile Thr Thr Lys Ser Met Ala
            420                 425                 430

Ala Pro Pro Ser Cys Gly Leu Ser Pro Leu Leu Val Leu Val Val Thr
        435                 440                 445

Ala Leu Ser Thr Leu Leu Ser Leu Thr Glu Thr Ser
    450                 455                 460

<210>  14
<211>  1380
<212>  DNA

<213>  Homo sapiens

<400>  14
atgttcctgg cgaccctgta cttcgcgctg ccgctcttgg acttgctcct gtcggccgaa      60

gtgagcggcg gagaccgcct ggattgcgtg aaagccagtg atcagtgcct gaaggagcag     120

agctgcagca ccaagtaccg cacgctaagg cagtgcgtgg cgggcaagga gaccaacttc     180

agcctggcat ccggcctgga ggccaaggat gagtgccgca gcgccatgga ggccctgaag     240

cagaagtcgc tctacaactg ccgctgcaag cggggtatga agaaggagaa gaactgcctg     300

cgcatttact ggagcatgta ccagagcctg cagggaaatg atctgctgga ggattcccca     360

tatgaaccag ttaacagcag attgtcagat atattccggg tggtcccatt catatcagtg     420

gagcacattc ccaaagggaa caactgcctg gatgcagcga aggcctgcaa cctcgacgac     480

atttgcaaga agtacaggtc ggcgtacatc accccgtgca ccaccagcgt gtccaatgat     540

gtctgcaacc gccgcaagtg ccacaaggcc ctccggcagt tctttgacaa ggtcccggcc     600

aagcacagct acggaatgct cttctgctcc tgccgggaca tcgcctgcac agagcggagg     660

cgacagacca tcgtgcctgt gtgctcctat gaagagaggg agaagcccaa ctgtttgaat     720

ttgcaggact cctgcaagac gaattacatc tgcagatctc gccttgcgga tttttttacc     780

aactgccagc cagagtcaag gtctgtcagc agctgtctaa aggaaaacta cgctgactgc     840

ctcctcgcct actcggggct tattggcaca gtcatgaccc ccaactacat agactccagt     900

agcctcagtg tggccccatg gtgtgactgc agcaacagtg ggaacgacct agaagagtgc     960

ttgaaatttt tgaatttctt caaggacaat acatgtctta aaaatgcaat tcaagccttt    1020

ggcaatggct ccgatgtgac cgtgtggcag ccagccttcc cagtacagac caccactgcc    1080

actaccacca ctgccctccg ggttaagaac aagcccctgg ggccagcagg gtctgagaat    1140

gaaattccca ctcatgtttt gccaccgtgt gcaaatttac aggcacagaa gctgaaatcc    1200

aatgtgtcgg gcaatacaca cctctgtatt tccaatggta attatgaaaa agaaggtctc    1260

ggtgcttcca gccacataac cacaaaatca atggctgctc ctccaagctg tggtctgagc    1320

ccactgctgg tcctggtggt aaccgctctg tccaccctat tatctttaac agaaacatca    1380


<210>  15
<211>  21
<212>  DNA
<213>  Artificial

<220>
<223>  siRNA (DNA/RNA chimeric molecule)

<400>  15
gaccauagcu ccugggagat t                                                21


<210>  16
<211>  21
<212>  DNA
<213>  Artificial

<220>
<223>  siRNA (DNA/RNA chimeric molecule)

<400>  16
ucucccagga gcuaugguct t                                                21


<210>  17
<211>  21

<212>  DNA
<213>  Artificial

<220>
<223>  siRNA (DNA/RNA chimeric molecule)

<400>  17
gaacuugguu cuuggaaaat t                                                21


<210>  18
<211>  21
<212>  DNA
<213>  Artificial

<220>
<223>  siRNA (DNA/RNA chimeric molecule)

<400>  18
uuuuccaaga accaaguuct t                                                21


<210>  19
<211>  21
<212>  DNA
<213>  Artificial

<220>
<223>  siRNA (DNA/RNA chimeric molecule)

<400>  19
ccacauggau ugaaaacaat t                                                21


<210>  20
<211>  21
<212>  DNA
<213>  Artificial

<220>
<223>  siRNA (DNA/RNA chimeric molecule)

<400>  20
uuguuuucaa uccauguggt t                                                21


<210>  21
<211>  21
<212>  DNA
<213>  Artificial

<220>
<223>  siRNA (DNA/RNA chimeric molecule)

<400>  21
auccgcgcga uaguacguat t                                                21


<210>  22
<211>  21
<212>  DNA
<213>  Artificial

<220>
<223>  siRNA (DNA/RNA chimeric molecule)

<400>  22
uacguacuau cgcgcggaut t                                                21


<210>  23
<211>  21
<212>  DNA
<213>  Artificial

<220>

<223>  siRNA (DNA/RNA chimeric molecule)

<400>  23
uuacgcguag cguaauacgt t                                                21


<210>  24
<211>  21
<212>  DNA
<213>  Artificial

<220>
<223>  siRNA (DNA/RNA chimeric molecule)

<400>  24
cguauuacgc uacgcguaat t                                                21


<210>  25
<211>  21
<212>  DNA
<213>  Artificial

<220>
<223>  siRNA (DNA/RNA chimeric molecule)

<400>  25
uauucgcgcg uauagcggut t                                                21


<210>  26
<211>  21
<212>  DNA
<213>  Artificial

<220>
<223>  siRNA (DNA/RNA chimeric molecule)

<400>  26
accgcuauac gcgcgaauat t                                                21


<210>  27
<211>  21
<212>  DNA
<213>  Artificial

<220>
<223>  siRNA (DNA/RNA chimeric molecule)

<400>  27
gagcagagcu gcagcaccat t                                                21


<210>  28
<211>  21
<212>  DNA
<213>  Artificial

<220>
<223>  siRNA (DNA/RNA chimeric molecule)

<400>  28
uggugcugca gcucugcuct t                                                21


<210>  29
<211>  21
<212>  DNA
<213>  Artificial

<220>
<223>  siRNA (DNA/RNA chimeric molecule)

<400>  29
gcagcugucu aaaggaaaat t                                                21

<210>  30
<211>  21
<212>  DNA
<213>  Artificial

<220>
<223>  siRNA (DNA/RNA chimeric molecule)

<400>  30
uuuuccuuua gacagcugct t                                                21


<210>  31
<211>  21
<212>  DNA
<213>  Artificial

<220>
<223>  siRNA (DNA/RNA chimeric molecule)

<400>  31
cucagaaggc uuugggauat t                                                21


<210>  32
<211>  21
<212>  DNA
<213>  Artificial

<220>
<223>  siRNA (DNA/RNA chimeric molecule)

<400>  32
uaucccaaag ccuucugagt t                                                21

## Description

### Technical Field

The present invention relates to a substance that inhibits a function of RET, such as an antibody against RET, and a use thereof, specifically to a prophylactic/therapeutic agent or diagnostic reagent for cancers (particularly, breast cancer), a cancer cell growth inhibitor and the like.
The present invention also relates to a substance that inhibits a function of GDNF (glial cell derived neurotrophic factor), such as an antibody against GDNF, and a use thereof, specifically to a prophylactic/therapeutic agent or diagnostic reagent for cancers (particularly, breast cancer), a cancer cell growth inhibitor and the like.
The present invention still also relates to a substance that inhibits a function of GFRα1 (GDNF family receptor alpha 1), such as an antibody against GFRα1, and a use thereof, specifically to a prophylactic/therapeutic agent or diagnostic reagent for cancers (particularly, breast cancer), a cancer cell growth inhibitor and the like.

### Background Art

The RET gene was discovered from a human lymphoma-derived DNA library as a gene that transforms NIH3T3 cells in 1985. Since then, two variants of RET mRNA (Refseq Accession Nos. NM_020630 and 020975) and two isoforms of protein, i.e., isoform a (Refseq Accession No. NP_066124), which consists of 1114 amino acids, and isoform c (Refseq Accession No. NP_065681), which consists of 1072 amino acids, have been reported. RET protein is putatively a receptor tyrosine kinase having a transmembrane domain, extracellular domain containing cadherin-like domains, and an intracellular tyrosine kinase domain, though the two different isoforms have 51 and 9 specific amino acids, respectively, from the C-terminus, which are thought to be present in cells. As a ligand for RET protein, GDNF (glial cell line-derived neurotrophic factor) has been reported, and as a co-receptor, GFRα1 (GDNF family receptor α1) has been reported. Physiologically, RET protein is thought to work in the development of various nerves and the kidneys.
An activation type mutation of the RET gene has been reported to be associated with multiple endocrine neoplasia type 2; a deactivation type mutation has been reported to be associated with Hirschsprung's disease; and the RET gene has been reported to be associated with thyroid papillocarcinoma when activated by inversion or translocation (non-patent document 1). By analysis of the expression sequence tag DNA database LIFESEQ (registered trademark), the RET gene has been reported to exhibit increased gene expression in adrenal cancer, prostatic cancer, breast cancer, and connective tissue cancer compared with normal tissue (patent document 1).

The GDNF gene was discovered from a human genome library as a human ortholg of the rat gene that encodes a protein purified from a culture supernatant of the glial cell line, B49 on the basis of its ability to promote dopaine uptake in midbrain culture in 1993. Since then, three variants of human GDNF mRNA (Refseq Accession Nos. NM_000514, 199231, and 199234), and three isoforms of protein, i.e., isoform 1 (Refseq Accession No. NP_000505), which consists of 211 amino acids, isoform 2 (Refseq Accession No. NP_954701), which consists of 185 amino acids, and isoform 3 (Refseq Accession No. NP_954704), which consists of 133 amino acids, have been reported. GDNF protein is thought to be a member of the TGF-beta family, which has a shared TGF-beta (transforming growth factor-beta)-like structure, though the three different isoforms have respective specific amino acid sequences on the N-terminal side. As a receptor of the GDNF protein, GFRα1 (GDNF family receptor alpha 1) has been reported, and as a co-receptor, RET tyrosine kinase has been reported. Physiologically, GDNF protein is thought to work as a neurotrophic factor for midbrain doperminergic neurons, motoneurons, noradrenergic neurons and the like.
A mutation of the GDNF gene has been reported to be associated with congenital central hypoventilation syndrome and Hirschsprung's disease (non-patent documents 2 and 3).

The GFRα1 gene was discoverd from a human cerebral substantia nigra-derived DNA library as a human ortholog of the rat gene that encodes a protein that binds to human GDNF (glial cell line-derived neurotrophic factor) in 1996.
Since then, two variants of human GFRα1 mRNA (Refseq Accession Nos. NM_005264 and 145793), and two isoforms of protein, i.e., isoform a (Refseq Accession No. NP_005255), which consists of 465 amino acids, and isoform b (Refseq Accession No. NP_665736), which consists of 460 amino acids, have been reported. Regarding GFRα1 protein, isoform b lacks five amino acid residues compared with isoform a, but both are putatively cysteine residue-rich GPI (glycosylphosphatidylinositol) anchor type proteins. As ligands of GFRα1 protein, GDNF, Neurturin and Artemin have been reported, and as a co-receptor, RET tyrosine kinase has been reported (non-patent document 4).
A mutation of the GFRα1 gene has been reported to be associated with medullary thyroid carcinoma (non-patent document 5).
By analysis of the expression sequence tag DNA database LIFESEQ (registered trademark), the GFRα1 gene has been reported to exhibit increased gene expression in breast cancer and brain tumors compared with normal tissue (patent document 1). The GFRα1 gene has also been reported to be a member of a group of genes that exhibit increased gene expression in breast cancer (patent document 2).
Patent Reference 1: WO03/024392
Patent Reference 2: WO02/059377
Non-Patent Reference 1: Endocrinology, Vol. 145: 5448-5451, 2004
Non-Patent Reference 2: Journal of Cell Science, Vol. 116: 3855-3862, 2005
Non-Patent Reference 3: Am. J. Hum. Genet, Vol. 62: 715-717, 1998
Non-Patent Reference 4: Anatomical Science International, Vol. 80: 42-52, 2005
Non-Patent Reference 5: Oncogene, Vol. 20: 2161-70, 2001

### Disclosure of the Invention

### Problems to be Solved by the Invention

The present invention is directed to provide a safe drug that targets a molecule expressed specifically in cancer cells, and induces inhibition of the growth of the cancer cells.

### [Means for Solving the Problems]

With the aim of accomplishing the above-described objects, the present inventors diligently investigated and found that administration of GDNF (glial cell line-derived neurotrophic factor) to the human breast cancer cell line MCF7 unexpectedly increased cell growth, and that administration of siRNA against the RET gene or GFRα1 gene to the human breast cancer cell line MCF7 unexpectedly suppressed the GDNF-dependent increase in cell growth, and thought that GDNF, GDNF-activated RET protein, and GDNF-activated GFRα1 protein induced cell growth.
The present inventors have further studied based on this finding and completed the present invention.

Accordingly, the present invention provides the following.
[1] A pharmaceutical agent comprising a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[2] The pharmaceutical agent of [1], wherein the substance is
   (1) an antibody to a protein comprising the same or substantially the same amino acid sequence as that shown in SEQ ID NO: 1 or SEQ ID NO: 3, or a partial peptide thereof or a salt thereof,
   (2) a low-molecular-weight compound that inhibits the function of a protein comprising the same or substantially the same amino acid sequence as that shown in SEQ ID NO: 1 or SEQ ID NO: 3, or a partial peptide thereof or a salt thereof, or a salt thereof,
   (3) an antisense nucleic acid to a nucleic acid encoding a protein comprising the same or substantially the same amino acid sequence as that shown in SEQ ID NO: 1 or SEQ ID NO: 3, or a partial peptide thereof, or
   (4) siRNA to RNA encoding a protein comprising the same or substantially the same amino acid sequence as that shown in SEQ ID NO: 1 or SEQ ID NO: 3, or a partial peptide thereof.
[3] A pharmaceutical agent comprising an antibody to a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[4] The pharmaceutical agent of [2] or [3], wherein the antibody is a monoclonal antibody.
[5] The pharmaceutical agent of [1] - [4], which is an agent for the prophylaxis or treatment of cancer.
[6] The pharmaceutical agent of [1] - [4], which is an agent for the prophylaxis or treatment of breast cancer.
[7] The pharmaceutical agent of [1] - [4], which is a growth inhibitor of a cancer cell.
[8] The pharmaceutical agent of [1] - [4], which is a growth inhibitor of a breast cancer cell.
[9] A diagnostic reagent for breast cancer comprising an antibody to a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[10] A method for the prophylaxis or treatment of cancer, comprising administering, to a mammal, an effective amount of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[11] A method for the prophylaxis or treatment of breast cancer, comprising administering, to a mammal, an effective amount of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[12] A method for inhibiting growth of a cancer cell, comprising administering, to a mammal, an effective amount of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[13] A method for inhibiting growth of a breast cancer cell, comprising administering, to a mammal, an effective amount of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[14] The method of [10] - [13], wherein the substance is
   (1) an antibody to a protein comprising the same or substantially the same amino acid sequence as that shown in SEQ ID NO: 1 or SEQ ID NO: 3, or a partial peptide thereof or a salt thereof,
   (2) a low-molecular-weight compound that inhibits the function of a protein comprising the same or substantially the same amino acid sequence as that shown in SEQ ID NO: 1 or SEQ ID NO: 3, or a partial peptide thereof or a salt thereof, or a salt thereof,
   (3) an antisense nucleic acid to a nucleic acid encoding a protein comprising the same or substantially the same amino acid sequence as that shown in SEQ ID NO: 1 or SEQ ID NO: 3, or a partial peptide thereof, or
   (4) siRNA to RNA encoding a protein comprising the same or substantially the same amino acid sequence as that shown in SEQ ID NO: 1 or SEQ ID NO: 3, or a partial peptide thereof.
[15] The method of [10] - [13], wherein the substance is an antibody to a protein comprising the same or substantially the same amino acid sequence as that shown in SEQ ID NO: 1 or SEQ ID NO: 3, or a partial peptide thereof or a salt thereof.
[16] The method of [15], wherein the antibody is a monoclonal antibody.
[17] Use of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof, for producing an agent for the prophylaxis or treatment of cancer.
[18] Use of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof, for producing an agent for the prophylaxis or treatment of breast cancer.
[19] Use of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof, for producing a growth inhibitor of a cancer cell.
[20] Use of a substance that inhibits the function of a protein comprising the same or substantially the same amino acid sequence as that shown in SEQ ID NO: 1 (RET protein isoform a) or SEQ ID NO: 3 (RET protein isoform c), or a partial peptide thereof or a salt thereof, for producing a growth inhibitor of a breast cancer cell.
[21] The use of [17]-[20], wherein the substance is an antibody to a protein comprising the same or substantially the same amino acid sequence as that shown in SEQ ID NO: 1 (RET protein isoform a) or SEQ ID NO: 3 (RET protein isoform c), or a partial peptide thereof or a salt thereof.
[22] The use of [21], wherein the antibody is a monoclonal antibody.

[23] A pharmaceutical agent comprising a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.
[24] The pharmaceutical agent of [23], wherein the substance is
   (1) an antibody to a protein comprising the same or substantially the same amino acid sequence as that shown in SEQ ID NO: 5, SEQ ID NO: 7 or SEQ ID NO: 9, or a partial peptide thereof or a salt thereof,
   (2) a low-molecular-weight compound that inhibits the function of a protein comprising the same or substantially the same amino acid sequence as that shown in SEQ ID NO: 5, SEQ ID NO: 7 or SEQ ID NO: 9, or a partial peptide thereof or a salt thereof, or a salt thereof,
   (3) an antisense nucleic acid to a nucleic acid encoding a protein comprising the same or substantially the same amino acid sequence as that shown in SEQ ID NO: 5, SEQ ID NO: 7 or SEQ ID NO: 9, or a partial peptide thereof, or
   (4) siRNA to RNA encoding a protein comprising the same or substantially the same amino acid sequence as that shown in SEQ ID NO: 5, SEQ ID NO: 7 or SEQ ID NO: 9, or a partial peptide thereof.
[25] A pharmaceutical agent comprising an antibody to a protein comprising the same or substantially the same amino acid sequence as that shown in SEQ ID NO: 5, SEQ ID NO: 7 or SEQ ID NO: 9, or a partial peptide thereof or a salt thereof.
[26] The pharmaceutical agent of [24] or [25], wherein the antibody is a monoclonal antibody.
[27] The pharmaceutical agent of [23] - [26], which is an agent for the prophylaxis or treatment of cancer.
[28] The pharmaceutical agent of [23] - [26], which is an agent for the prophylaxis or treatment of breast cancer.
[29] The pharmaceutical agent of [23] - [26], which is a growth inhibitor of a cancer cell.
[30] The pharmaceutical agent of [23] - [26], which is a growth inhibitor of a breast cancer cell.
[31] A diagnostic reagent of breast cancer comprising an antibody to a protein comprising the same or substantially the same amino acid sequence as that shown in SEQ ID NO: 5, SEQ ID NO: 7 or SEQ ID NO: 9, or a partial peptide thereof or a salt thereof.
[32] A diagnostic reagent of breast cancer comprising a nucleic acid encoding a protein comprising the same or substantially the same amino acid sequence as that shown in SEQ ID NO: 5, SEQ ID NO: 7 or SEQ ID NO: 9, or a partial peptide thereof.
[33] A method for the prophylaxis or treatment of cancer, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.
[34] A method for the prophylaxis or treatment of breast cancer, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.
[35] A method for inhibiting growth of a cancer cell, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.
[36] A method for inhibiting growth of a breast cancer cell, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.
[37] The method of [33] - [36], wherein the substance is
   (1) an antibody to a protein comprising the same or substantially the same amino acid sequence as that shown in SEQ ID NO: 5, SEQ ID NO: 7 or SEQ ID NO:9, or a partial peptide thereof or a salt thereof,
   (2) a low-molecular-weight compound that inhibits the function of a protein comprising the same or substantially the same amino acid sequence as that shown in SEQ ID NO: 5, SEQ ID NO: 7 or SEQ ID NO:9, or a partial peptide thereof or a salt thereof, or a salt thereof,
   (3) an antisense nucleic acid to a nucleic acid encoding a protein comprising the same or substantially the same amino acid sequence as that shown in SEQ ID NO: 5, SEQ ID NO: 7 or SEQ ID NO:9, or a partial peptide thereof, or
   (4) siRNA to RNA encoding a protein comprising the same or substantially the same amino acid sequence as that shown in SEQ ID NO: 5, SEQ ID NO: 7 or SEQ ID NO:9, or a partial peptide thereof.
[38] The method of [33] - [36], wherein the substance is an antibody to a protein comprising the same or substantially the same amino acid sequence as that shown in SEQ ID NO: 5, SEQ ID NO: 7 or SEQ ID NO: 9, or a partial peptide thereof or a salt thereof.
[39] The method of [38], wherein the antibody is a monoclonal antibody.
[40] Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing an agent for the prophylaxis or treatment of cancer.
[41] Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing an agent for the prophylaxis or treatment of breast cancer.
[42] Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing a growth inhibitor of a cancer cell.
[43] Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing a growth inhibitor of a breast cancer cell.
[44] The use of [40] - [43], wherein the substance is an antibody to a protein comprising the same or substantially the same amino acid sequence as that shown in SEQ ID NO: 5, SEQ ID NO: 7 or SEQ ID NO: 9, or a partial peptide thereof or a salt thereof.
[45] The use of [44], wherein the antibody is a monoclonal antibody.

[46] A pharmaceutical agent comprising a substance that inhibits the function of a protein comprising the same or substantially the same amino acid sequence as that shown in SEQ ID NO: 11 or SEQ ID NO: 13, or a partial peptide thereof or a salt thereof.
[47] The pharmaceutical agent of [46], wherein the substance is
   (1) an antibody to a protein comprising the same or substantially the same amino acid sequence as that shown in SEQ ID NO: 11 or SEQ ID NO: 13, or a partial peptide thereof or a salt thereof,
   (2) a low-molecular-weight compound that inhibits the function of a protein comprising the same or substantially the same amino acid sequence as that shown in SEQ ID NO: 11 or SEQ ID NO: 13, or a partial peptide thereof or a salt thereof, or a salt thereof,
   (3) an antisense nucleic acid to a nucleic acid encoding a protein comprising the same or substantially the same amino acid sequence as that shown in SEQ ID NO: 11 or SEQ ID NO: 13, or a partial peptide thereof, or
   (4) siRNA to RNA encoding a protein comprising the same or substantially the same amino acid sequence as that shown in SEQ ID NO: 11 or SEQ ID NO: 13, or a partial peptide thereof.
[48] A pharmaceutical agent comprising an antibody to a protein comprising the same or substantially the same amino acid sequence as that shown in SEQ ID NO: 11 or SEQ ID NO: 13, or a partial peptide thereof or a salt thereof.
[49] The pharmaceutical agent of [47] or [48], wherein the antibody is a monoclonal antibody.
[50] The pharmaceutical agent of [46] - [49], which is an agent for the prophylaxis or treatment of cancer.
[51] The pharmaceutical agent of [46] - [49], which is an agent for the prophylaxis or treatment of breast cancer.
[52] The pharmaceutical agent of [46] - [49], which is a growth inhibitor of a cancer cell.
[53] The pharmaceutical agent of [46] - [49], which is a growth inhibitor of a breast cancer cell.
[54] A diagnostic reagent of breast cancer comprising an antibody to a protein comprising the same or substantially the same amino acid sequence as that shown in SEQ ID NO: 11 or SEQ ID NO: 13, or a partial peptide thereof or a salt thereof.
[55] A diagnostic reagent of breast cancer comprising a nucleic acid encoding a protein comprising the same or substantially the same amino acid sequence as that shown in SEQ ID NO: 11 or SEQ ID NO: 13, or a partial peptide thereof.
[56] A method for the prophylaxis or treatment of cancer, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.
[57] A method for the prophylaxis or treatment of breast cancer, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.
[58] A method for inhibiting growth of a cancer cell, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.
[59] A method for inhibiting growth of a breast cancer cell, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.
[60] The method of [56] - [59], wherein the substance is
   (1) an antibody to a protein comprising the same or substantially the same amino acid sequence as that shown in SEQ ID NO: 11 or SEQ ID NO: 13, or a partial peptide thereof or a salt thereof,
   (2) a low-molecular-weight compound that inhibits the function of a protein comprising the same or substantially the same amino acid sequence as that shown in SEQ ID NO: 11 or SEQ ID NO: 13, or a partial peptide thereof or a salt thereof, or a salt thereof,
   (3) an antisense nucleic acid to a nucleic acid encoding a protein comprising the same or substantially the same amino acid sequence as that shown in SEQ ID NO: 11 or SEQ ID NO: 13, or a partial peptide thereof, or
   (4) siRNA to RNA encoding a protein comprising the same or substantially the same amino acid sequence as that shown in SEQ ID NO: 11 or SEQ ID NO: 13, or a partial peptide thereof.
[61] The method of [56] - [59], wherein the substance is an antibody to a protein comprising the same or substantially the same amino acid sequence as that shown in SEQ ID NO: 11 or SEQ ID NO: 13, or a partial peptide thereof or a salt thereof.
[62] The method of [61], wherein the antibody is a monoclonal antibody.
[63] Use of a substance that inhibits the function of a protein comprising the same or substantially the same amino acid sequence as that shown in SEQ ID NO: 11 or SEQ ID NO: 13, or a partial peptide thereof or a salt thereof, for producing an agent for the prophylaxis or treatment of cancer.
[64] Use of a substance that inhibits the function of a protein comprising the same or substantially the same amino acid sequence as that shown in SEQ ID NO: 11 or SEQ ID NO: 13, or a partial peptide thereof or a salt thereof, for producing an agent for the prophylaxis or treatment of breast cancer.
[65] Use of a substance that inhibits the function of a protein comprising the same or substantially the same amino acid sequence as that shown in SEQ ID NO: 11 or SEQ ID NO: 13, or a partial peptide thereof or a salt thereof, for producing a growth inhibitor of a cancer cell.
[66] Use of a substance that inhibits the function of a protein comprising the same or substantially the same amino acid sequence as that shown in SEQ ID NO: 11 or SEQ ID NO: 13, or a partial peptide thereof or a salt thereof, for producing a growth inhibitor of a breast cancer cell.
[67] The use of [63] - [66], wherein the substance is an antibody to a protein comprising the same or substantially the same amino acid sequence as that shown in SEQ ID NO: 11 or SEQ ID NO: 13, or a partial peptide thereof or a salt thereof.
[68] The use of [67], wherein the antibody is a monoclonal antibody.
[69] A diagnostic reagent of breast cancer comprising a nucleic acid encoding a protein comprising the same or substantially the same amino acid sequence as that shown in SEQ ID NO: 1 or SEQ ID NO: 3, or a partial peptide thereof.
[70] An agent for the prophylaxis or treatment of breast cancer comprising a substance that inhibits the function of GDNF (a protein comprising the same or substantially the same amino acid sequence as that shown in SEQ ID NO: 5, SEQ ID NO: 7 or SEQ ID NO: 9), GFRα1 (a protein comprising the same or substantially the same amino acid sequence as that shown in SEQ ID NO: 11 or SEQ ID NO: 13) and RET (a protein comprising the same or substantially the same amino acid sequence as that shown in SEQ ID NO: 1 or SEQ ID NO: 3).
[71] The agent of [70], wherein the function of GDNF, GFRα1 and RET is an activation of RET (a protein comprising the same or substantially the same amino acid sequence as that shown in SEQ ID NO: 1 or SEQ ID NO: 3) by GDNF (a protein comprising the same or substantially the same amino acid sequence as that shown in SEQ ID NO: 5, SEQ ID NO: 7 or SEQ ID NO: 9), or promotion of breast cancer cell growth by GDNF (a protein comprising the same or substantially the same amino acid sequence as that shown in SEQ ID NO: 5, SEQ ID NO: 7 or SEQ ID NO: 9).
[72] The agent of [70], wherein the substance is
   (1) an antibody to a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof or a salt thereof,
   (2) a low-molecular-weight compound that inhibits the function of GDNF, GFRα1 and RET, or a salt thereof,
   (3) an antisense nucleic acid to a nucleic acid encoding a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof, or
   (4) siRNA to RNA encoding a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof, and having a signal transduction inhibitory activity by GDNF (a protein comprising the same or substantially the same amino acid sequence as that shown in SEQ ID NO: 5, SEQ ID NO: 7 or SEQ ID NO: 9), or a breast cancer cell growth inhibitory activity by GDNF (a protein comprising the same or substantially the same amino acid sequence as that shown in SEQ ID NO: 5, SEQ ID NO: 7 or SEQ ID NO: 9).
[73] The agent of [70], wherein the substance is
   (1) an antibody to a protein comprising the same or substantially the same amino acid sequence as that shown in SEQ ID NO: 5, SEQ ID NO: 7 or SEQ ID NO: 9, or a partial peptide thereof or a salt thereof,
   (2) an antibody to a protein comprising the same or substantially the same amino acid sequence as that shown in SEQ ID NO: 11 or SEQ ID NO: 13, or a partial peptide thereof or a salt thereof, or
   (3) an antibody to a protein comprising the same or substantially the same amino acid sequence as that shown in SEQ ID NO: 1 or SEQ ID NO: 3, or a partial peptide thereof or a salt thereof.
[74] The agent of [72] or [73], wherein the antibody is a monoclonal antibody.
[75] The agent of [70], which is used for the treatment of breast cancer expressing GFRα1 protein (a protein comprising the same or substantially the same amino acid sequence as that shown in SEQ ID NO: 11 or SEQ ID NO: 13) and RET protein (a protein comprising the same or substantially the same amino acid sequence as that shown in SEQ ID NO: 1 or SEQ ID NO: 3). [76] The agent of [70], which is used for the treatment of breast cancer expressing GDNF protein (a protein comprising the same or substantially the same amino acid sequence as that shown in SEQ ID NO: 5, SEQ ID NO: 7 or SEQ ID NO: 9), GFRα1 protein (a protein comprising the same or substantially the same amino acid sequence as that shown in SEQ ID NO: 11 or SEQ ID NO: 13) and RET protein (a protein comprising the same or substantially the same amino acid sequence as that shown in SEQ ID NO: 1 or SEQ ID NO: 3).
[77] A method for the prophylaxis or treatment of breast cancer, comprising administering, to a mammal, an effective amount of a substance that inhibits the function of GDNF, GFRα1 and RET. [78] A method for the prophylaxis or treatment of breast cancer, comprising administering, to a patient with breast cancer expressing GFRα1 protein and RET protein, an effective amount of a substance that inhibits the function of GDNF, GFRα1 and RET.
[79] A method for the prophylaxis or treatment of breast cancer, comprising administering, to a patient with breast cancer expressing GDNF protein, GFRα1 protein and RET protein, an effective amount of a substance that inhibits the function of GDNF, GFRα1 and RET.
[80] The method of any of [77] - [79], wherein the substance is
   (1) an antibody to a protein comprising the same or substantially the same amino acid sequence as that shown in SEQ ID NO: 5, SEQ ID NO: 7 or SEQ ID NO: 9, or a partial peptide thereof or a salt thereof,
   (2) an antibody to a protein comprising the same or substantially the same amino acid sequence as that shown in SEQ ID NO: 11 or SEQ ID NO: 13, or a partial peptide or a salt thereof, or
   (3) an antibody to a protein comprising the same or substantially the same amino acid sequence as that shown in SEQ ID NO: 1 or SEQ ID NO: 3, or a partial peptide thereof or a salt thereof.
[81] Use of a substance that inhibits the function of GDNF, GFRα1 and RET, for producing an agent for the prophylaxis or treatment of breast cancer.
[82] Use of a substance that inhibits the function of GDNF, GFRα1 and RET, for producing an agent for the prophylaxis or treatment of breast cancer to patients of breast cancer expressing GFRα1 protein and RET protein.
[83] Use of a substance that inhibits the function of GDNF, GFRα1 and RET, for producing an agent for the prophylaxis or treatment of breast cancer to patients of breast cancer expressing GDNF protein, GFRα1 protein and RET protein.
[84] The use of any of [81] - [83], wherein the substance is
   (1) an antibody to a protein comprising the same or substantially the same amino acid sequence as that shown in SEQ ID NO: 5, SEQ ID NO: 7 or SEQ ID NO: 9, or a partial peptide or a salt thereof,
   (2) an antibody to a protein comprising the same or substantially the same amino acid sequence as that shown in SEQ ID NO: 11 or SEQ ID NO: 13, or a partial peptide or a salt thereof, or
   (3) an antibody to a protein comprising the same or substantially the same amino acid sequence as that shown in SEQ ID NO: 1 or SEQ ID NO: 3, or a partial peptide or a salt thereof, and the like.

### Effect of the Invention

A substance that inhibits a function of RET, such as an antibody against RET, a substance that inhibits a function of GDNF, such as an antibody against GDNF, and a substance that inhibits a function of GFRα1, such as an antibody against GFRα1, can be safely used as, for example, prophylactic/therapeutic agents for breast cancer and the like, cancer cell apoptosis promoters, cancer cell growth inhibitors and the like.

### Brief Description of the Drawings

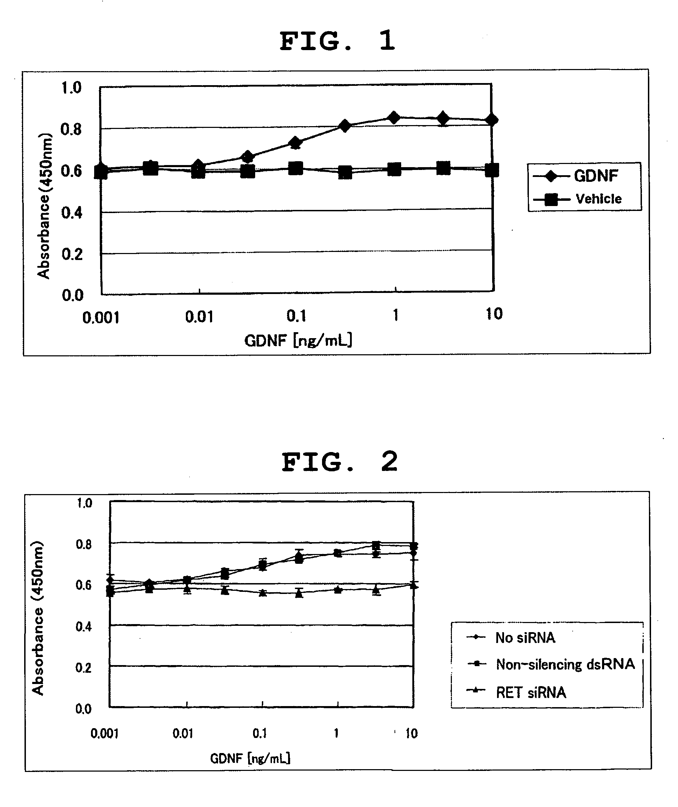

[FIG. 1] Shown is the relationship between GDNF concentration (ng/mL) and cell growth promoting activity in a human breast cancer cell line, determined using the Cell-Counting Kit-8 solution (Wako Pure Chemical Industries). The abscissa indicates the concentration (µg/mL) of GDNF administered, and the ordinate indicates absorbance at 450 nm.
[FIG. 2] Shown is the relationship between GDNF concentration (ng/mL) in the presence of siRNA against the RET gene and cell growth promoting activity in a human breast cancer cell line, determined using the Cell-Counting Kit-8 solution (Wako Pure Chemical Industries). The abscissa indicates the concentration (ng/mL) of GDNF administered, and the ordinate indicates absorbance at 450 nm.

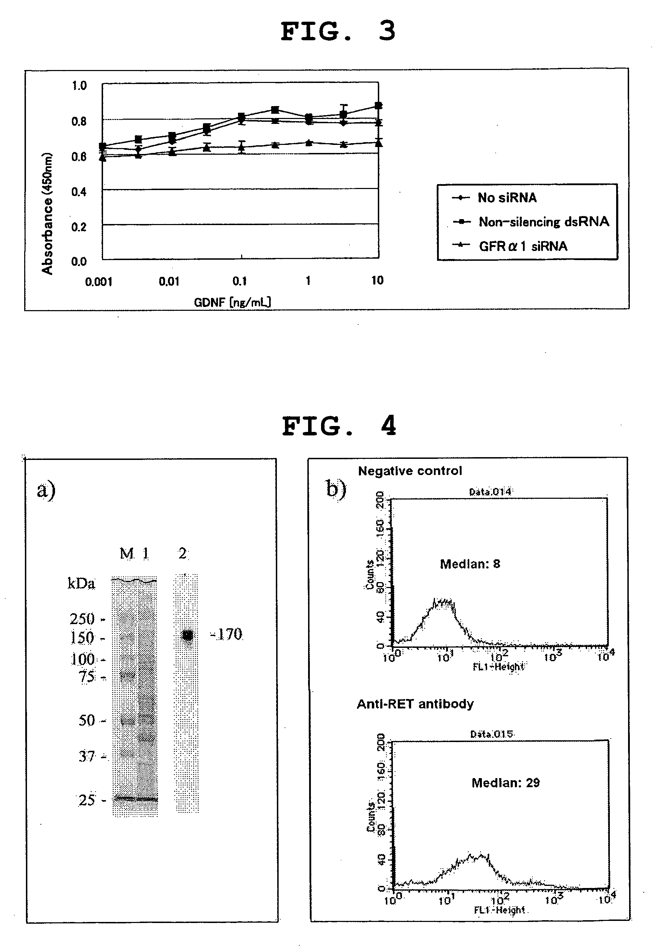

[FIG. 3] Shown is the relationship between GDNF concentration (ng/mL) in the presence of siRNA against the GFRα1 gene and cell growth promoting activity in a human breast cancer cell line, determined using the Cell-Counting Kit-8 solution (Wako Pure Chemical Industries). The abscissa indicates the concentration (ng/mL) of GDNF administered, and the ordinate indicates absorbance at 450 nm.
[FIG. 4] a) Shown is the expression of RET protein in a human breast cancer cell line, analyzed using the Western blot method. b) Shown is the expression of RET protein in a human breast cancer cell line, analyzed using flow cytometry technique.

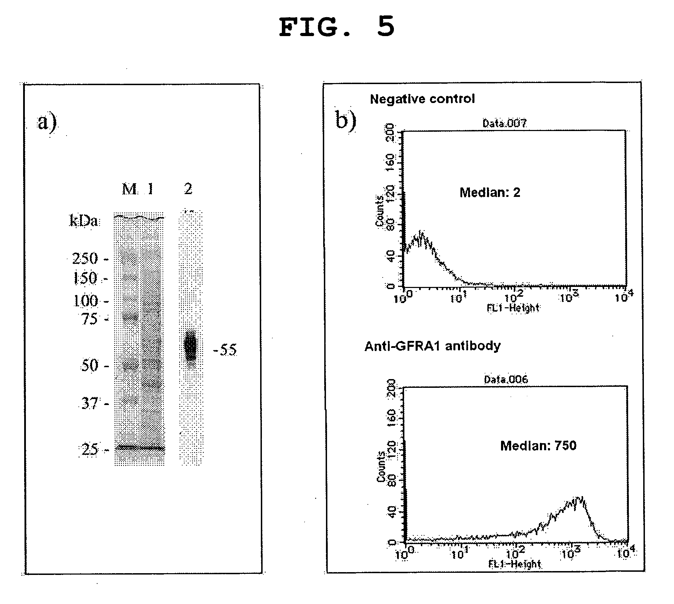

[FIG. 5] a) Shown is the expression of GFRα1 protein in a human breast cancer cell line, analyzed using the Western blot method. b) Shown is the expression of GFRα1 protein in a human breast cancer cell line, analyzed using flow cytometry technique.

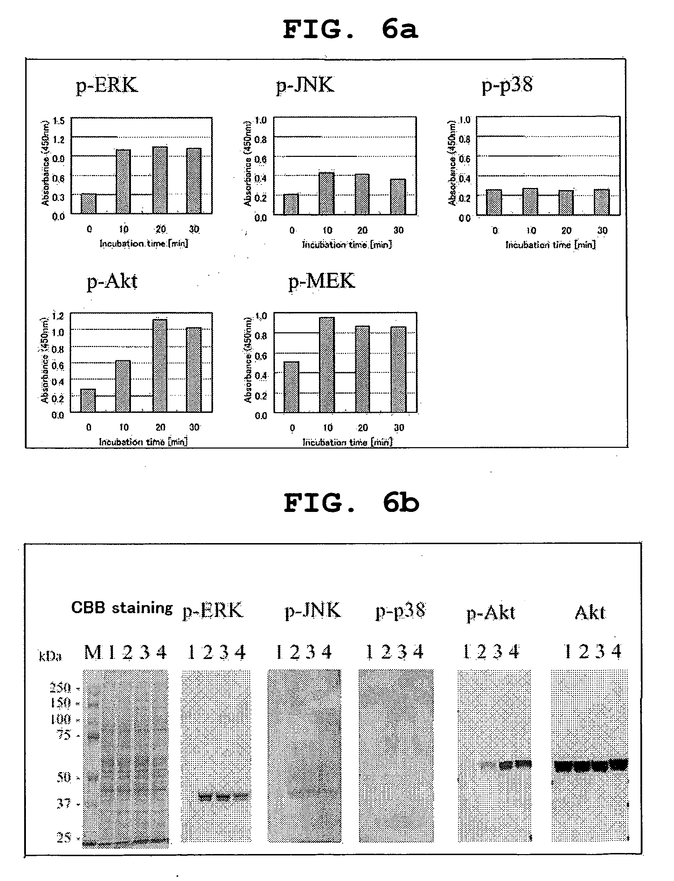

[FIG. 6a] Shown is the relationship between the time of reaction with GDNF and the phosphorylation of signal transduction system protein in a human breast cancer cell line. Determined using ELISA. The abscissa indicates the time (min) of reaction with GDNF administered, and the ordinate indicates absorbance at 450 nm.
[FIG. 6b] Shown is the relationship between the time of reaction with GDNF and the phosphorylation of signal transduction system protein in a human breast cancer cell line. Analyzed using the Western blot method. Electrophoresed on lanes 1, 2, 3, and 4 were lyzates of cells reacted with GDNF for 0, 10, 20, and 30 minutes, respectively.

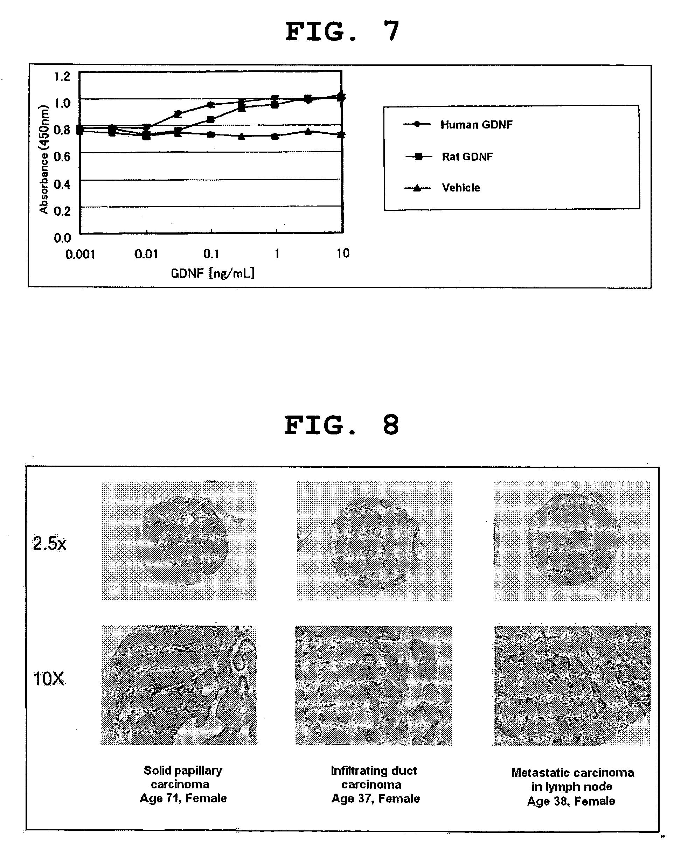

[FIG. 7] Shown is the relationship between rat GDNF concentration (ng/mL) and cell growth promoting activity in a human breast cancer cell line, determined using the Cell-Counting Kit-8 solution (Wako Pure Chemical Industries). The abscissa indicates the concentration (ng/mL) of rat GDNF administered, and the ordinate indicates absorbance at 450 nm.
[FIG. 8] Shown is the expression of RET protein in human breast cancer tissue, determined using an immunohistochemistry technique.

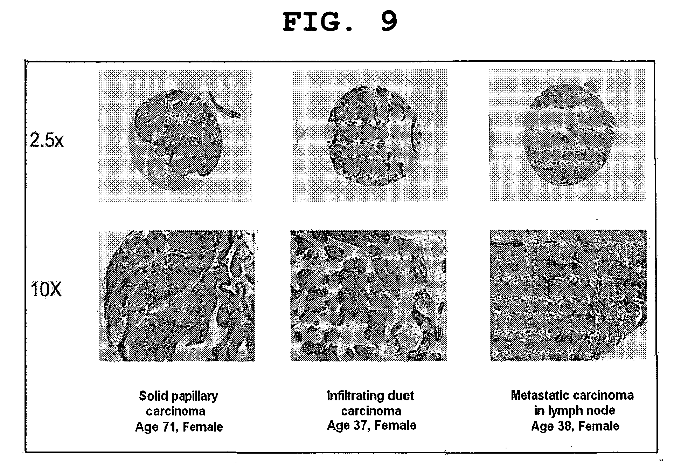

[FIG. 9] Shown is the expression of GFRα1 protein in human breast cancer tissue, determined using an immunohistochemistry technique.

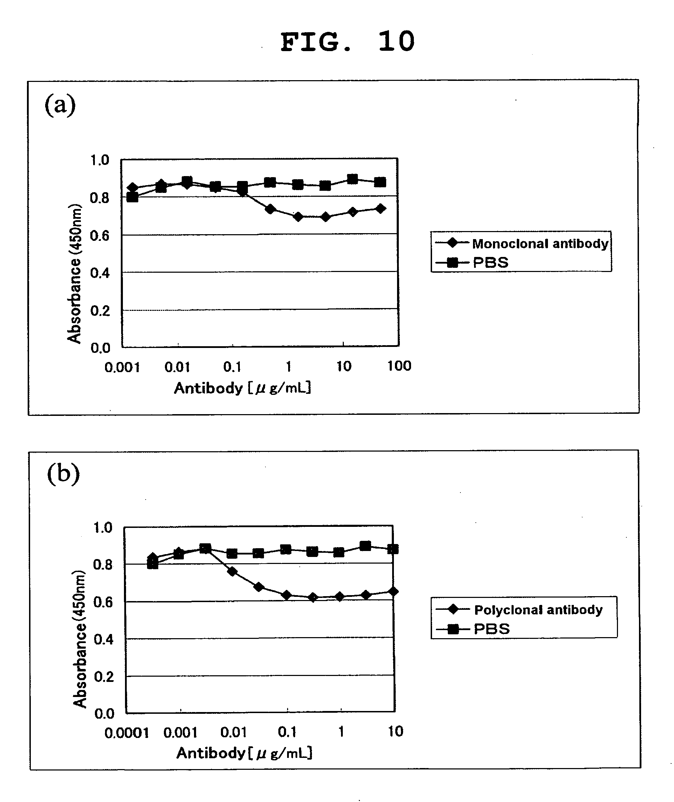

[FIG. 10] Shown is the relationship between anti-GDNF antibody concentration (µg/mL) and cell growth inhibitory activity in a human breast cancer cell line, determined using the Cell-Counting Kit-8 solution (Wako Pure Chemical Industries). The abscissa indicates the concentration (µg/mL) of anti-GDNF antibody administered, and the ordinate indicates absorbance at 450 nm.

### Best Mode for Carrying out the Invention

### (I. anti-RET antibody and the like)

A protein comprising the same or substantially the same amino acid sequence as that shown by SEQ ID NO:1 (hereinafter sometimes to be abbreviated as "RET protein·isoform a") or a protein comprising the same or substantially the same amino acid sequence as that shown by SEQ ID NO:3 (hereinafter sometimes to be abbreviated as "RET protein·isoform c" (hereinafter both are sometimes to be collectively abbreviated as "RET" or "protein I of the present invention") may be a protein derived from a cell (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell (e.g., breast cancer cell) thereof, and the like) of a human or warm-blooded animal (for example, guinea pigs, rats, mice, chicken, rabbits, pigs, sheep, cattle, monkeys and the like) or any tissue in which these cells are present, for example, brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, skeletal muscle, and the like, and may be a synthetic protein.

As substantially the same amino acid sequence as that shown by SEQ ID NO:1 or SEQ ID NO:3, an amino acid sequence having a homology of about 50% or more, preferably about 60% or more, more preferably about 70% or more, still more preferably about 80% or more, particularly preferably about 90% or more, most preferably about 95% or more, to the amino acid sequence shown by SEQ ID NO:1 or SEQ ID NO:3 and the like can be mentioned.

As the protein comprising substantially the same amino acid sequence as that shown by SEQ ID NO:1 or SEQ ID NO:3, for example, a protein comprising the above-mentioned substantially the same amino acid sequence as that shown by SEQ ID NO:1 or SEQ ID NO:3, and having-substantially the same quality of activity as a protein comprising the amino acid sequence shown by SEQ ID NO:1 or SEQ ID NO:3 and the like are preferable.

Here, 'a homology' means a ratio (%) of identical amino acid residues and similar amino acid residues to all overlapping amino acid residues in the best alignment (preferably, the algorithm considers introduction of gaps on one or both sides of the sequence for the best alignment) where two amino acid sequences are aligned using a mathematical algorithm known in the technical field. 'A similar amino acid' means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected to give no change in the phenotype of protein (i.e., constitutive amino acid substitution). Specific examples of constitutive amino acid substitution are obvious in the relevant technical field, and are described in various documents (see, for example, Bowie et al., Science, 247:1306-1310 (1990)).
Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

As the substantially equivalent activity described above, there are, for example, an activity to promote proliferation of cancer cells (e.g., breast cancer cells), and the like, by intracellularly transmitting the stimulation of GDNF. The substantially equivalent is used to mean that the nature of the activities is equivalent in terms of quality (e.g., physiologically or pharmacologically). Thus, the level of activities of the protein I of the present invention are preferably equivalent to those of a protein having an amino acid sequence represented by SEQ ID NO: 1 or SEQ ID NO: 3 (e.g., about 0.01 to 100 times, preferably about 0.1 to 10 times, more preferably 0.5 to 2 times), but differences in quantitative factors such as a level of these activities, a molecular weight of the protein, and the like may be present and allowable.
A measurement of the activity of RET can be performed in accordance with a method known per se. For example, as described in an Example below, by measuring cell growth when cancer cells (e.g., breast cancer cells) that are co-expressing RET and GFRα1 are stimulated with GDNF, the activity can be evaluated.

Examples of RET also include what are called muteins of proteins comprising (i) an amino acid sequence having 1 or 2 or more (for example, about 1 to 50, preferably about 1 to 30, more preferably about 1 to 10, still more preferably several (1 to 5)) amino acids deleted from the amino acid sequence shown by SEQ ID NO:1 or SEQ ID NO:3, (ii) an amino acid sequence having 1 or 2 or more (for example, about 1 to 50, preferably about 1 to 30, more preferably about 1 to 10, still more preferably several (1 to 5)) amino acids added to the amino acid sequence shown by SEQ ID NO:1 or SEQ ID NO:3, (iii) an amino acid sequence having 1 or 2 or more (for example, about 1 to 50, preferably about 1 to 30, more preferably about 1 to 10, still more preferably several (1 to 5)) amino acids inserted in the amino acid sequence shown by SEQ ID NO:1 or SEQ ID NO:3, (iv) an amino acid sequence having 1 or 2 or more (for example, about 1 to 50, preferably about 1 to 30, more preferably about 1 to 10, still more preferably several (1 to 5)) amino acids substituted by other amino acids in the amino acid sequence shown by SEQ ID NO:1 or SEQ ID NO:3, or (v) an amino acid sequence comprising a combination thereof. The protein preferably has a substantially homogeneous activity as a protein containing an amino acid sequence shown in SEQ ID NO:1 or SEQ ID NO:3.
When an amino acid sequence is inserted, deleted or substituted as described above, the position of the insertion, deletion or substitution is not subject to limitation.

For the proteins mentioned herein, the left end indicates the N-terminus (amino terminus) and the right end indicates the C-terminus (carboxyl terminus), according to the common practice of peptide designation. For the protein comprising the same amino acid sequence as that shown by SEQ ID NO:1 used in the present invention, the C-terminus may be any of a carboxyl group (-COOH), a carboxylate (-COO⁻), an amide (-CONH₂) or an ester (-COOR).

Here, as R in the ester, a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl, a phenyl-C₁₋₂ alkyl group such as benzyl and phenethyl, a C₇₋₁₄ aralkyl group such as an α-naphthyl-C₁₋₂ alkyl group such as α-naphthylmethyl, a pivaloyloxymethyl group; and the like can be used.

When the RET has a carboxyl group (or a carboxylate) in addition to that on the C-terminal, one in which the carboxyl group is amidated or esterified is also included in the RET used in the present invention. In this case, as the ester, the above-described C-terminal ester and the like, for example, can be used.

Furthermore, the RET also includes a protein wherein the amino group of the N-terminal amino acid residue thereof (e.g., methionine residue) is protected by a protecting group (for example, a C₁₋₆ acyl group such as C₁₋₆ alkanoyl such as a formyl group or an acetyl group, and the like), a protein wherein the N-terminal glutamine residue, which is produced by cleavage in vivo, has been converted to pyroglutamic acid, a protein wherein a substituent (for example, -OH, -SH, an amino group, an imidazole group, an indole group, a guanidino group and the like) on an amino acid side chain in the molecule is protected by an appropriate protecting group (for example, a C₁₋₆ acyl group such as a C₁₋₆ alkanoyl group such as a formyl group or an acetyl group, and the like), a conjugated protein such as what is called a glycoprotein, which has a sugar chain bound thereto, and the like.

As specific examples of RET, a protein comprising the amino acid sequence shown by SEQ ID NO:1 (human RET protein isoform a), a protein comprising the amino acid sequence shown by SEQ ID NO:3 (human RET protein isoform c) and the like can be mentioned.

The partial peptide of RET may be any of the partial peptides of RET described above, preferably having substantially the same quality of activity as RET described above. Here, 'substantially the same quality of activity' is as defined above. A determination of 'substantially the same quality of activity' can be performed as described above. The partial peptide of RET preferably has immunogenicity.

For example, a peptide having at least 20 or more, preferably 50 or more, more preferably 70 or more, still more preferably 100 or more, most preferably 200 or more, amino acids of the constituent amino acids of the sequence of the RET and the like are used.

In addition, the partial peptide of the RET used in the present invention may have (1) 1 or 2 or more (preferably about 1 to 20, more preferably about 1 to 10, still more preferably several (1 to 5)) amino acids deleted from the amino acid sequence thereof, or (2) 1 or 2 or more (preferably about 1 to 20, more preferably about 1 to 10, still more preferably several (1 to 5)) amino acids added to the amino acid sequence thereof, or (3) 1 or 2 or more (preferably about 1 to 20, more preferably about 1 to 10, still more preferably several (1 to 5)) amino acids inserted in the amino acid sequence thereof, or (4) 1 or 2 or more (preferably about 1 to 20, more preferably about 1 to 10, still more preferably several, still yet more preferably about 1 to 5) amino acids substituted by other amino acids in the amino acid sequence thereof, or (5) a combination thereof.

For the partial peptide of the RET, the C-terminus may be any of a carboxyl group (-COOH), a carboxylate (-COO⁻), an amide (-CONH₂) or an ester (-COOR).
Furthermore, the partial peptide of the RET, like the foregoing RET, also includes a partial peptide wherein a carboxyl group (or carboxylate) is present at a position other than the C-terminus, a partial peptide wherein the amino group of the N-terminal amino acid residue (e.g., methionine residue) is protected by a protecting group, a partial peptide wherein glutamine residue, which is produced upon cleavage at the N-terminal in vivo, has been converted to pyroglutamic acid, a partial peptide wherein a substituent on a side chain of an amino acid in the molecule is protected by an appropriate protecting group, a conjugated peptide such as what is called a glycopeptide having a sugar chain bound thereto, and the like.

The length of such an immunogenic peptide is not particularly limited, as long as the peptide has immunogenicity; for example, one having 8, preferably 10, more preferably 12, continuous amino acid residues can be mentioned.

Useful salts of RET or a partial peptide thereof include salts with physiologically acceptable acids (e.g., inorganic acids, organic acids), bases (e.g., alkali metal salts) and the like, and physiologically acceptable acid addition salts are particularly preferable. Such salts include, for example, salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Useful substances that inhibit a function of RET or a partial peptide thereof or a salt thereof include, for example,
(1) an antibody against RET or a partial peptide thereof or a salt thereof,
(2) a low-molecular compound that inhibits a function of RET or a partial peptide thereof or a salt thereof, or a salt thereof,
(3) an antisense nucleic acid against a nucleic acid that encodes RET or a partial peptide thereof, or
(4) an siRNA against the RNA that encodes RET or a partial peptide thereof and the like.

Although the antibody against RET or a partial peptide thereof or a salt thereof (hereinafter, sometimes abbreviated 'the antibody I of the present invention') may be a polyclonal antibody or a monoclonal antibody, as long as it is an antibody capable of recognizing RET or a partial peptide thereof or a salt thereof, the antibody is preferably a monoclonal antibody. Although the isotype of the antibody is not particularly limited, it is preferably IgG, IgM or IgA. The antibody I of the present invention may be any of a mouse antibody, rat antibody, rabbit antibody, human antibody, humanized antibody, chimeric antibody thereof and the like. Alternatively, antibodies obtained by antibody display methods, such as the phage display method, using a non-human warm-blooded animal (e.g., rabbit, goat, bovine, chicken, mouse, rat, sheep, pig, horse, cat, dog, monkey, chimpanzee and the like) or human antibody gene library and the like can also be included in the antibody I of the present invention. The antibody I of the present invention is preferably a human monoclonal antibody.

The antibody I of the present invention is not particularly limited with respect to molecular morphology, as long as it has at least a complementarity determining region (CDR) for specifically recognizing and binding to RET or a partial peptide thereof or a salt thereof; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like.

As the antibody I of the present invention, an antibody that recognizes an extracellular region of RET is preferable.
As examples of the extracellular region of RET, the following can be mentioned:
A region consisting of amino acid numbers 1 to 635 in a protein consisting of the amino acid sequence shown by SEQ ID NO:1; and
a region consisting of amino acid numbers 1 to 635 in a protein consisting of the amino acid sequence shown by SEQ ID NO:3.

An antibody against RET or a partial peptide thereof or a salt thereof (hereinafter, in the explanation of antibodies, these are sometimes comprehensively abbreviated 'RETs') can be produced in accordance with a method of antibody or antiserum production known per se.

Described below are a method of preparing an antigen for the antibody I of the present invention, and a method of producing the antibody.

### (1) Preparation of antigen

As the antigen used to prepare the antibody I of the present invention, any of the above-described RETs (for example, a protein comprising the amino acid sequence shown by SEQ ID NO:1 or SEQ ID NO:3 (RET) or a partial peptide thereof or a salt thereof), a fusion protein between an extracellular region protein of RET and another protein (peptide) or a salt thereof, or a (synthetic) peptide having 1 kind or 2 or more kinds of the same antigen determinant as RET and the like can be used (hereinafter, these are also simply referred to as the antigen I of the present invention).

As specific examples of the antigen I of the present invention, a cell line that naturally or artificially highly expresses RETs or a membrane fraction thereof, an extracellular region protein of RET or a salt thereof, a fusion protein between an extracellular region of RET and another protein (peptide), or a (synthetic) peptide having 1 kind or 2 or more kinds of the same antigen determinant as RET and the like can be mentioned.

As examples of another protein or peptide, FLAG-tag, His-tag, Myc-tag, V5-tag, GST-tag, S-tag, T7-tag, the Fc region of an antibody (human antibody, mouse antibody and the like) and the like can be mentioned.

The length of such a (synthetic) peptide is not particularly limited, as long as the peptide has immunogenicity; for example, one having 8, preferably 10, more preferably 12, continuous amino acid residues can be mentioned.

RET or a partial peptide thereof or a salt thereof can be produced from the above-described human or warm-blooded animal cells or tissue by a method of protein purification known per se or a method based thereon, and can also be produced by culturing a transformant comprising a nucleic acid (DNA, RNA and the like) that encodes the protein. RET or a partial peptide thereof or a salt thereof can also be produced in accordance with the method of peptide synthesis described below. A fusion protein between an extracellular region of RET and another protein (peptide) can be produced by culturing a transformant comprising a nucleic acid (DNA, RNA and the like) that encodes the fusion protein.

(a) When the antigen I of the present invention is prepared from a human or warm-blooded animal (for example, guinea pig, rat, mouse, chicken, rabbit, pig, sheep, bovine, monkey and the like) tissue or cells, the tissue or cells may be homogenized, after which a crude fraction (e.g., membrane fraction, soluble fraction) can be used as the antigen as is. Alternatively, the antigen can be purified and isolated by performing extraction with an acid, surfactant or alcohol and the like, and applying the extract to a combination of salting-out, dialysis, chromatographies such as gel filtration reversed-phase chromatography, ion exchange chromatography, and affinity chromatography. The antigen obtained may be used as the immunogen as is, and may be subjected to limited degradation using a peptidase and the like to yield a partial peptide that can be used as the immunogen.

(b) When an RETs or a fusion protein between an extracellular region of RET and another protein (peptide) or a salt thereof is produced using a transformant comprising a nucleic acid that encodes the antigen I of the present invention, the nucleic acid can be prepared according to a commonly known method of cloning [for example, a method described in Molecular Cloning (2nd ed.; J. Sambrook et al., Cold Spring Harbor Lab. Press, 1989) and the like].

The nucleic acid that encodes RET or a partial peptide thereof may be any of the above-described nucleic acids comprising the base sequence that encodes the amino acid sequence of RET or a partial amino acid sequence thereof, used in the present invention. The nucleic acid may be DNA or RNA, or a DNA/RNA chimera, and is preferably DNA. In addition, the nucleic acid may be a double-strand or single-strand. The double-strand may be a double-stranded DNA, a double-stranded RNA, or a DNA:RNA hybrid.

The DNA that encodes RET or a partial peptide thereof can be exemplified by genomic DNA, cDNA derived from human or other warm-blooded animal (e.g., simian, bovine, horse, swine, sheep, goat, rabbit, mouse, rat, guinea pig, hamster, chicken and the like) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or corresponding precursor cells, stem cells, cancer cells (e.g., breast cancer cells) and the like]; or any tissues or organs where such cells are present [for example, brain or parts of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testis, ovary, placenta, uterus, bone, joint, adipose tissue (e.g., brown adipose tissue, white adipose tissue), skeletal muscle and the like], synthetic DNA and the like. As the RNA that encodes RET or a partial peptide thereof, mRNA (mature mRNA) or early transcription product and the like can be mentioned.

As the method of cloning a DNA that fully encodes RET or a partial peptide thereof, a method wherein the DNA is amplified by a PCR method using a synthetic DNA primer having a portion of the base sequence that encodes RET or a partial peptide thereof, a method wherein the desired DNA is selected from a cDNA library by a hybridization method using a DNA fragment or synthetic DNA that encodes a portion or entire region of RET as the probe, and the like can be mentioned. The template polynucleotide used for the PCR may be any one comprising the base sequence that encodes RET or a partial peptide thereof; for example, genomic DNA, a genomic DNA library, cDNA derived from the above-described cell/tissue, a cDNA library derived from the above-described cell/tissue, synthetic DNA and the like can be used. The hybridization can be performed by, for example, a method described in Molecular Cloning, 2nd ed. (J. Sambrook et al., Cold Spring Harbor Lab. Press, 1989) and the like. A commercially available library can also be used according to the instructions of the attached manufacturer's protocol. More preferably, the hybridization can be carried out under high stringent conditions.

High-stringent conditions refer to, for example, conditions involving a sodium concentration of about 19 to 40mM, preferably about 19 to 20mM, and a temperature of about 50 to 70°C, preferably about 60 to 65°C. In particular, a case wherein the sodium concentration is about 19mM and the temperature is about 65°C is most preferred. Those skilled in the art can easily regulate the conditions to obtain a desired stringency by appropriately changing the salt concentration of hybridization solution, hybridization reaction temperature, probe concentration, probe length, number of mismatches, hybridization reaction time, salt concentration of washing solution, washing temperature, and the like.

More specifically, useful nucleic acids (DNA and the like) that encode RET include (i) a nucleic acid comprising the base sequence shown by SEQ ID NO:2 (this nucleic acid encodes a protein comprising the amino acid sequence shown by SEQ ID NO:1 (human RET protein isoform a)), or a nucleic acid comprising a base sequence that hybridizes with the base sequence shown by SEQ ID NO:2 under high stringent conditions, and encoding a protein or peptide having substantially the same quality of activity as the above-described protein comprising the amino acid sequence shown by SEQ ID NO:1 and the like, (ii) a nucleic acid comprising the base sequence shown by SEQ ID NO:4 (the nucleic acid encodes a protein comprising the amino acid sequence shown by SEQ ID NO:3 (human RET protein isoform c)), or a nucleic acid comprising a base sequence that hybridizes with the base sequence shown by SEQ ID NO:4 under high stringent conditions, and encoding a protein or peptide having substantially the same quality of activity as the above-described protein comprising the amino acid sequence shown by SEQ ID NO:3 and the like.
Useful nucleic acids capable of hybridizing with the base sequence shown by SEQ ID NO:2 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:2.
Useful nucleic acids capable of hybridizing with the base sequence shown by SEQ ID NO:4 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:4.
Homology of the base sequences in the present specification can be calculated under the following conditions (an expectation value = 10; gaps are allowed; matrix = BLOSUM62; filtering = ON; match score = 1; mismatch score = -3) using a homology scoring algorithm NCBI BLAST (National Center for Biotechnology Information Basic Local Alignment Search Tool).

The base sequence of the DNA can be converted according to a method known per se, such as the ODA-LA PCR method, the Gapped duplex method, or the Kunkel method, or a method based thereon, using PCR, a commonly known kit, for example, Mutan^{™}-super Express Km (Takara Bio Inc.), Mutan^{™}-K (Takara Bio Inc.) and the like.

The cloned DNA that encodes the RET or the partial peptide thereof can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codons can be added using an appropriate synthetic DNA adapter. To obtain a DNA that encodes a fusion protein between an extracellular region of RET and another protein (peptide) or a salt thereof, a DNA that encodes an extracellular region of RET cloned or synthesized as described above and a DNA that encodes another protein (peptide) can be joined by a method known per se or a method based thereon.

By transforming a host with an expression vector comprising the DNA that encodes the antigen I of the present invention, acquired as described above, and culturing the transformant obtained, the antigen I of the present invention can be produced.
An expression vector for the antigen I of the present invention can be produced by, for example, (a) cutting out a desired DNA fragment from the DNA that encodes the antigen I of the present invention, and (b) joining the DNA fragment downstream of a promoter in an appropriate expression vector.

Useful vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter used in the present invention may be any promoter appropriate for the host used to express the gene. For example, when an animal cell is used as the host, the SRα promoter, the SV40 promoter, the LTR promoter, the CMV promoter, the HSV-TK promoter and the like can be mentioned. Of these promoters, the CMV (cytomegalovirus) promoter, the SRα promoter and the like are preferably used.

When the host is a bacterium of the genus Escherichia, the trp promoter, the lac promoter, the recA promoter, the λP_{L} promoter, the lpp promoter, the T7 promoter and the like are preferred. When the host is a bacterium of the genus Bacillus, the SPO1 promoter, the SPO2 promoter, the penP promoter and the like are preferred. When the host is yeast, the PHO5 promoter, the PGK promoter, the GAP promoter, the ADH promoter and the like are preferred. When the host is an insect cell, the polyhedrin promoter, the P10 promoter and the like are preferred.

Useful expression vectors include, in addition to the above, expression vectors that optionally comprise an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene-defective Chinese hamster cell is used and the dhfr gene is used as the selection marker, a target gene can also be selected using a thymidine-free medium.

In addition, as required, a signal sequence that matches with the host may be added to the 5'-terminal side of the DNA encoding antigen I of the present invention. Useful signal sequences include a PhoA signal sequence, an OmpA signal sequence and the like when the host is a bacterium of the genus Escherichia; an α-amylase signal sequence, a subtilisin signal sequence and the like when the host is a bacterium of the genus Bacillus; an MFα signal sequence, an SUC2 signal sequence and the like when the host is yeast; and an insulin signal sequence, an α-interferon signal sequence, an antibody molecule signal sequence and the like when the host is an animal cell.

Using the thus-constructed vector comprising a DNA that encodes the antigen I of the present invention, a transformant can be produced.

As useful examples of the host, a bacterium of the genus Escherichia, a bacterium of the genus Bacillus, yeast, an insect cell, an insect, an animal cell, and the like can be mentioned.

As specific examples of the bacterium of the genus *Escherichia, Escherichia* coli K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)), and the like can be mentioned.

As useful examples of the bacterium of the genus Bacillus, Bacillus subtilis MI114 (Gene, Vol. 24, 255 (1983)), 207-21 (Journal of Biochemistry, Vol. 95, 87 (1984)) and the like can be mentioned.

As useful examples of the yeast, Saccharomyces cerevisiae AH22, AH22R⁻, NA87-11A, DKD-5D and 20B-12, Schizosaccharomyces pombe NCYC1913 and NCYC2036, Pichia pastoris KM71 and the like can be mentioned.

As useful examples of the insect cell, Spodoptera frugiperda cell (Sf cell), MG1 cell derived from the mid-intestine of Trichoplusia ni, High Five^{™} cell derived from an egg of Trichoplusia ni, cell derived from Mamestra brassicae, cell derived from Estigmena acrea, and the like can be mentioned when the virus is AcNPV. When the virus is BmNPV, Bombyx mori N cell (BmN cell) and the like can be used. As useful examples of the Sf cell, Sf9 cell (ATCC CRL1711), Sf21 cell (both in Vaughn, J. L. et al., In Vivo, 13, 213-217 (1977)), and the like can be mentioned.

As useful examples of the insect, a larva of Bombyx mori (Maeda et al., Nature, Vol. 315, 592 (1985)), and the like can be mentioned.

As useful examples of the animal cell, monkey cell COS-7, Vero cell, Chinese hamster cell CHO (hereinafter abbreviated as CHO cell), Chinese hamster cell (CHO) lacking the dhfr gene (hereinafter abbreviated as CHO(dhfr⁻) cell), mouse L cell, mouse AtT-20 cell, mouse myeloma cell, mouse ATDC5 cell, mouse NSO cell, mouse FM3A cell, rat GH3 cells, human FL cell, human fetal HEK293 cell, human fetal cell 293F cell, and the like can be used.

Transformation can be performed according to the choice of host by a commonly known method.
A bacterium of the genus *Escherichia* can be transformed, for example, in accordance with a method described in Proc. Natl. Acad. Sci. USA, Vol.69, 2110 (1972), Gene, Vol.17, 107 (1982) and the like.

A bacterium of the genus *Bacillus* can be transformed, for example, according to a method described in Molecular & General Genetics, Vol.168, 111 (1979) and the like.

Yeast can be transformed, for example, in accordance with a method described in Methods in Enzymology, Vol.194, 182-187 (1991), Proc. Natl. Acad. Sci. USA, Vol.75, 1929 (1978) and the like.

An insect cell or insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, Vol.52, 456 (1973).

Thus, a transformant transformed with an expression vector comprising a DNA that encodes the antigen I of the present invention can be obtained.

Transformation can be performed according to the choice of host by a commonly known method.
When a transformant whose host is a bacterium of the genus *Escherichia* or a bacterium of the genus *Bacillus* is cultured, the culture medium used is preferably a liquid medium, in which a carbon source, a nitrogen source, an inorganic substance and others necessary for the growth of the transformant are contained. As examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, and potato extract can be mentioned; as examples of the inorganic substance, calcium chloride, sodium dihydrogen phosphate, magnesium chloride and the like can be mentioned.
In addition, yeast extract, vitamins, a growth promoting factor and the like may be added. The pH of the medium is desirably about 5 to 8.

As an example of the medium used to culture a bacterium of the genus *Escherichia,* an M9 medium comprising glucose and casamino acid [Miller, Journal of Experiments in Molecular Genetics, 431-433, Cold Spring Harbor Laboratory, New York, 1972] is preferable. As required, in order to increase promoter efficiency, a chemical agent, for example, 3β-indolylacrylic acid, may be added to the medium.

When the host is a bacterium of the genus *Escherichia,* cultivation is normally performed at about 15 to 43°C for about 3 to 24 hours, and the culture may be aerated or agitated as necessary.

When the host is a bacterium of the genus *Bacillus,* cultivation is normally performed at about 30 to 40°C for about 6 to 24 hours, and the culture may be aerated or agitated as necessary.

When a transformant whose host is yeast is cultured, as examples of the medium, Burkholder's minimal medium [Proc. Natl. Acad. Sci. USA, Vol.77, 4505(1980)] and an SD medium supplemented with 0.5% casamino acid [Proc. Natl. Acad. Sci. USA, Vol.81, 5330(1984)] can be mentioned. The pH of the medium is preferably adjusted to about 5 to 8. Cultivation is normally performed at about 20°C to 35°C for about 24 to 72 hours, and the culture may be aerated or agitated as necessary.

When a transformant whose host is an insect cell or insect is cultured, as the medium, Grace's Insect Medium (Nature, 195, 788(1962)) supplemented with inactivated 10% bovine serum and other additives as appropriate and the like are used. The pH of the medium is preferably adjusted to about 6.2 to 6.4. Cultivation is normally performed at about 27°C for about 3 to 5 days, and the culture may be aerated or agitated as necessary.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally performed at about 30 to 40°C for about 15 to 60 hours, and the culture may be aerated or agitated as necessary.

Thus, the antigen I of the present invention can be produced in the cells, on the cell membrane or out of the cells of the transformant.

Separation and purification of the RET from the above-described culture can be performed by, for example, the method described below.

When the antigen I of the present invention is extracted from a cultured bacterium or cells, a method is used as appropriate wherein the bacterium or cells are collected by a commonly known method after cultivation, suspended in an appropriate buffer solution, and disrupted by means of sonication, lysozyme and/or freeze-thawing and the like, after which a crude extract of the protein is obtained by centrifugation or filtration. The buffer solution may contain a protein denaturant such as urea or guanidine hydrochloride and a surfactant such as Triton X-100^{™}. When the antigen I of the present invention is secreted in the culture broth, the bacterium or cells are separated from the supernatant by a method known per se, and the supernatant is collected, after completion of the cultivation.

Purification of the antigen I of the present invention contained in the thus-obtained culture supernatant or extract can be performed by an appropriate combination of methods of separation/purification known per se. These commonly known methods of separation/purification include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like.

When the antigen I of the present invention thus obtained is a free form, the free form can be converted to a salt by a method known per se or a method based thereon; conversely, when the protein is obtained in the form of a salt, the salt can be converted to a free form or another salt by a method known per se or a method based thereon.

The antigen I of the present invention produced by the transformant can be optionally modified or partially deprived of a polypeptide by allowing an appropriate protein-modifying enzyme to act thereon before the purification or after the purification. As the protein-modifying enzyme used, for example, trypsin, chymotrypsin, arginyl endopeptidase, protein kinase, glycosidase and the like are used.

The presence of the antigen I of the present invention thus produced can be measured by an enzyme immunoassay, Western blotting and the like using a specific antibody.

(c) RET-expressing mammalian cells themselves can be used directly as the antigen I of the present invention. Preferably useful mammalian cells include natural cells as described in section (a) above, cells transformed by a method as described in section (b) above and the like. The host used for the transformation may be any cells collected from humans, monkeys, rats, mice, hamsters and the like; preferably useful cells include HEK293 cells, COS7 cells, CHO-K1 cells, NIH3T3 cells, Balb3T3 cells, FM3A cells, L929 cells, SP2/0 cells, P3U1 cells, NS0 cells, B16 cells, or P388 cells and the like.
(d) A peptide having 1 kind or 2 kinds or more of the same antigen determinant as that of an RET can be produced according to a commonly known method of peptide synthesis, or by cleaving an RET with an appropriate peptidase. The method of peptide synthesis may be any of, for example, a solid phase synthesis process and a liquid phase synthesis process. That is, a desired peptide can be produced by condensing a partial peptide or amino acids capable of constituting the peptide and the remaining portion, and eliminating any protecting group the resultant product may have. As examples of the commonly known methods of condensation and elimination of the protecting group, the methods described below can be mentioned.
   (i) M. Bodanszky and M.A. Ondetti, Peptide Synthesis, Interscience Publishers, New York (1966)
   (ii) Schroeder and Luebke, The Peptide, Academic Press, New York (1965)
   (iii) Nobuo Izumiya, et al.: Peptide Gosei-no-Kiso to Jikken, published by Maruzen Co. (1975);
   (iv) Haruaki Yajima and Shunpei Sakakibara: Seikagaku Jikken Koza 1, Tanpakushitsu no Kagaku IV, 205 (1977)
   (v) Haruaki Yajima, ed.: Zoku Iyakuhin no Kaihatsu, Vol. 14, Peptide Synthesis, published by Hirokawa Shoten.

After the reaction, the partial peptide used in the present invention can be purified and isolated by a combination of ordinary methods of purification, for example, solvent extraction, distillation, column chromatography, liquid chromatography, recrystallization and the like. When the peptide obtained by the above-described method is a free form, the free form can be converted to an appropriate salt by a commonly known method; conversely, when the peptide is obtained in the form of a salt, the salt can be converted to a free form by a commonly known method.

### (2) Preparation of monoclonal antibody

### (a) Preparation of monoclonal antibody producing cell by hybridoma method

The antigen I of the present invention is administered to a warm-blooded animal. The method of immunization may be any method allowing promotion of antibody production; intravenous injection, intraperitoneal injection, intramuscular injection or subcutaneous injection and the like are preferably used.
Natural mammalian cells or transformed mammalian cells that express the protein I used in the present invention can be injected to an immunized animal in suspension in a medium used for tissue culture (e.g., RPMI1640) or a buffer solution (e.g., Hanks' Balanced Salt Solution).

The antigen I of the present invention permits direct use for immunization in an insolubilized form. The antigen I of the present invention may be used for immunization in the form of a conjugate thereof bound or adsorbed to a suitable carrier. Regarding the mixing ratio of the carrier and the antigen I of the present invention (hapten), any carrier can be bound or adsorbed in any ratio, as long as an antibody against the antigen I of the present invention bound or adsorbed to the carrier is efficiently produced; usually, a natural or synthetic polymeric carrier in common use for preparation of an antibody against a hapten antigen, bound or adsorbed in a ratio of 0.1 to 100 parts by weight to 1 part by weight of the hapten, can be used. As examples of the natural polymeric carrier, the serum albumin of a mammal such as cattle, rabbit, or human, the thyroglobulin of a mammal such as cattle or rabbit, the hemoglobin of a mammal such as cattle, rabbit, human, or sheep, keyhole limpet hemocyanin and the like are used. As examples of the synthetic polymeric carrier, various latexes of polymers or copolymers of polyamino acids, polystyrenes, polyacryls, polyvinyls, polypropylenes and the like, and the like can be used.

Various condensing agents can be used for crosslinking the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like are conveniently used. When amino groups are crosslinked together, it is also possible to react one amino group with an activated ester reagent having a dithiopyridyl group (for example, 3-(2-pyridyldithio)propionic acid N-succinimidyl (SPDP) and the like), followed by reduction, to introduce the thiol group, and to introduce a maleimide group into the other amino group using a maleimide activated ester reagent, followed by a reaction of both.

In order to increase antibody productivity during the administration, complete Freund's adjuvant or incomplete Freund's adjuvant may be administered. The administration is usually made every 2 to 6 weeks about 2 to 10 times in total. In preparing the monoclonal antibody I of the present invention, the DNA immunization method may be utilized (see, for example, Nature, Vol.356, term 152 to term 154). As the warm-blooded animal, for example, monkeys, rabbits, dogs, guinea pigs, mice, rats, sheep, goat, chicken and the like can be mentioned, and mice and rats are preferably used.

In preparing monoclonal antibody-producing cells, a monoclonal antibody-producing hybridoma can be prepared by selecting an individual showing an antibody titer from among antigen-immunized warm-blooded animals, for example, mice, collecting the spleen or lymph nodes 2 to 5 days after final immunization, and fusing antibody-producing cells contained therein with myeloma cells of the same or different animal species. A measurement of antibody titer in antiserum may be made by, for example, reacting the labeled protein described below with the antiserum, and thereafter determining the activity of the labeling agent bound to the antibody. The fusion may be operated by a known method, for example, the method of Koehler and Milstein [Nature, 256, 495 (1975)]. As examples of fusogen, polyethylene glycol (PEG), Sendai virus and the like can be mentioned, and PEG is preferably used.

As examples of the myeloma cell, NS-1, P3U1, SP2/0, AP-1 and the like can be mentioned, and SP2/0 or P3U1 and the like are preferably used. A preferable ratio of the number of antibody-producing cells (splenocytes) and number of myeloma cells used is generally about 1:1 to 20:1; cell fusion can be efficiently performed by adding a PEG (preferably PEG1000 to PEG6000) at concentrations of about 10 to 80%, and conducting incubation generally at 20 to 40°C, preferably at 30 to 37°C, generally for 1 to 10 minutes.

Electrofusion may be used for cell fusion to prepare monoclonal antibody-producing cells.

Hybridoma can be selected by a method known per se or a method according thereto. Generally, it can be selected in a medium for animal cells supplemented with HAT (hypoxanthine, aminopterin, thymidine). Any medium for the selection and breeding can be used as far as the hybridoma can grow therein. For example, an RPMI 1640 medium comprising 1 to 20%, preferably 10 to 20%, fetal calf serum, a GIT medium (Wako Pure Chemical Industries, Ltd.) comprising 1 to 10% fetal calf serum, a serum free medium for hybridoma culture (SFM-101, Nissui Seiyaku Co., Ltd.) and the like can be used. Cultivation temperature is normally 20 to 40°C, preferably about 37°C. Cultivation time is normally 5 days to 3 weeks, preferably 1 week to 2 weeks. The cultivation can be performed normally in the presence of 5% gaseous carbon dioxide.

For screening monoclonal antibody-producing hybridomas, various methods can be used; for example, a method wherein a hybridoma culture supernatant is added to a solid phase (e.g., microplates) having a protein antigen or protein-expressing cells adsorbed directly thereto or along with a carrier, then an anti-immunoglobulin antibody (for example, anti-mouse immunoglobulin antibody is used in cases where the splenocytes used for cell fusion are from a mouse) or protein A, labeled with a radioactive substance, enzyme or the like, is added, and the monoclonal antibody bound to the solid phase is detected, a method wherein a hybridoma culture supernatant is added to a solid phase having an anti-immunoglobulin antibody or protein A adsorbed thereto, a protein labeled with a radioactive substance, enzyme or the like is added, and the monoclonal antibody bound to the solid phase is detected, and the like can be mentioned.

### (b) Preparation of monoclonal antibody by other methods

Preparation of the antibody I of the present invention is not limited to the method described in (a); for example, what is called the antibody display technique, wherein an antibody gene library prepared from human or warm-blooded animal (for example, monkey, rabbit, dog, guinea pig, mouse, rat, sheep, goat, camel, chicken and the like) B lymphocytes by a commonly known method is presented on cell surfaces of bacteriophages, Escherichia coli, yeast, animal cells and the like, or on ribosome and the like, can be used [Nature Biotechnology 23, 1105 (2005)]. The human or warm-blooded animal may be naive, and may also be a cancer patient with high expression of the antigen I of the present invention or a warm-blooded animal immunized with the antigen I of the present invention by the method described in (a). The form of the antibody to be presented to cell surfaces is exemplified by, but not limited to, the IgG molecule, IgM molecule, Fab fragment, single-chain Fv (scFv) fragment and the like.

The gene for a monoclonal antibody (fragment) that specifically binds to the antigen I of the present invention is obtained by reacting antibody (fragment)-presenting cells or antibody (fragment)-presenting ribosome carrying the above-described antibody gene library with the antigen I of the present invention for a given time, washing away the non-specifically binding portion, thereafter eluting and recovering the portion that binds specifically to the antigen I of the present invention, allowing the antibody (fragment)-presenting cells or antibody (fragment)-presenting ribosome to grow by a commonly known method, thereafter repeating this procedure several times, and finally isolating the desired product from the cloned antibody (fragment)-presenting cells or antibody (fragment)-presenting ribosome by a commonly known method. The monoclonal antibody fragment gene thus obtained can be recombined with the corresponding region of the IgG antibody gene by a commonly known method to obtain a monoclonal IgG antibody gene.

The antibody I of the present invention can also be obtained by immunizing antibody-producing cells isolated from a human or the above-described warm-blooded animal with the antigen I of the present invention in vitro by a method known per se, and thereafter preparing a hybridoma in the same manner as (a).

### (c) Production of monoclonal antibody

The monoclonal antibody I of the present invention can be produced by culturing the monoclonal antibody-producing hybridoma obtained in (a), or a recombinant cell line wherein an antibody gene isolated by a commonly known method from the monoclonal antibody-producing hybridoma obtained in (a) or the monoclonal antibody gene obtained in (b) is artificially expressed. The monoclonal antibody I of the present invention can also be produced by inserting the antibody gene in a warm-blooded animal or plant chromosome by a commonly known method, and allowing the antibody I to be produced in warm-blooded animal blood, milk, or eggs, plants, fungi and the like [Curr. Opin. Biotevhnol. 7, 536 (1996), Nature Rev. Genet 4, 794 (2003), Appl. Environ. Microbiol. 70, 2567 (2004)]. Useful warm-blooded animals include, for example, bovine, goat, sheep, pigs, chicken, mice, rabbits and the like. Useful plants include tobacco, corn, potato, duckweed and the like.

The monoclonal antibody I of the present invention can be purified from the above-described monoclonal antibody-containing material by a method known per se, for example, a method of immunoglobulin separation and purification [e.g., salting-out, alcohol precipitation, isoelectric point precipitation, electrophoresis, absorption-desorption using an ion exchanger (e.g., DEAE) or a hydrophobicity column, ultracentrifugation, gel filtration, affinity purification for separating and purifying only an antibody by means of a carrier wherein an antigen or a substance with affinity for the antibody, such as protein A or protein G, has been immobilized].

### (3) Preparation of polyclonal antibody

The polyclonal antibody I of the present invention can be produced according to a method know per se or a method based thereon. For example, the polyclonal antibody can be produced by immunizing the antigen I of the present invention or a complex of the antigen and a carrier protein to a warm-blooded animal in the same manner as the above-described method of producing a monoclonal antibody, collecting a product containing an antibody to the antigen from the immunized animal, and separating and purifying the antibody.
Regarding the complex of the immune antigen and carrier protein used to immunize a warm-blooded animal, any type of carrier protein and any mixing ratio of the carrier and antigen can be used, as long as an antibody against the antigen used for immunization as crosslinked to the carrier is efficiently produced; for example, a method wherein bovine serum albumin, bovine thyroglobulin or the like is crosslinked in a ratio of about 0.1 to 20, preferably about 1 to 5, parts by weight to 1 part by weight of the hapten, is used.
Various condensing agents can be used for coupling the antigen and carrier protein; glutaraldehyde, carbodiimide, maleimide activated ester, activated ester reagents containing a thiol group or dithiopyridyl group, and the like can be used.
The condensation product is administered to a warm-blooded animal as is or along with a carrier or a diluent to a site permitting antibody production. In order to increase antibody productivity during the administration, complete Freund's adjuvant or incomplete Freund's adjuvant may be administered. The administration is usually made about every 2 to 6 weeks about 3 to 10 times in total.
The polyclonal antibody can be collected from blood, ascites fluid, breast milk, egg and the like, of a warm-blooded animal immunized by the above-described method.
The polyclonal antibody titer in antiserum can be measured in the same manner as the measurement of the antibody titer of the antiserum described above. Separation and purification of the polyclonal antibody can be performed according to the same method of immunoglobulin separation and purification as the above-described separation and purification of a monoclonal antibody.

A nucleic acid comprising a base sequence complementary to the target region of a desired nucleic acid, i.e., a nucleic acid capable of hybridizing with a desired nucleic acid, can be described as being 'antisense' against the desired nucleic acid. Meanwhile, a nucleic acid comprising a base sequence having a homology to the target region of a desired nucleic acid can be described as being 'sense' against the desired nucleic acid. Here, 'having a homology' or 'being complementary' refers to having an identity or complementarity of about 70% or more, preferably about 80% or more, more preferably about 90% or more, most preferably about 95% or more, between base sequences.

A nucleic acid comprising a base sequence complementary to the base sequence that encodes RET or a portion thereof (hereinafter, also referred to as 'antisense RET' or 'the antisense nucleic acid I of the present invention') can be designed and synthesized on the basis of the base sequence information on a cloned or determined nucleic acid that encodes RET. Such a nucleic acid is capable of inhibiting the replication or expression of the gene that encodes RET. Specifically, antisense RET is capable of hybridizing with the RNA transcribed from the gene that encodes RET, and inhibiting the synthesis (processing) or function (translation into protein) of mRNA.

The target region of antisense RET is not particularly limited in its length as long as the translation into RET protein is inhibited as a result of hybridization of an antisense nucleic acid, and the region may be the whole sequence or a partial sequence of the mRNA that encodes the protein, which can be exemplified by a short strand of about 15 bases and a long strand of the whole mRNA or early transcription product. In consideration of the ease of synthesis and the issue of antigenicity, an oligonucleotide consisting of about 15 to 30 bases is preferable, but this is not to be construed as limiting. Specifically, for example, the 5'-end hairpin loop, 5'-end 6-base-pair repeat, 5'-end untranslated region, translation initiation codon, protein coding region, translation termination codon, 3'-end untranslated region, 3'-end palindrome region, and 3'-end hairpin loop of the nucleic acid that encodes RET can be chosen as the target region, and any region in the gene that encodes RET can be chosen as the target. For example, the intron portion of the gene is preferably used as the target region.
Furthermore, antisense RET may be one that is not only capable of hybridizing with the mRNA or early transcription product that encodes RET to inhibit the translation into protein, but also capable of binding to the gene that encodes RET which is a double-stranded DNA to form a triplex and inhibit the transcription of RNA.

Examples of the antisense nucleic acid include deoxyribonucleotides containing 2-deoxy-D-ribose, ribonucleotides containing D-ribose, other types of nucleotides which are N-glycosides of the purine or pyrimidine base, or other polymers having non-nucleotide backbones (for example, commercially available protein nucleic acids and synthetic sequence-specific nucleic acid polymers) or other polymers containing special linkages (provided that the polymers contain nucleotides having such a configuration that allows base pairing or base stacking, as is found in DNA or RNA) and the like. The antisense nucleic acid may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates and the like) and those with charged linkages or sulfur-containing linkages (e.g., phosphorothioates, phosphorodithioates and the like), those having side chain groups such as proteins (nucleases, nuclease inhibitors, toxins, antibodies, signal peptides, poly-L-lysine and the like), saccharides (e.g., monosaccharides, and the like), those with intercalators (e.g., acridine, psoralen and the like), those containing chelators (e.g., metals, radioactive metals, boron, oxidative metals and the like), those containing alkylating agents, those with modified linkages (e.g., α anomeric nucleic acids and the like), and the like. Herein the terms 'nucleoside', 'nucleotide' and 'nucleic acid' are used to refer to moieties that contain not only the purine and pyrimidine bases, but also other heterocyclic bases, which have been modified. These modifications may include methylated purines and pyrimidines, acylated purines and pyrimidines or other heterocyclic rings. Modified nucleotides and modified nucleotides may also have modifications on the sugar moiety thereof, wherein, for example, one or more hydroxyl groups may optionally be substituted with a halogen atom, an aliphatic group, and the like, or may be converted to functional groups such as ether or amine.

Preferably, the antisense nucleic acid is an optionally modified RNA or DNA. Specific examples of the modified nucleic acid (RNA, DNA) include, but are not limited to, those resistant to degradation such as sulfur derivatives, thiophosphate derivatives of nucleic acids, polynucleosideamide and oligonucleosideamide. Antisense RET can preferably be designed with the following aims. Specifically, antisense nucleic acid in cells is further stabilized, the cell permeability of antisense nucleic acid is increased, affinity for target sense strand is increased, and, the toxicity, if any, of antisense nucleic acid is reduced. Many such modifications are known in the art, and are disclosed in, for example, J. Kawakami et al., Pharm Tech Japan, Vol.8, pp.247, 1992; Vol.8, pp.395, 1992; S.T. Crooke et al. ed., Antisense Research and Applications, CRC Press, 1993 and elsewhere.

The antisense nucleic acid may contain altered or modified sugars, bases or linkages, and can be provided in a specialized form such as liposomes or microspheres, or can be applied to gene therapy, or can be provided in combination with attached moieties. Such attached moieties include polycations such as polylysine that act as charge neutralizers of the phosphate backbone, or hydrophobic moieties such as lipids (for example, phospholipids, cholesterols and the like) that enhance the interaction with cell membranes or increase uptake of the nucleic acid. Preferred lipids to be attached are cholesterols or derivatives thereof (for example, cholesteryl chloroformate, cholic acid and the like). These moieties can be attached to the nucleic acid at the 3' or 5'-end thereof and can also be attached thereto via a base, sugar, or intramolecular nucleoside linkage. Other moieties may be capping groups specifically placed at the 3' or 5'-end of the nucleic acid to prevent degradation by nucleases such as exonuclease and RNase. Such capping groups include, but are not limited to, hydroxyl protecting groups known in the art, including glycols such as polyethylene glycol and tetraethylene glycol.

A ribozyme capable of specifically cleaving an RNA (mRNA or early transcription product and the like) that encodes RET in the coding region (in case of early transcription product, the intron portion is included) can also be included in the antisense RET. 'Ribozyme' refers to an RNA having an enzyme activity for nucleic acid cleavage; however, since it has recently been demonstrated that an oligo-DNA having the base sequence of the enzyme activity site also has such nucleic acid cleavage activity, this term is used herein as including DNA, as long as it has sequence-specific nucleic acid cleavage activity. The most versatile ribozyme is self-splicing RNA, which is found in infectious RNAs such as viroid and virusoid, and is known in the hammerhead type, hairpin type and the like. The hammerhead type exhibits enzyme activity with about 40 bases, and it is possible to specifically cleave only a target mRNA by rendering several bases at both ends adjacent to the hammerhead structure portion (about 10 bases in total) complementary to the desired cleavage site of mRNA. Because this type of ribozyme has RNA as the only substrate, the same has a further advantage that genomic DNA is never targeted. When RET mRNA assumes a double-stranded structure per se, the target sequence can be rendered single-stranded by using a hybrid ribozyme coupled with an RNA motif derived from a viral nucleic acid capable of binding specifically to RNA helicase [Proc. Natl. Acad. Sci. USA, 98(10): 5572-5577 (2001)]. Furthermore, when ribozyme is used in the form of an expression vector comprising the DNA that encodes the same, the ribozyme may be a hybrid ribozyme further coupled with a sequence of altered tRNA to promote the transfer of the transcription product to cytoplasm [Nucleic Acids Res., 29(13): 2780-2788 (2001)].

A double-stranded oligo-RNA (siRNA) (siRNA against an RNA that encodes RET) having a base sequence complementary to a partial sequence in the coding region of an RNA (mRNA or early transcription product and the like) that encodes RET (in case of early transcription product, the intron portion is included) can also be included in antisense RET. The phenomenon of so-called RNA interference (RNAi), in which introducing short double-stranded RNA into a cell results in the degradation of a mRNA complementary to one of the chains of the RNA, is known to occur in nematodes, insects, plants, and the like, but since it was confirmed that this phenomenon also occurs in mammalian cells [Nature, 411 (6836): 494-498 (2001)], it has been widely used as an alternative to ribozyme.

The antisense nucleic acid I of the present invention can be prepared by determining a target region of mRNA or early transcription product on the basis of the information of a cDNA sequence or a genomic DNA sequence that encodes RET, and synthesizing a sequence complementary thereto using a commercially available DNA/RNA synthesizer (Applied Biosystems, Beckman and the like). siRNA having an RNAi activity can be prepared by synthesizing a sense strand and an antisense strand respectively with the DNA/RNA automatic synthesizer, denaturing in a suitable annealing-buffer solution at, for example, about 90°C to 95°C for about 1 minute, and annealing at about 30°C to 70°C for about 1 to 8 hours. In addition, a longer double-stranded polynucleotide can be prepared by synthesizing complementary oligonucleotide strands in an alternately overlapping manner, annealing the oligonucleotides, and ligating with ligase.

The gene expression inhibitory activity of antisense RET can be examined using a transformant containing a nucleic acid that encodes RET, an in vivo or in vitro RET-encoding-gene expression system or an in vivo or in vitro RET translation system.

The above-described substances that inhibit a function (for example, RET activity and expression) of RET, such as the antibody I of the present invention and the antisense nucleic acid I of the present invention, have, for example, the following uses.

As shown in an Example below, by allowing GDNF to act on cancer cells (for example, breast cancer cells), cell growth is promoted, and this cell growth is suppressed by siRNA against RET. This fact shows that the growth of a cancer cells (for example, breast cancer cells) is promoted due to GDNF/RET signal activation, and a substance capable of inhibiting an activity or expression of RET inhibits the growth of a cancer cells (for example, breast cancer cells), and is effective in the prophylaxis/treatment of cancers (for example, breast cancer).

Because the antibody I of the present invention is capable of inhibiting RET activity by binding specifically to RET, and also because the antisense nucleic acid I of the present invention is capable of inhibiting RET expression, it is possible to inhibit an activity or expression of RET in cancer cells by administering the antibody I of the present invention to a cancer (for example, breast cancer) patient, or administering the antisense nucleic acid I of the present invention to a patient to introduce (and express) the same into target cells, to thereby inhibit the growth of the cancer cells, and prevent/treat cancers.

As shown in an Example below, RET is expressed on the surface of cancer cells (for example, breast cancer cells). Therefore, the antibody I of the present invention is also capable of killing cancer cells and preventing/treating cancers by binding to RET on the cancer cell surface, and inducing antibody-dependent cellular cytotoxicity (ADCC) or complement-dependent cytotoxicity (CDC).

Therefore, a pharmaceutical comprising the above-described substance that inhibits a function of RET (for example, RET activity and expression), such as a) the antibody I of the present invention or b) the antisense nucleic acid I of the present invention, can be used as, for example, a prophylactic/therapeutic agent for cancers (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like) (preferably, a prophylactic/therapeutic agent for breast cancer), a cancer cell apoptosis promoter, a cancer cell (preferably, breast cancer cells) growth inhibitor, cancer cell cycle alteration inducer, cancer metastasis suppressant, cancer cell adhesion inhibitor and the like.

When the antibody I of the present invention is used as the above-described prophylactic/therapeutic agent and the like, the antibody can be prepared as a pharmaceutical preparation in accordance with a conventional method.
When the antisense nucleic acid I of the present invention is used as the above-described prophylactic/therapeutic agent and the like, the nucleic acid, as is or after being inserted into an appropriate expression vector such as retrovirus vector, adenovirus vector, or adenovirus associated virus vector in a functional way, can be prepared as a pharmaceutical preparation in accordance with a conventional method. The nucleic acid can be administered as is, or along with an auxiliary for promoting its ingestion, using a gene gun or a catheter such as a hydrogel catheter.

A pharmaceutical comprising a substance that inhibits a function of RET (for example, RET activity and expression), such as the antibody I of the present invention or the antisense nucleic acid I of the present invention, is of low toxicity and can be administered in the form of liquid preparations as they are, or as pharmaceutical compositions in suitable dosage forms, to human or non-human mammals (e.g., rats, rabbits, sheep, pigs, bovine, cats, dogs, monkeys and the like), orally or parenterally (e.g., intravascularly, subcutaneously and the like).

The substance that inhibits a function of RET (e.g., RET activity and expression) such as the antibody I or the antisense nucleic acid I and the like of the present invention may be administered as is, or may be administered as an appropriate pharmaceutical composition. The pharmaceutical composition used for the administration may comprise the antibody I of the present invention or the antisense nucleic acid I of the present invention and a pharmacologically acceptable carrier, diluent or filler. Such a pharmaceutical composition is provided as a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. The injection can be prepared by, for example, dissolving, suspending or emulsifying the antibody I of the present invention or the antisense nucleic acid I of the present invention in a sterile aqueous or oily solution normally used for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50mol) adduct of hydrogenated castor oil)] and the like. As examples of oily solutions, sesame oil, soybean oil and the like can be used, which may be used in combination with solubilizers such as benzyl benzoate, benzyl alcohol. The injectable preparation prepared is preferably filled in an appropriate ampoule. A suppository used for rectal administration may also be prepared by mixing the above-described antibody or the antisense nucleic acid in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a commonly known method, and may contain a carrier, diluent or filler normally used in the field of pharmaceutical making. As the carrier or filler for tablets, for example, lactose, starch, sucrose, and magnesium stearate are used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. As the content amount of the antibody, it is preferable that normally 5 to 500 mg, particularly 5 to 100 mg for injections or 10 to 250 mg for other dosage forms, per medication unit dosage form, of the above-described antibody be contained. Regarding the content of antisense nucleic acid, it is preferable that the above-described antisense nucleic acid be contained at normally 5 to 500 mg, particularly 5 to 100 mg for an injection, or 10 to 250 mg for other dosage forms, per unit dosage form.

The dosage of the above-described prophylactic/therapeutic agents and the like comprising the antibody I of the present invention varies also depending on the subject of administration, target disease, symptoms, route of administration and the like; for example, when the agent is used for the treatment/prevention of breast cancer in an adult, the antibody I of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

The dosage of the above-described prophylactic/therapeutic agents and the like comprising the antisense nucleic acid I of the present invention varies also depending on the subject of administration, target disease, symptoms, route of administration and the like; for example, when the agent is used for the treatment/prevention of breast cancer in an adult, the antisense nucleic acid I of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

Each of the foregoing compositions may contain another active ingredient, as long as no undesirable interaction is produced when blended with the above-described antibody or antisense nucleic acid.

Furthermore, the substance inhibiting the function of RET (e.g., RET activity and expression) such as the antibody I or the antisense nucleic acid I of the present invention may be used in combination with other drugs, for example, alkylating agents (e.g., cyclophosphamide, ifosfamide and the like), metabolic antagonists (e.g., methotrexate, 5-fluorouracil and the like), anticancer antibiotics (e.g., mitomycin, adriamycin and the like), plant-derived anticancer agents (e.g., vincristine, vindesine, Taxol and the like), cisplatin, carboplatin, ethopoxide, irinotecan and the like. The antibody I of the present invention or antisense nucleic acid I of the present invention and the above-mentioned drugs may be administered to a patient simultaneously or at different times.

Because the antibody I of the present invention specifically recognizes RET, and can be used for quantitation of RET in a test liquid, particularly for quantitation by sandwich immunoassay and the like, the same is useful as, for example, a diagnostic reagent for decreased expression or increased expression of the protein and the like. As shown in an Example below, cancer cells (for example, breast cancer cells) express RET, and undergo the action of GDNF, whereby cell growth is promoted; when cancer cells are treated with siRNA against RET to suppress the amount expressed, the growth of the cancer cells is suppressed. Therefore, by detecting and quantifying RET in a test sample such as cells, tissue, or body fluid using the antibody I of the present invention, cancers (for example, breast cancer), particularly cancers that are highly sensitive to GDNF (for example, breast cancer) can be detected. Hence, the antibody I of the present invention is useful as a diagnostic reagent for cancers (for example, breast cancer). For example, by quantifying RET in the sample using the antibody I of the present invention, when an increase in the expression of RET is detected, the subject can be diagnosed as having, for example, a cancer (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like, particularly breast cancer), or as being likely to suffer from a cancer in the future. Furthermore, by quantifying the expression of RET in cancer cells, the GDNF sensitivity of the cancer can be determined. If an increase in the expression of RET in cancer cells is detected, the cancer can be judged to be a cancer that is highly sensitive to GDNF, and grows vigorously GDNF-dependently.

As examples of the method of RET quantitation using the antibody I of the present invention,
(i) a method of quantifying RET in a test liquid, comprising competitively reacting the antibody I of the present invention, a test liquid and a labeled form of RET, and determining the ratio of labeled RET bound to the antibody,
(ii) a method of quantifying RET in a test liquid, comprising simultaneously or sequentially reacting a test liquid, the antibody I of the present invention insolubilized on a carrier and another antibody I of the present invention which has been labeled, and thereafter determining the activity of the labeling agent on the insolubilizing carrier and the like can be mentioned.

In the method of quantitation (ii) above, the two kinds of antibodies are desirably ones that specifically recognize different portions of RET. For example, provided that one antibody is an antibody that recognizes the N-terminus of RET, the other antibody can be an antibody that reacts with the C-terminus of RET.

As the labeling agent used for the assay methods using a labeled substance, a radioisotope, an enzyme, a fluorescent substance, a luminescent substance and the like are used. As the radioisotope, for example, [¹²⁵I] ,[¹³¹I], [³H], [¹⁴C] and the like are used; as the above-described enzyme, stable enzymes with a high specific activity are preferable; for example, P-galactosidase, β-glucosidase, alkaline phosphatase, peroxidase, malate dehydrogenase and the like are used; as examples of the fluorescent substance, cyanine fluorescent dyes (e.g., Cy2, Cy3, Cy5, Cy5.5, Cy7 (manufactured by Amersham Biosciences K.K.) and the like), fluorescamine, fluorescein isothiocyanate and the like are used; as examples of the luminescent substance, luminol, luminol derivatives, luciferin, lucigenin and the like are used. Furthermore, a biotin-avidin system can also be used for the binding of the antibody or antigen and the labeling agent.

As the test liquid, when RET is localized in cells, a cell homogenate obtained by suspending the cells in an appropriate buffer, and then breaking the cells by ultrasonication, freeze-thaw cycling and the like, is used, and when RET is secreted extracellularly, a cell culture supernatant or a body fluid (blood, serum, plasma, urine, sweat, breast milk and the like) is used. If necessary, the quantification may be carried out after separating and purifying RET from a homogenate, a cell-culture supernatant or a body fluid and the like. In addition, intact cells can be used as the sample, as long as label detection is possible.

The method of quantitating RET using the antibody I of the present invention is not to be particularly limited; any assay may be used, as long as it is an assay wherein the amount of antibody, antigen or antibody-antigen complex corresponding to the amount of antigen in the subject liquid (for example, protein content) is detected by a chemical or physical means, and this is applied to a standard curve generated using standard solutions containing known amounts of antigen to calculate the RET content. For example, nephelometry, the competitive method, the immunometric method, and the sandwich method are suitably used; in terms of sensitivity and specificity, it is particularly preferable to use the sandwich method described below.

For insolubilization of the antigen or antibody, physical adsorption may be used, and chemical binding methods conventionally used to insolubilize or immobilize proteins, enzymes and the like may be used as well. As examples of the carrier, insoluble polysaccharides such as agarose, dextran, and cellulose; synthetic resins, for example, polystyrene, polyacrylamide, silicon and the like, or glass and the like can be mentioned.

In the sandwich method, the antibody I of the present invention insolubilized is reacted with a test liquid (primary reaction), then reacted with the antibody I of the present invention labeled (secondary reaction), after which the activity of the labeling agent on the insolubilizing carrier is measured, whereby the amount of the protein I used in the present invention in the test liquid can be quantified. The primary and secondary reactions may be performed simultaneously or with a time lag. The labeling agent and the method for insolubilization can be the same as those described above. In the immunoassay by the sandwich method, the antibody used for the solid phase or the antibody for labeling is not necessarily from one kind, but a mixture of two or more kinds of antibodies may be used for increasing the measurement sensitivity and other purposes.

In the method of measuring RET by the sandwich method, the antibody I of the present invention used in the primary reaction and that used in the secondary reaction are preferably antibodies having different sites for RET binding. Hence, for example, provided that the antibody used in the secondary reaction recognizes the C-terminus of RET, the antibody used in the primary reaction is preferably an antibody that recognizes a site other than the C-terminus, for example, the N-terminus.

The antibody I of the present invention can be used in measuring systems other than the sandwich method, for example, the competitive method, the immunometric method, nephelometry, and the like.

In the competitive method, an antigen in a test liquid and a labeled form of antigen are reacted competitively against an antibody, an unreacted labeled antigen (F) is separated from an antibody-bound labeled antigen (B) (B/F separation), and the labeled amount of B or F is determined, thereby to quantify the antigen in the test liquid. The present reaction method includes a liquid phase method in which B/F separation is performed using a soluble antibody as the antibody and using polyethylene glycol or a secondary antibody against the antibody and the like; and a solid phase method in which a solid-phased antibody is used as a primary antibody or a soluble antibody is used as a primary antibody and a solid-phased antibody is used as a secondary antibody.

In the immunometric method, the antigen in a test liquid and a solid-phase-immobilized antigen are competitively reacted with a given amount of the antibody of the present invention labeled, after which the solid phase and the liquid phase are separated, or the antigen in the test liquid and an excess amount of the antibody of the present invention labeled are reacted, and then a solid-phase-immobilized antigen is added to bind the unreacted portion of the antibody of the present invention labeled to the solid phase, after which the solid phase and the liquid phase are separated. Next, the amount of labeling agent in either phase is measured to quantify the amount of antigen in the test liquid.

Also, in nephelometry, the amount of insoluble precipitate resulting from an antigen-antibody reaction in the gel or in the solution is measured. Even when the amount of antigen in the test solution is small and only a small amount of precipitate is obtained, laser nephelometry, which utilizes laser scattering, and the like are preferably used.

Using the antibody I of the present invention, RET can be quantified, and can also be detected by tissue staining and the like. For these purposes, the antibody molecule itself may be used, and the F(ab')₂, Fab', or Fab fraction of the antibody molecule may also be used.

In applying these individual immunological measurement methods to the method I of the present invention, it is unnecessary to set special conditions, procedures and the like. Making ordinary technical considerations for those skilled in the art to the ordinary conditions and procedures in each method, a measurement system of RET can be constructed. For details of these general technical means, compendia, books and the like can be referred to.

For example, see edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.

As described above, the RET can be sensitively quantified by the antibody I used in the present invention.

The antibody I of the present invention can be used for preparing an antibody column for purification of RET, detecting RET in each fraction during purification, analyzing the behavior of RET in test cells and for other purposes.

Because a nucleic acid comprising the base sequence that encodes RET or a portion thereof (hereinafter, also referred to as 'sense-RET'), or a nucleic acid comprising a base sequence complementary to the base sequence or a portion thereof (antisense RET) is capable of detecting an abnormality in the RET-encoding DNA or mRNA (gene abnormality) in a human or other warm-blooded animal (for example, rats, mice, hamsters, rabbits, sheep, goat, pigs, bovine, horses, cats, dogs, monkeys, chimpanzees, birds and the like), when used as a probe and the like, the same is useful as, for example, a gene diagnostic reagent for damage or mutation in the DNA, splicing abnormality or decreased expression in mRNA, or amplification in the DNA, increased expression in mRNA and the like. The nucleic acid comprising a portion of the base sequence that encodes RET is not particularly limited, as long as it has a length sufficient for a probe (for example, about 15 bases or more), and does not need to encode a partial peptide of RET.
The above-described gene diagnosis using sense or antisense RET can be performed by, for example, Northern hybridization, quantitative RT-PCR, PCR-SSCP assay, allele-specific PCR, PCR-SSOP assay, DGGE assay, RNase protection assay, PCR-RFLP assay and the like that are known per se .
As shown in an Example below, cancer cells (for example, breast cancer cells) express RET, and undergo the action of GDNF, whereby cell growth is promoted; when cancer cells are treated with siRNA against RET to suppress the amount expressed, the growth of the cancer cells is suppressed. Therefore, by detecting and quantifying RET in a test sample such as cells, tissue, or body fluid using sense or antisense RET, cancers (for example, breast cancer), particularly cancers that are highly sensitive to GDNF (for example, breast cancer) can be detected. Hence, sense or antisense RET is useful as a diagnostic reagent for cancers (for example, breast cancer). For example, by quantifying the expression of RET in the sample using a sense or antisense RET, when an increase in the expression of RET is detected, the subject can be diagnosed as having a cancer (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like, particularly breast cancer), or as being likely to suffer from a cancer in the future. Furthermore, by quantifying the expression of RET in cancer cells, the GDNF sensitivity of the cancer can be determined. If an increase in the expression of RET in cancer cells is detected, the cancer can be judged to be a cancer that is highly sensitive to GDNF, and grows vigorously GDNF-dependently.

### (II. Anti-GDNF antibody and the like)

A protein comprising the same or substantially the same amino acid sequence as the amino acid sequence shown by SEQ ID NO:5 (hereinafter, sometimes abbreviated 'GDNF protein isoform 1'), a protein comprising the same or substantially the same amino acid sequence as the amino acid sequence shown by SEQ ID NO:7 (hereinafter, sometimes abbreviated 'GDNF protein isoform 2') or a protein comprising the same or substantially the same amino acid sequence as the amino acid sequence shown by SEQ ID NO:9 (hereinafter, sometimes abbreviated 'GDNF protein isoform 3') (hereinafter, these three are sometimes together referred to as 'GDNF' or 'the protein II of the present invention') may be a protein derived from human or warm-blooded animal (for example, guinea pigs, rat, mice, chicken, rabbits, pigs, sheep, bovine, monkeys and the like) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells (e.g., breast cancer cells) and the like]; or any tissues where such cells are present, for example, brain and various parts of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, and the like, and may be a synthetic protein.

The amino acid sequence which is substantially the same amino acid sequence as that represented by SEQ ID NO: 5, SEQ ID NO: 7 or SEQ ID NO: 9, includes amino acid sequences having about 50% or more homology, preferably about 60% homology or more, more preferably about 70% or more homology, even more preferably about 80% or more homology, particularly preferably about 90% or more homology and most preferably about 95% or more homology, to the amino acid sequence shown by SEQ ID NO: 5, SEQ ID NO: 7 or SEQ ID NO: 9, and the like.

Preferable proteins comprising the same or substantially the same amino acid sequence as the amino acid sequence shown by SEQ ID NO:5, SEQ ID NO:7 or SEQ ID NO:9 include, for example, the above-described protein comprising substantially the same amino acid sequence as the amino acid sequence shown by SEQ ID NO:5, SEQ ID NO:7 or SEQ ID NO:9, and having substantially the same quality of activity as the amino acid sequence shown by SEQ ID NO:5, SEQ ID NO:7 or SEQ ID NO:9 and the like.

Herein, the 'homology' means a ratio (%) of the same amino acid and similar amino acid residue to the total overlapped amino acid residue, in the best alignment when two amino acid sequences are aligned with the use of a mathematical algorithm commonly known in the technical field (preferably, the algorithm considers introduction of gaps on one or both side of the sequence for the best alignment). The term 'similar amino acid' refers to an amino acid similar in its physiochemical properties, and the examples include amino acids classified in a same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid including a hydroxyl group (Ser, Thr), amino acid having a short side chain(Gly, Ala, Ser, Thr, Met), and the like. A substitution by such similar amino acid is expected to give no change in the phenotype of protein (thus is a conservative amino acid substitution). A specific example of the conservative amino acid substitution is well-known in the technical field, and is disclosed in various documents (for example, refer Bowie et al, Science, 247: 1306-1310 (1990)).
Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

As the substantially equivalent activity described above, there are, for example, an activity to promote proliferation of cancer cells (e.g., breast cancer cells), and the like. The substantially equivalent is used to mean that the nature of the activities is equivalent in terms of quality (e.g., physiologically or pharmacologically). Thus, the level of activities of the protein II of the present invention are preferably equivalent to those of a protein having an amino acid sequence represented by SEQ ID NO: 5, SEQ ID NO: 7 or SEQ ID NO: 9 (e.g., about 0.01 to 100 times, preferably about 0.1 to 10 times, more preferably 0.5 to 2 times), but differences in quantitative factors such as a level of these activities, a molecular weight of the protein, and the like may be present and allowable.
A measurement of the activity of GDNF can be performed in accordance with a method known per se. For example, as described in an Example below, by measuring cell growth when cancer cells (e.g., breast cancer cells) are stimulated with GDNF, the activity can be evaluated.

Examples of GDNF include what are called muteins of proteins comprising (i) an amino acid sequence having 1 or 2 or more (e.g., about 1 to 50, preferably about 1 to 30, more preferably about 1 to 10, still more preferably several (1 to 5)) amino acids deleted from the amino acid sequence shown by SEQ ID NO:5, SEQ ID NO:7 or SEQ ID NO:9, (ii) an amino acid sequence having 1 or 2 or more (e.g., about 1 to 50, preferably about 1 to 30, more preferably about 1 to 10, still more preferably several (1 to 5)) amino acids added to the amino acid sequence shown by SEQ ID NO:5, SEQ ID NO:7 or SEQ ID NO:9, (iii) an amino acid sequence having 1 or 2 or more (e.g., about 1 to 50, preferably about 1 to 30, more preferably about 1 to 10, still more preferably several (1 to 5)) amino acids inserted in the amino acid sequence shown by SEQ ID NO:5, SEQ ID NO:7 or SEQ ID NO:9, (iv) an amino acid sequence having 1 or 2 or more (e.g., about 1 to 50, preferably about 1 to 30, more preferably about 1 to 10, still more preferably several (1 to 5)) amino acids substituted by other amino acids in the amino acid sequence shown by SEQ ID NO:5, SEQ ID NO:7 or SEQ ID NO:9, or (v) an amino acid sequence comprising a combination thereof., and the like. The protein preferably has substantially the same quality of activity as a protein having the amino acid sequence shown by SEQ ID NO:5, SEQ ID NO:7 or SEQ ID NO:9.
When an amino acid sequence is inserted, deleted or substituted as described above, the position of the insertion, deletion or substitution is not subject to limitation.

For the proteins mentioned herein, the left end indicates the N-terminus (amino terminus) and the right end indicates the C-terminus (carboxyl terminus), according to the common practice of peptide designation. For the protein comprising the same amino acid sequence as that shown by SEQ ID NO:5 used in the present invention, the C-terminus may be any of a carboxyl group (-COOH), a carboxylate (-COO⁻), an amide (-CONH₂) or an ester (-COOR) .

Here, as R in the ester, a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl, a phenyl-C₁₋₂ alkyl group such as benzyl and phenethyl, a C₇₋₁₄ aralkyl group such as an α-naphthyl-C₁₋₂ alkyl group such as α-naphthylmethyl, a pivaloyloxymethyl group; and the like can be used.

When the GDNF has a carboxyl group (or a carboxylate) in addition to that on the C-terminal, one in which the carboxyl group is amidated or esterified is also included in the GDNF used in the present invention. In this case, as the ester, the above-described C-terminal ester and the like, for example, can be used.

Furthermore, the GDNF also includes a protein wherein the amino group of the N-terminal amino acid residue thereof (e.g., methionine residue) is protected by a protecting group (for example, a C₁₋₆ acyl group such as C₁₋₆ alkanoyl such as a formyl group or an acetyl group, and the like), a protein wherein the N-terminal glutamine residue, which is produced by cleavage in vivo, has been converted to pyroglutamic acid, a protein wherein a substituent (for example, -OH, -SH, an amino group, an imidazole group, an indole group, a guanidino group and the like) on an amino acid side chain in the molecule is protected by an appropriate protecting group (for example, a C₁₋₆ acyl group such as a C₁₋₆ alkanoyl group such as a formyl group or an acetyl group, and the like), a conjugated protein such as what is called a glycoprotein, which has a sugar chain bound thereto, and the like.

Specific examples of GDNF include a protein comprising the amino acid sequence shown by SEQ ID NO:5 (human GDNF protein isoform 1), a protein comprising the amino acid sequence shown by SEQ ID NO:7 (human GDNF protein isoform 2), a protein comprising the amino acid sequence shown by SEQ ID NO:9 (human GDNF protein isoform 3) and the like.

The partial peptide of GDNF may be any partial peptide of GDNF described above, preferably having substantially the same quality of activity as the above-described GDNF. Here, 'substantially the same quality of activity' is as defined above. A determination of 'substantially the same quality of activity' can be performed as described above. The partial peptide of GDNF preferably has immunogenicity.

For example, a peptide having at least 20 or more, preferably 50 or more, more preferably 70 or more, still more preferably 100 or more, most preferably 200 or more, amino acids of the constituent amino acids of the sequence of the GDNF and the like are used.

In addition, the partial peptide of the GDNF used in the present invention may have (1) 1 or 2 or more (preferably about 1 to 20, more preferably about 1 to 10, still more preferably several (1 to 5)) amino acids deleted from the amino acid sequence thereof, or (2) 1 or 2 or more (preferably about 1 to 20, more preferably about 1 to 10, still more preferably several (1 to 5)) amino acids added to the amino acid sequence thereof, or (3) 1 or 2 or more (preferably about 1 to 20, more preferably about 1 to 10, still more preferably several (1 to 5)) amino acids inserted in the amino acid sequence thereof, or (4) 1 or 2 or more (preferably about 1 to 20, more preferably about 1 to 10, still more preferably several, still yet more preferably about 1 to 5) amino acids substituted by other amino acids in the amino acid sequence thereof, or (5) a combination thereof.

For the partial peptide of the GDNF, the C-terminus may be any of a carboxyl group (-COOH), a carboxylate (-COO⁻), an amide (-CONH₂) or an ester (-COOR).
Furthermore, the partial peptide of the GDNF, like the foregoing GDNF, also includes a partial peptide wherein a carboxyl group (or carboxylate) is present at a position other than the C-terminus, a partial peptide wherein the amino group of the N-terminal amino acid residue (e.g., methionine residue) is protected by a protecting group, a partial peptide wherein glutamine residue, which is produced upon cleavage at the N-terminal in vivo, has been converted to pyroglutamic acid, a partial peptide wherein a substituent on a side chain of an amino acid in the molecule is protected by an appropriate protecting group, a conjugated peptide such as what is called a glycopeptide having a sugar chain bound thereto, and the like.

The length of such an immunogenic peptide is not particularly limited, as long as the peptide has immunogenicity; for example, one having 8, preferably 10, more preferably 12, continuous amino acid residues can be mentioned.

Useful salts of GDNF or a partial peptide thereof include salts with physiologically acceptable acids (e.g., inorganic acids, organic acids), bases (e.g., alkali metal salts) and the like, and physiologically acceptable acid addition salts are particularly preferable. Such salts include, for example, salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Useful substances that inhibit a function of GDNF or a partial peptide thereof or a salt thereof include,
(1) an antibody against GDNF or a partial peptide thereof or a salt thereof,
(2) a low-molecular compound that inhibits a function of GDNF or a partial peptide thereof or a salt thereof, or a salt thereof,
(3) an antisense nucleic acid against the nucleic acid that encodes GDNF or a partial peptide thereof, or
(4) an siRNA against RET that encodes GDNF or a partial peptide thereof, and the like.

Although the antibody against GDNF or a partial peptide thereof or a salt thereof (hereinafter, sometimes abbreviated 'the antibody II of the present invention') may be a polyclonal antibody or a monoclonal antibody, as long as it is an antibody capable of recognizing GDNF or a partial peptide thereof or a salt thereof, the antibody is preferably a monoclonal antibody. Although the isotype of the antibody is not particularly limited, it is preferably IgG, IgM or IgA. The antibody II of the present invention may be any of a mouse antibody, rat antibody, rabbit antibody, human antibody, humanized antibody, chimeric antibody thereof and the like. Alternatively, antibodies obtained by an antibody display method, such as the phage display method, using a non-human warm-blooded animal (e.g., rabbits, goat, bovine, chicken, mice, rats, sheep, pigs, horses, cats, dogs, monkeys, chimpanzees and the like) or human antibody gene library and the like can also be included in the antibody II of the present invention. The antibody II of the present invention is preferably human monoclonal antibody.

The antibody II of the present invention is not particularly limited with respect to molecular morphology, as long as it has at least a complementarity determining region (CDR) for specifically recognizing and binding to GDNF or a partial peptide thereof or a salt thereof; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like.

An antibody against GDNF or a partial peptide thereof or a salt thereof (hereinafter, in the explanation of antibodies, these are sometimes comprehensively abbreviated 'GDNFs') can be produced in accordance with a method of antibody or antiserum production known per se.

Described below are the method of preparing an antigen of the antibody II of the present invention, and the method of producing the antibody.

### (1) Preparation of antigen

As the antigen used to prepare the antibody II of the present invention, any of the above-described GDNFs (e.g., a protein comprising the amino acid sequence shown by SEQ ID NO:5, SEQ ID NO:7 or SEQ ID NO:9 (GDNF) or a partial peptide thereof or a salt thereof), or a (synthetic) peptide having 1 kind or 2 or more kinds of the same antigen determinant as GDNFs and the like can be used (hereinafter, these are also simply referred to as the antigen II of the present invention).

As specific examples of the antigen II of the present invention, a cell line that naturally or artificially highly expresses GDNFs or a membrane fraction thereof, or a (synthetic) peptide having 1 kind or 2 or more kinds of the same antigen determinant as GDNFs and the like can be mentioned.

The length of such a (synthetic) peptide is not particularly limited, as long as the peptide has immunogenicity; for example, one having 8, preferably 10, more preferably 12, continuous amino acid residues can be mentioned.

GDNF or a partial peptide thereof or a salt thereof can also be produced from the above-described human or warm-blooded animal cells or tissue by a method of protein purification known per se or a method based thereon, and can also be produced by culturing a transformant comprising a nucleic acid (DNA, RNA and the like) that encodes the protein. GDNF or a partial peptide thereof or a salt thereof can also be produced in accordance with the method of peptide synthesis described below.

(a) When the antigen II of the present invention is prepared from a human or warm-blooded animal (for example, guinea pig, rat, mouse, chicken, rabbit, pig, sheep, bovine, monkey and the like) tissue or cells, the tissue or cells may be homogenized, after which a crude fraction (e.g., membrane fraction, soluble fraction) can be used as the antigen as is. Alternatively, the antigen can be purified and isolated by performing extraction with an acid, surfactant or alcohol and the like, and applying the extract to a combination of salting-out, dialysis, gel filtration, chromatographies such as reversed-phase chromatography, ion exchange chromatography, and affinity chromatography. The antigen obtained may be used as the immunogen as is, and may also be subjected to limited degradation using a peptidase and the like to yield a partial peptide that can be used as the immunogen.

(b) When GDNFs are produced using a transformant comprising a nucleic acid that encodes the antigen II of the present invention, the nucleic acid can be prepared by a commonly known method of cloning [for example, Molecular Cloning (2nd ed.; J. Sambrook et al., Cold Spring Harbor Lab. Press, 1989)].

The nucleic acid that encodes GDNF or a partial peptide thereof may be any one comprising the base sequence that encodes the aforementioned amino acid sequence of GDNF used in the present invention or a partial amino acid sequence thereof. The nucleic acid may be DNA or RNA, or DNA/RNA chimera, and preferably is DNA. In addition, the nucleic acid may be a double-strand, or single-strand. The double-strand may include a double-stranded DNA, a double-stranded RNA, and DNA:RNA hybrid.

The DNA encoding GDNF or its partial peptide can be exemplified by genomic DNA, cDNA derived from human or other mammalian (e.g., simian, bovine, horse, swine, sheep, goat, rabbit, mouse, rat, guinea pig, hamster, chicken etc.) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells (e.g., breast tumor cells), etc.]; or any tissues or organs where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue), skeletal muscle, etc.], synthetic DNA, etc. As the RNA that encodes GDNF or a partial peptide thereof, mRNA (mature mRNA) or early transcription product and the like can be mentioned.

As the method of cloning a DNA that fully encodes GDNF or a partial peptide thereof, a method wherein the DNA is amplified by a PCR method using a synthetic DNA primer having a portion of the base sequence that encodes GDNF or a partial peptide thereof, a method wherein the desired DNA is selected from a cDNA library by a hybridization method using a DNA fragment or synthetic DNA that encodes a portion or entire region of GDNF as the probe, and the like can be mentioned.
The template polynucleotide used for the PCR may be any one comprising the base sequence that encodes GDNF or a partial peptide thereof; for example, genomic DNA, genomic DNA library, cDNA derived from the above-described cell/tissue, a cDNA library derived from the above-described cell/tissue, synthetic DNA and the like can be used. The hybridization can be carried out, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out more preferably under high stringent conditions.

The high stringent conditions used herein are, for example, those in a sodium concentration at about 19 to 40 mM, preferably about 19 to 20 mM at a temperature of about 50°C to 70°C, preferably about 60°C to 65°C. In particular, hybridization conditions in a sodium salt concentration at about 19 mM at a temperature of about 65°C are most preferred. Those skilled in the art can simply regulate the condition to a desired stringency by appropriately changing a concentration of hybridization solution, temperature of hybridization reaction, probe concentration, length of probe, number of mismatch, time for hybridization reaction, salt concentration of washing solution, temperature for washing, etc.

More specifically, as the nucleic acid (DNA and the like) that encodes GDNF, (i) a nucleic acid comprising the base sequence shown by SEQ ID NO:6 (the nucleic acid encodes a protein comprising the amino acid sequence shown by SEQ ID NO:5 (human GDNF protein isoform 1)), or the nucleic acid comprising a base sequence that hybridizes with the base sequence shown by SEQ ID NO:6 under high stringent conditions, and encoding a protein or peptide having substantially the same quality of activity as the above-described protein comprising the amino acid sequence shown by SEQ ID NO:5 and the like, (ii) a nucleic acid comprising the base sequence shown by SEQ ID NO:8 (the nucleic acid encodes a protein comprising the amino acid sequence shown by SEQ ID NO:7 (human GDNF protein isoform 2)), or a nucleic acid comprising a base sequence that hybridizes with the base sequence shown by SEQ ID NO:8 under high stringent conditions, and encoding a protein or peptide having substantially the same quality of activity as the above-described protein comprising the amino acid sequence shown by SEQ ID NO:7, iii) a nucleic acid comprising the base sequence shown by SEQ ID NO:10 (the nucleic acid encodes a protein comprising the amino acid sequence shown by SEQ ID NO:9 (human GDNF protein isoform 3)), or a nucleic acid comprising a base sequence that hybridizes with the base sequence shown by SEQ ID NO:10 under high stringent conditions, and encoding a protein or peptide having substantially the same quality of activity as the above-described protein comprising the amino acid sequence shown by SEQ ID NO:9, and the like are used.
Useful nucleic acids capable of hybridizing with the base sequence shown by SEQ ID NO:6 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:6 and the like.
Useful nucleic acids capable of hybridizing with the base sequence shown by SEQ ID NO:8 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO: 8.
Useful nucleic acids capable of hybridizing with the base sequence shown by SEQ ID NO:10 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:10.
Homology of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool).

The base sequence of the DNA can be converted according to a method known per se, such as the ODA-LA PCR method, the Gapped duplex method, or the Kunkel method, or a method based thereon, using PCR, a commonly known kit, for example, Mutant^{™}-super Express Km (Takara Bio Inc.), Mutan^{™}-K (Takara Bio Inc.) and the like.

The cloned DNA that encodes the GDNF or the partial peptide thereof can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codons can be added using an appropriate synthetic DNA adapter.

By transforming the host with an expression vector comprising a DNA that encodes the antigen II of the present invention, acquired as described above, and culturing the transformant obtained, the antigen II of the present invention can be produced.
An expression vector for the antigen II of the present invention can be produced by, for example, (a) cutting out a desired DNA fragment from the DNA that encodes the antigen II of the present invention, and (b) joining the DNA fragment downstream of a promoter in an appropriate expression vector.

Useful vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter used in the present invention may be any promoter appropriate for the host used to express the gene.
For example, when an animal cell is used as the host, the SRα promoter, the SV40 promoter, the LTR promoter, the CMV promoter, the HSV-TK promoter and the like can be mentioned. Of these promoters, the CMV (cytomegalovirus) promoter, the SRα promoter and the like are preferably used.

When the host is a bacterium of the genus Escherichia, the trp promoter, the lac promoter, the recA promoter, the λP_{L} promoter, the lpp promoter, the T7 promoter and the like are preferred. When the host is a bacterium of the genus Bacillus, the SPO1 promoter, the SPO2 promoter, the penP promoter and the like are preferred. When the host is yeast, the PHO5 promoter, the PGK promoter, the GAP promoter, the ADH promoter and the like are preferred. When the host is an insect cell, the polyhedrin promoter, the P10 promoter and the like are preferred.

Useful expression vectors include, in addition to the above, expression vectors that optionally comprise an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene-defective Chinese hamster cell is used and the dhfr gene is used as the selection marker, a target gene can also be selected using a thymidine-free medium.

In addition, as required, a signal sequence that matches with the host may be added to the 5'-terminal side of the DNA encoding antigen II of the present invention. Useful signal sequences include a PhoA signal sequence, an OmpA signal sequence and the like when the host is a bacterium of the genus Escherichia; an α-amylase signal sequence, a subtilisin signal sequence and the like when the host is a bacterium of the genus Bacillus; an MFα signal sequence, an SUC2 signal sequence and the like when the host is yeast; and an insulin signal sequence, an α-interferon signal sequence, an antibody molecule signal sequence and the like when the host is an animal cell.

Using the thus-constructed vector comprising a DNA that encodes the antigen II of the present invention, a transformant can be produced.

As useful examples of the host, a bacterium of the genus Escherichia, a bacterium of the genus Bacillus, yeast, an insect cell, an insect, an animal cell, and the like can be mentioned.

As specific examples of the bacterium of the genus *Escherichia, Escherichia* coli K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of molecular Biology, Vol. 41, 459 (1969)), C600 (Genetics, Vol. 39, 440 (1954)), and the like can be mentioned.

As useful examples of the bacterium of the genus Bacillus, Bacillus subtilis MI114 (Gene, Vol. 24, 255 (1983)), 207-21 (Journal of Biochemistry, Vol. 95, 87 (1984)) and the like can be mentioned.

As useful examples of the yeast, Saccharomyces cerevisiae AH22, AH22R⁻, NA87-11A, DKD-5D and 20B-12, Schizosaccharomyces pombe NCYC1913 and NCYC2036, Pichia pastoris KM71 and the like can be mentioned.

As useful examples of the insect cell, Spodoptera frugiperda cell (Sf cell), MG1 cell derived from the mid-intestine of Trichoplusia ni, High Five^{™} cell derived from an egg of Trichoplusia ni, cell derived from Mamestra brassicae, cell derived from Estigmena acrea, and the like can be mentioned when the virus is AcNPV. When the virus is BmNPV, Bombyx mori N cell (BmN cell) and the like can be used. As useful examples of the Sf cell, Sf9 cell (ATCC CRL1711), Sf21 cell (both in Vaughn, J. L. et al., In Vivo, 13, 213-217 (1977)), and the like can be mentioned.

As useful examples of the insect, a larva of Bombyx mori (Maeda et al., Nature, Vol. 315, 592 (1985)), and the like can be mentioned.

Useful animal cells include, for example, monkey COS-7 cells, Vero cells, Chinese hamster CHO cells (hereinafter, abbreviated CHO cells), Chinese hamster CHO cells lacking the dhfr gene (hereinafter, abbreviated CHO(dhfr⁻) cells), mouse L cells, mouse AtT-20 cells, mouse myeloma cells, mouse ATDC5 cells, mouse NS0 cells, mouse FM3A cells, rat GH3 cells, human FL cells, human fetal HEK293 cells, human fetal cell 293F cells, and the like.

Transformation can be performed according to the choice of host by a commonly known method.
A bacterium of the genus *Escherichia* can be transformed, for example, in accordance with a method described in Proc. Natl. Acad. Sci. USA, Vol.69, 2110 (1972), Gene, Vol.17, 107 (1982) and the like.

A bacterium of the genus *Bacillus* can be transformed, for example, according to a method described in Molecular & General Genetics, Vol.168, 111 (1979) and the like.

Yeast can be transformed, for example, in accordance with a method described in Methods in Enzymology, Vol.194, 182-187 (1991), Proc. Natl. Acad. Sci. USA, Vol.75, 1929 (1978) and the like.

An insect cell or insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, Vol.52, 456 (1973).

Thus, a transformant transformed with an expression vector comprising a DNA that encodes the antigen II of the present invention can be obtained.

Transformation can be performed according to the choice of host by a commonly known method.
When a transformant whose host is a bacterium of the genus *Escherichia* or a bacterium of the genus *Bacillus* is cultured, the culture medium used is preferably a liquid medium, in which a carbon source, a nitrogen source, an inorganic substance and others necessary for the growth of the transformant are contained. As examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, and potato extract can be mentioned; as examples of the inorganic substance, calcium chloride, sodium dihydrogen phosphate, magnesium chloride and the like can be mentioned.
In addition, yeast extract, vitamins, a growth promoting factor and the like may be added. The pH of the medium is desirably about 5 to 8.

As an example of the medium used to culture a bacterium of the genus *Escherichia,* an M9 medium comprising glucose and casamino acid [Miller, Journal of Experiments in Molecular Genetics, 431-433, Cold Spring Harbor Laboratory, New York, 1972] is preferable. As required, in order to increase promoter efficiency, a chemical agent, for example, 3β-indolylacrylic acid, may be added to the medium.

When the host is a bacterium of the genus *Escherichia,* cultivation is normally performed at about 15 to 43°C for about 3 to 24 hours, and the culture may be aerated or agitated as necessary.

When the host is a bacterium of the genus *Bacillus,* cultivation is normally performed at about 30 to 40°C for about 6 to 24 hours, and the culture may be aerated or agitated as necessary.

When a transformant whose host is yeast is cultured, as examples of the medium, Burkholder's minimal medium [Proc. Natl. Acad. Sci. USA, Vol.77, 4505(1980)] and an SD medium supplemented with 0.5% casamino acid [Proc. Natl. Acad. Sci. USA, Vol.81, 5330(1984)] can be mentioned. The pH of the medium is preferably adjusted to about 5 to 8. Cultivation is normally performed at about 20°C to 35°C for about 24 to 72 hours, and the culture may be aerated or agitated as necessary.

When a transformant whose host is an insect cell or insect is cultured, as the medium, Grace's Insect Medium (Nature, 195, 788(1962)) supplemented with inactivated 10% bovine serum and other additives as appropriate and the like are used. The pH of the medium is preferably adjusted to about 6.2 to 6.4. Cultivation is normally performed at about 27°C for about 3 to 5 days, and the culture may be aerated or agitated as necessary.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally performed at about 30 to 40°C for about 15 to 60 hours, and the culture may be aerated or agitated as necessary.

Thus, the antigen II of the present invention can be produced in the cells, on the cell membrane or out of the cells of the transformant.

Separation and purification of the GDNF from the above-described culture can be performed by, for example, the method described below.

When the antigen II of the present invention is extracted from a cultured bacterium or cells, a method is used as appropriate wherein the bacterium or cells are collected by a commonly known method after cultivation, suspended in an appropriate buffer solution, and disrupted by means of sonication, lysozyme and/or freeze-thawing and the like, after which a crude extract of the protein is obtained by centrifugation or filtration. The buffer solution may contain a protein denaturant such as urea or guanidine hydrochloride and a surfactant such as Triton X-100^{™}. When the antigen II of the present invention is secreted in the culture broth, the bacterium or cells are separated from the supernatant by a method known per se, and the supernatant is collected, after completion of the cultivation.

Purification of the antigen II of the present invention contained in the thus-obtained culture supernatant or extract can be performed by an appropriate combination of methods of separation/purification known per se. These commonly known methods of separation/purification include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like.

When the antigen II of the present invention thus obtained is a free form, the free form can be converted to a salt by a method known per se or a method based thereon; conversely, when the protein is obtained in the form of a salt, the salt can be converted to a free form or another salt by a method known per se or a method based thereon.

The antigen II of the present invention produced by the transformant can be optionally modified or partially deprived of a polypeptide by allowing an appropriate protein-modifying enzyme to act thereon before the purification or after the purification. As the protein-modifying enzyme used, for example, trypsin, chymotrypsin, arginyl endopeptidase, protein kinase, glycosidase and the like are used.

The presence of the antigen II of the present invention thus produced can be measured by an enzyme immunoassay, Western blotting and the like using a specific antibody.

(c) GDNF-expressing mammalian cells themselves can be used directly as the antigen II of the present invention. As the mammalian cells, natural cells as described in section (a) above, cells transformed by a method as described in section (b) above and the like can be used. The host used for the transformation may be any cells collected from humans, monkeys, rats, mice, hamsters and the like; HEK293 cells, COS7 cells, CHO-K1 cells, NIH3T3 cells, Balb3T3 cells, FM3A cells, L929 cells, SP2/0 cells, P3U1 cells, NSO cells, B16 cells, or P388 cells and the like are preferably used.
(d) A peptide having 1 kind or 2 kinds or more of the same antigen determinant as that of a GDNF can be produced according to a commonly known method of peptide synthesis, or by cleaving a GDNF with an appropriate peptidase. The method of peptide synthesis may be any of, for example, a solid phase synthesis process and a liquid phase synthesis process. That is, a desired peptide can be produced by condensing a partial peptide or amino acids capable of constituting the peptide and the remaining portion, and eliminating any protecting group the resultant product may have. As examples of the commonly known methods of condensation and elimination of the protecting group, the methods described below can be mentioned.
   (i) M. Bodanszky and M.A. Ondetti, Peptide Synthesis, Interscience Publishers, New York (1966)
   (ii) Schroeder and Luebke, The Peptide, Academic Press, New York (1965)
   (iii) Nobuo Izumiya, et al.: Peptide Gosei-no-Kiso to Jikken, published by Maruzen Co. (1975);
   (iv) Haruaki Yajima and Shunpei Sakakibara: Seikagaku Jikken Koza 1, Tanpakushitsu no Kagaku IV, 205 (1977)
   (v) Haruaki Yajima, ed.: Zoku Iyakuhin no Kaihatsu, Vol. 14, Peptide Synthesis, published by Hirokawa Shoten.

After the reaction, the partial peptide used in the present invention can be purified and isolated by a combination of ordinary methods of purification, for example, solvent extraction, distillation, column chromatography, liquid chromatography, recrystallization and the like. When the peptide obtained by the above-described method is a free form, the free form can be converted to an appropriate salt by a commonly known method; conversely, when the peptide is obtained in the form of a salt, the salt can be converted to a free form by a commonly known method.

### (2) Preparation of monoclonal antibody

### (a) Preparation of monoclonal antibody-producing cell by hybridoma method,

The antigen II of the present invention is administered to a warm-blooded animal. Immunization may be done by any method, as long as it can stimulate antibody production, and preferably used are intravenous injection, intraperitoneal injection, intramuscular injection, subcutaneous injection, etc. Naturally occurring mammalian animal cells or transformed mammalian animal cells, which express the protein II of the present invention, can be injected to immune animal as a suspension of the cells in a medium used for tissue culture (e.g., RPMI 1640) or buffer (e.g., Hanks' Balanced Salt Solution).

The antigen II of the present invention permits direct use for immunization in an insolubilized form. The antigen II of the present invention may be used for immunization in the form of a conjugate thereof bound or adsorbed to a suitable carrier. Regarding the mixing ratio of the carrier and the antigen II of the present invention (hapten), any carrier can be bound or adsorbed in any ratio, as long as an antibody against the antigen II of the present invention bound or adsorbed to the carrier is efficiently produced; usually, a natural or synthetic polymeric carrier in common use for preparation of an antibody against a hapten antigen, bound or adsorbed in a ratio of 0.1 to 100 parts by weight to 1 part by weight of the hapten, can be used. As examples of the natural polymeric carrier, the serum albumin of a mammal such as cattle, rabbit, or human, the thyroglobulin of a mammal such as cattle or rabbit, the hemoglobin of a mammal such as cattle, rabbit, human, or sheep, keyhole limpet hemocyanin and the like are used. As examples of the synthetic polymeric carrier, various latexes of polymers or copolymers of polyamino acids, polystyrenes, polyacryls, polyvinyls, polypropylenes and the like, and the like can be used.

Various condensing agents can be used for crosslinking the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like are conveniently used. When amino groups are crosslinked together, it is also possible to react one amino group with an activated ester reagent having a dithiopyridyl group (for example, 3-(2-pyridyldithio)propionic acid N-succinimidyl (SPDP) and the like), followed by reduction, to introduce the thiol group, and to introduce a maleimide group into the other amino group using a maleimide activated ester reagent, followed by a reaction of both.

In order to increase antibody productivity during the administration, complete Freund's adjuvant or incomplete Freund's adjuvant may be administered. The administration is usually made every 2 to 6 weeks about 2 to 10 times in total. In preparing the monoclonal antibody II of the present invention, the DNA immunization method may be utilized (see, for example, Nature, Vol.356, term 152 to term 154). As the warm-blooded animal, for example, monkeys, rabbits, dogs, guinea pigs, mice, rats, sheep, goat, chicken and the like can be mentioned, and mice and rats are preferably used.

In preparing monoclonal antibody-producing cells, a monoclonal antibody-producing hybridoma can be prepared by selecting an individual showing an antibody titer from among antigen-immunized warm-blooded animals, for example, mice, collecting the spleen or lymph nodes 2 to 5 days after final immunization, and fusing antibody-producing cells contained therein with myeloma cells of the same or different animal species. A measurement of antibody titer in antiserum may be made by, for example, reacting the labeled protein described below with the antiserum, and thereafter determining the activity of the labeling agent bound to the antibody. The fusion may be operated by a known method, for example, the method of Koehler and Milstein [Nature, 256, 495 (1975)]. As examples of fusogen, polyethylene glycol (PEG), Sendai virus and the like can be mentioned, and PEG is preferably used.

As examples of the myeloma cell, NS-1, P3U1, SP2/0, AP-1 and the like can be mentioned, and SP2/0 or P3U1 and the like are preferably used. A preferable ratio of the number of antibody-producing cells (splenocytes) and number of myeloma cells used is generally about 1:1 to 20:1; cell fusion can be efficiently performed by adding a PEG (preferably PEG1000 to PEG6000) at concentrations of about 10 to 80%, and conducting incubation generally at 20 to 40°C, preferably at 30 to 37°C, generally for 1 to 10 minutes.

Electrofusion may be used for cell fusion to prepare monoclonal antibody-producing cells.

Hybridoma can be selected by a method known per se or a method according thereto. Generally, it can be selected in a medium for animal cells supplemented with HAT (hypoxanthine, aminopterin, thymidine). Any medium for the selection and breeding can be used as far as the hybridoma can grow therein. For example, an RPMI 1640 medium comprising 1 to 20%, preferably 10 to 20%, fetal calf serum, a GIT medium (Wako Pure Chemical Industries, Ltd.) comprising 1 to 10% fetal calf serum, a serum free medium for hybridoma culture (SFM-101, Nissui Seiyaku Co., Ltd.) and the like can be used. Cultivation temperature is normally 20 to 40°C, preferably about 37°C. Cultivation time is normally 5 days to 3 weeks, preferably 1 week to 2 weeks. The cultivation can be performed normally in the presence of 5% gaseous carbon dioxide.

For screening monoclonal antibody-producing hybridomas, various methods can be used; for example, a method wherein a hybridoma culture supernatant is added to a solid phase (e.g., microplates) having a protein antigen or protein-expressing cells adsorbed directly thereto or along with a carrier, then an anti-immunoglobulin antibody (for example, anti-mouse immunoglobulin antibody is used in cases where the splenocytes used for cell fusion are from a mouse) or protein A, labeled with a radioactive substance, enzyme or the like, is added, and the monoclonal antibody bound to the solid phase is detected, a method wherein a hybridoma culture supernatant is added to a solid phase having an anti-immunoglobulin antibody or protein A adsorbed thereto, a protein labeled with a radioactive substance, enzyme or the like is added, and the monoclonal antibody bound to the solid phase is detected, and the like can be mentioned.

### (b) Preparation of monoclonal antibody by other methods

The method of preparing the antibody II of the present invention is not limited to the method described in (a); for example, what is called the antibody display technique, wherein an antibody gene library prepared from human or warm-blooded animal (e.g., monkeys, rabbits, dogs, guinea pigs, mice, rats, sheep, goat, camel, chicken and the like) B lymphocytes by a commonly known method, presented on the cell surfaces of bacteriophages, Escherichia coli, yeast, animal cells and the like on ribosome and the like, can be used [Nature Biotechnology 23, 1105 (2005)]. The human or warm-blooded animal may be naive, and may also be a cancer patient with high expression of the antigen II of the present invention or a warm-blooded animal immunized with the antigen II of the present invention by the method described in (a). The form of the antibody to be presented to the cell surface is exemplified by, but not limited to, the IgG molecule, IgM molecule, Fab fragment, single-chain Fv (scFv) fragment and the like.

The gene for a monoclonal antibody (fragment) that specifically binds to the antigen II of the present invention is obtained by reacting antibody (fragment)-presenting cells or antibody (fragment) presenting ribosomes that are carrying the above-described antibody gene library with the antigen II of the present invention for a given time, washing away the non-specifically binding portion, thereafter eluting and recovering the portion that specifically binds to the antigen II of the present invention, allowing the antibody (fragment)-presenting cells or antibody (fragment)-presenting ribosomes to grow by a commonly known method, thereafter repeating this procedure several times, and isolating the desired product from finally cloned antibody (fragment)-presenting cells or antibody (fragment)-presenting ribosomes by a commonly known method.
The monoclonal antibody fragment gene thus obtained can be recombined with the corresponding region of the IgG antibody gene by a commonly known method to obtain a monoclonal IgG antibody gene.

The antibody II of the present invention can also be obtained by immunizing antibody-producing cells isolated from a human or the above-described warm-blooded animal with the antigen II of the present invention by a method known per se in vitro, and thereafter preparing a hybridoma in the same manner as (a).

### (c) Production of monoclonal antibody

The monoclonal antibody II of the present invention can be produced by culturing a monoclonal antibody-producing hybridoma obtained in (a), or a recombinant cell line wherein an antibody gene isolated by a commonly known method from a monoclonal antibody-producing hybridoma obtained in (a) or a monoclonal antibody gene obtained in (b) is artificially expressed. The monoclonal antibody II of the present invention can also be produced by inserting the antibody gene in a warm-blooded animal or plant chromosome by a commonly known method, and allowing the antibody II to be produced in warm-blooded animal blood, milk, or eggs, plants, fungi and the like [Curr. Opin. Biotevhnol. 7, 536 (1996), Nature Rev. Genet 4, 794 (2003), Appl. Environ. Microbiol. 70, 2567 (2004)]. Useful warm-blooded animals include, for example, bovine, goat, sheep, pigs, chicken, mice, rabbits and the like. Useful plants include tobacco, corn, potato, duckweed and the like.

The monoclonal antibody II of the present invention can be purified from the above-described monoclonal antibody-containing material by a method known per se, for example, a method of immunoglobulin separation and purification [e.g., salting-out, alcohol precipitation, isoelectric point precipitation, electrophoresis, absorption-desorption using an ion exchanger (e.g., DEAE) or a hydrophobicity column, ultracentrifugation, gel filtration, affinity purification for separating and purifying only an antibody by means of a carrier wherein a substance with affinity for the antibody, such as an antigen or protein A or protein G].

### (3) Preparation of polyclonal antibody

The polyclonal antibody II of the present invention can be produced according to a method known per se or a method based thereon. For example, the polyclonal antibody can be produced by immunizing the antigen II of the present invention or a complex of the antigen and a carrier protein to a warm-blooded animal in the same manner as the above-described method of producing a monoclonal antibody, collecting a product containing an antibody to the antigen from the immunized animal, and separating and purifying the antibody.
Regarding the complex of the immune antigen and carrier protein used to immunize a warm-blooded animal, any type of carrier protein and any mixing ratio of the carrier and antigen can be used, as long as an antibody against the antigen used for immunization as crosslinked to the carrier is efficiently produced; for example, a method wherein bovine serum albumin, bovine thyroglobulin or the like is crosslinked in a ratio of about 0.1 to 20, preferably about 1 to 5, parts by weight to 1 part by weight of the hapten, is used.
Various condensing agents can be used for coupling the antigen and carrier protein; glutaraldehyde, carbodiimide, maleimide activated ester, activated ester reagents containing a thiol group or dithiopyridyl group, and the like can be used.
The condensation product is administered to a warm-blooded animal as is or along with a carrier or a diluent to a site permitting antibody production. In order to increase antibody productivity during the administration, complete Freund's adjuvant or incomplete Freund's adjuvant may be administered. The administration is usually made about every 2 to 6 weeks about 3 to 10 times in total.
The polyclonal antibody can be collected from blood, ascites fluid, breast milk, egg and the like, of a warm-blooded animal immunized by the above-described method.
The polyclonal antibody titer in antiserum can be measured in the same manner as the measurement of the antibody titer of the antiserum described above. Separation and purification of the polyclonal antibody can be performed according to the same method of immunoglobulin separation and purification as the above-described separation and purification of a monoclonal antibody.

A nucleic acid comprising a base sequence complementary to the target region of the desired nucleic acid, i.e., a nucleic acid capable of hybridizing with the desired nucleic acid, can be described as being 'antisense' against the desired nucleic acid. Meanwhile, a nucleic acid comprising a base sequence having a homology to the target region of the desired nucleic acid can be described as being 'sense' against the desired nucleic acid. Here, 'having a homology' or 'being complementary' refers to having an identity or complementarity of about 70% or more, preferably about 80% or more, more preferably about 90% or more, most preferably about 95% or more between base sequences.

A nucleic acid comprising a base sequence complementary to the base sequence that encodes GDNF or a portion thereof (hereinafter, also referred to as 'antisense GDNF' or 'the antisense nucleic acid II of the present invention') can be designed and synthesized on the basis of the base sequence information on the cloned or determined nucleic acid that encodes GDNF. Such a nucleic acid is capable of inhibiting the replication or expression of the gene that encodes GDNF. Specifically, antisense GDNF is capable of hybridizing with the RNA transcribed from the gene that encodes GDNF, and inhibiting the synthesis (processing) or function (translation into protein) of mRNA.

Target region of an antisense GDNF is not particularly limited in its length as long as the translation into a GDNF protein is inhibited as a result of hybridization of an antisense nucleic acid, and it may be a whole sequence or a partial sequence of mRNA encoding the protein which can be exemplified by a short strand of about 15 bases and a long strand of mRNA or whole sequence of early transcription product. In consideration of a simple synthesis and an antigenic problem, oligonucleotide comprising about 15 to 30 bases is preferable, but it is not limited thereto. In specific, for example, the 5' end hairpin loop of nucleic acid encoding GDNF, 5' end 6-base-pair repeats, 5' end untranslated region, translation initiation codon, protein coding region, translation termination codon, 3' end untranslated region, 3' end palindrome region, 3' end hairpin loop, and the like, may be selected as target regions, though any other region may be selected as a target in the genes encoding GDNF. For example, an intron-part of the gene can be exemplified as a preferable target region.
Further, the antisense GDNF may be a polynucleotide in which the translation into a protein is inhibited by hybridizing with mRNA encoding GDNF or with early transcription product, and it may as well as be the polynucleotide capable of forming a triplex by binding with the double-stranded DNA which is the gene encoding GDNF and inhibiting the transcription of RNA.

Examples of the antisense nucleic acid include deoxyribonucleotides containing 2-deoxy-D-ribose, ribonucleotides containing D-ribose, any other type of nucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense nucleic acid may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms 'nucleoside', 'nucleotide' and 'nucleic acid' are used to refer to moieties that contain not only the purine and pyrimidine bases, but also other heterocyclic bases, which have been modified. Such modifications may include methylated purines and pyrimidines, acylated purines and pyrimidines or other heterocyclic rings. Modified nucleotides and modified nucleotides also include modifications on its sugar moiety, wherein, for example, one or more hydroxyl groups may optionally be substituted with a halogen atom(s), an aliphatic group(s), etc., or may be converted into the functional groups such as ethers, amines, or the like.

Preferably, the antisense nucleic acid is an optionally modified RNA or DNA. Specific examples of the modified nucleic acid (RNA, DNA) include, but are not limited to, those resistant to degradation such as sulfur derivatives, thiophosphate derivatives of nucleic acids, polynucleosideamide and oligonucleosideamide. Antisense GDNF can preferably be designed with the following aims. Specifically, antisense nucleic acid in cells is further stabilized, the cell permeability of antisense nucleic acid is increased, affinity for target sense strand is increased, and, the toxicity, if any, of antisense nucleic acid is reduced. Many such modifications are known in the art, and are disclosed in, for example, J. Kawakami et al., Pharm Tech Japan, Vol.8, pp.247, 1992; Vol.8, pp.395, 1992; S.T. Crooke et al. ed., Antisense Research and Applications, CRC Press, 1993 and elsewhere.

The antisense nucleic acid may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

In addition, the antisense GDNF includes a ribozyme capable of specifically cleaving the internal (intron is included in early transcription products) coding region of RNA encoding GDNF (mRNA or an early transcription product etc.). 'ribozyme' is RNA having enzymatic activity for cleaving nucleic acid, but since recently it is discovered that the oligoDNA having a base sequence of the enzymatic activity site also has the activity for cleaving nucleic acid, the present specification also includes DNA as long as it has a sequence specific enzymatic activity for cleaving nucleic acid.
Riobzyme with mostly high-generality includes self-splicing RNA which can be found in infectious RNA such as viroid, a virusoid, etc., and hammer-head type or hairpin type are known. The hammer-head type exhibits enzymatic activity at about 40 bases, and it is possible to specifically cleave only the target mRNA by complementary arranging several bases (about 10 bases in total) of both ends adjacent to the part having a hammer-head structure. This type of ribozyme has a further advantage that genomic DNA is never targeted as its substrate is only RNA. When GDNF forms itself a double-stranded structure, the target sequence can be formed into a single-strand by using hybrid ribozyme coupled with RNA motif derived from viral nucleic acid which specifically binds to RNA helicase [Proc. Natl. Acad. Sci. USA, 98 (10): 5572-5577 (2001)]. Also, in a case where ribozyme is used in the form of an expression vector having DNA which encodes the ribozyme, the ribozyme can be hybrid ribozyme further coupled with the sequence of modified tRNA so as to promote transport to cytoplasm of a transcriptional product [Nucleic Acids Res., 29 (13): 2780-2788 (2001)].

Double-stranded oligoRNA (siRNA) (siRNA to RNA encoding GDNF) comprising a base sequence complementary to a partial sequence of coding region (intron is included in early transcription products) in RNA encoding GDNF (mRNA or early transcription products etc.) is also included in antisense GDNF. The phenomenon of so-called RNA interference (RNAi) in which mRNA complementary to one of the chains of the RNA introduced is degraded by introducing short-stranded mRNA into a cell, is known to occur in nematode, an insect, plant, etc., but after it is confirmed that the phenomenon also occurs in mammalian cells [Nature, 411 (6836): 494-498 (2001)], it is widely used as an alternative technology of ribozyme.

The antisense nucleic acid II of the present invention can be prepared by determining a target region of mRNA or early transcription product on the basis of the information of a cDNA sequence encoding GDNF or genomic DNA sequence of the gene of the present invention, and synthesizing its complementary sequence with the use of a commercially available DNA/RNA automatic synthesizer (Applied Biosystems, Beckman, etc.). siRNA having an RNAi activity can be prepared according a process comprising synthesizing a sense strand and an antisense strand respectively with the DNA/RNA automatic synthesizer, denaturing in a suitable annealing-buffer solution at, for example, about 90°C to 95°C for about 1 minute, and annealing at about 30°C to 70°C for about 1 to 8 hours. In addition, longer double-stranded polynucleotide can be prepared according to a process comprising synthesizing complementary oligonucleotides in an alternately overlapping manner, annealing the oligonucleotides, and ligating with ligase.

The gene expression inhibitory activity of antisense GDNF can be examined using a transformant containing a nucleic acid that encodes GDNF, an in vivo or in vitro GDNF-encoding-gene expression system or an in vivo or in vitro GDNF translation system.

The above-described substances that inhibit a function (e.g., GDNF activity and expression) of GDNF, such as the antibody II of the present invention and the antisense nucleic acid II of the present invention, have, for example, the following uses.

As shown in an Example below, by allowing GDNF to act on cancer cells (e.g., breast cancer cells), cell growth is promoted. This fact shows that the growth of a cancer cells (e.g., breast cancer cells) is promoted by GDNF, and that a substance capable of inhibiting an activity or expression of GDNF inhibits the growth of a cancer cells (e.g., breast cancer cells), and is effective in the prophylaxis/treatment of cancers (e.g., breast cancer).

Because the antibody II of the present invention is capable of inhibiting GDNF activity by binding specifically to GDNF, and also because the antisense nucleic acid II of the present invention is capable of inhibiting GDNF expression, it is possible to inhibit an activity or expression of GDNF in tissue and weakening the action of GDNF on cancer cells by administering the antibody II of the present invention to a cancer (e.g., breast cancer) patient, or administering the antisense nucleic acid II of the present invention to a patient to introduce (and express) the same into target cells, to thereby inhibit the growth of a cancer cells, and prevent/treat cancers.

Therefore, a pharmaceutical comprising the above-described substance that inhibits a function of GDNF, such as a) the antibody II of the present invention or b) the antisense nucleic acid II of the present invention, can be used as, for example, a prophylactic/therapeutic agent for cancers (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like) (preferably, a prophylactic/therapeutic agent for breast cancer), cancer cell apoptosis promoter, cancer cell (preferably, breast cancer cells) growth inhibitor, cancer cell cycle alteration inducer, cancer metastasis suppressant, cancer cell adhesion inhibitor and the like.

When the antibody II of the present invention is used as the above-described prophylactic/therapeutic agent and the like, the antibody can be prepared as a pharmaceutical preparation in accordance with a conventional method.
When the antisense nucleic acid II of the present invention is used as the above-described prophylactic/therapeutic agent and the like, the nucleic acid, as is or after being inserted into an appropriate expression vector such as retrovirus vector, adenovirus vector, or adenovirus associated virus vector in a functional way, can be prepared as a pharmaceutical preparation in accordance with a conventional method. The nucleic acid can be administered as is, or along with an auxiliary for promoting its ingestion, using a gene gun or a catheter such as a hydrogel catheter.

The preventive/remedy agents for the above diseases comprising a substance inhibiting the function of GDNF such as the antibody II of the present invention, antisense nucleic acid II of the present invention and the like are low-toxic and can be administered as they are in the form of liquid preparations, or as pharmaceutical compositions of suitable preparations to human or non-human mammals (e.g., rats, rabbits, sheep, swine, bovine, feline, canine, simian, etc.), orally or parenterally (e.g., intravascularly, subcutaneously, etc.).

The substance inhibiting the function of GDNF such as the antibody II of the present invention, antisense nucleic acid II of the present invention and the like may be administered in itself, or may be administered as an appropriate pharmaceutical composition. The pharmaceutical composition used for the administration may contain a pharmacologically acceptable carrier, diluent or excipient with the antibody II of the present invention, antisense nucleic acid II of the present invention. Such a pharmaceutical composition is provided in the form of pharmaceutical preparations suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody II of the present invention or antisense nucleic acid II of the present invention described above or the salts thereof in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the antibody described above or antisense nucleic acid with conventional bases for suppositories.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a carrier, a diluent or excipient conventionally used in the field of pharmaceutical preparations. Examples of the carrier or excipient for tablets are lactose, starch, sucrose, magnesium stearate, etc.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules), suppositories, etc. The amount of the antibody contained is generally 5 to 500 mg per dosage unit form; it is preferred that the antibody described above is contained in 5 to 100 mg especially in the form of injection, and in 10 to 250 mg for the other forms. Regarding the content of antisense nucleic acid, it is preferable that the above-described antisense nucleic acid be contained at normally 5 to 500 mg, particularly 5 to 100 mg for an injection, or 10 to 250 mg for other dosage forms, per unit dosage form.

The dosage of the above-mentioned prophylactic/therapeutic agents and the like comprising the antibody II of the present invention varies also depending on the subject of administration, target disease, symptoms, route of administration and the like; for example, when the agent is used for the treatment/prevention of breast cancer in an adult, the antibody II of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

The dosage of the above-described prophylactic/therapeutic agents and the like comprising the antisense nucleic acid II of the present invention varies also depending on the subject of administration, target disease, symptoms, route of administration and the like; for example, when the agent is used for the treatment/prevention of breast cancer in an adult, the antisense nucleic acid II of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

Each composition described above may further contain other active components unless they cause any adverse interaction with the antibody described above or antisense nucleic acid due to blending.

Furthermore, a substance inhibiting the function of GDNF such as the antibody II of the present invention, antisense nucleic acid II of the present invention and the like may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, irinotecan, etc. The antibody II of the present invention or antisense nucleic acid II of the present invention and the above-mentioned drug may be administered simultaneously or at staggered times to the patient.

Because the antibody II of the present invention specifically recognizes GDNF, and can be used for quantitation of GDNF in a test liquid, particularly for quantitation by sandwich immunoassay and the like, the same is useful as, for example, a diagnostic reagent for decreased expression or increased expression of the protein. As shown in an Example below, cancer cells (e.g., breast cancer cells) undergo the action of GDNF, whereby the cell growth thereof is promoted. Therefore, by detecting and quantifying GDNF in a test sample such as cells, tissue, or body fluid using the antibody II of the present invention, whether or not the subject is in a state wherein cancers (e.g., breast cancer), particularly cancers that are highly sensitive to GDNF (e.g., breast cancer) are likely to grow can be detected. Hence, the antibody II of the present invention is useful as a diagnostic reagent for cancers (e.g., breast cancer). For example, by quantifying GDNF in the sample using the antibody II of the present invention, when an increase in the expression of GDNF is detected, the subject can be diagnosed to be in a state wherein cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc., particularly breast cancer) is likely to grow.

Quantification of GDNF using antibody II of the present invention includes, for example:
(i) a method comprising competitively reacting the antibody II of the present invention, a sample fluid, and a labeled form of GDNF, and determining the labeled protein of the present invention that binds to the antibody, thereby to quantify GDNF in the test sample fluid; and
(ii) a method comprising simultaneously or continuously reacting a test sample fluid, the immobilized antibody II of the present invention on a carrier, and a labeled form of another antibody II of the present invention, and measuring the activity of the label on the immobilizing carrier, thereby to quantify GDNF in the test sample fluid.

In the quantification method of (ii) above, two species of antibodies are desirably the ones that each recognizes the different part in GDNF. For example, when one antibody recognizes the N-terminal region of GDNF, another antibody reacting with the C-terminal region of GDNF is used.

Examples of labeling agents, which are employed for the aforesaid measuring methods using labeling agents, are radioisotopes, enzymes, fluorescent substances, luminescent substances, etc. Examples of radioisotopes are [¹²⁵I], [¹³¹I], [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include cyanine fluorescent dyes (e.g., Cy2, Cy3, Cy5, Cy5.5, Cy7 (manufactured by Amersham Biosciences K.K.) and the like), fluorescamine, fluorescein isothiocyanate, etc. Examples of the luminescent substances are luminol, a luminol derivative, luciferin, lucigenin, etc. Furthermore, a biotin-(strepto)avidin system may be used as well for binding of an antibody or antigen to a labeling agent.

As the test sample fluid, when GDNF is localized in a cell, a cell homogenate obtained by suspending cells in an appropriate buffer, and then breaking cells by ultrasonication, freeze-thaw cycling, etc., is used, and when GDNF is secreted outside the cell, cell culture supernatant or body fluid (blood, serum, plasma, urine, sweat, breast milk and the like) is used. If necessary, the quantification can be carried out after separating and purifying GDNF from a homogenate, a cell-culture supernatant or body fluid. In addition, an intact cell can be used as a specimen as long as the label detection is possible.

The quantification method of GDNF using the antibody II of the present invention is not particularly limited. Any quantification method may be used, so long as the amount of an antibody, antigen or antibody-antigen complex corresponding to the amount of antigen (e.g., protein amount) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use, for example, the sandwich method described later.

In the immobilization of antigens or antibodies, physical adsorption may be used. Alternatively, chemical binding that is conventionally used for immobilization/stabilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, the immobilized antibody II of the present invention is reacted with a test sample (primary reaction), then with a labeled form of another antibody II of the present invention (secondary reaction), and the amount or activity of the label on the immobilizing carrier is measured, whereby the amount of the protein II of the present invention in the test sample can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one species, but a mixture of two or more species of antibody may be used, for example, to increase the measurement sensitivity.

In the method of measuring GDNF by the sandwich method, the antibody II of the present invention used in the primary reaction and that used in the secondary reaction are preferably antibodies having different sites for GDNF binding. Hence, regarding the antibodies used in the primary reaction and the secondary reaction, for example, provided that the antibody used in the secondary reaction recognizes the C-terminus of GDNF, the antibody used in the primary reaction is preferably an antibody that recognizes a site other than the C-terminus, for example, the N-terminus.

The antibody II of the present invention can also be used in measuring system other than the sandwich method such as in the competitive method, the immunometric method, nephrometry, etc.

In the competitive assay, an antigen in a test sample and a labeled form of antigen are reacted competitively against an antibody, an unreacted labeled antigen (F) is separated from an antibody-bound labeled antigen (B) (B/F separation), and the labeled amount of B or F is determined, thereby to quantify the antigen in the test sample. The present reaction method includes a liquid phase method in which the B/F separation is carried out by using a soluble antibody as an antibody and using polyethylene glycol or a secondary antibody against the antibody etc.; and a solid phase method in which a solid-phased antibody is used as a primary antibody or a soluble antibody is used as a primary antibody and a solid-phased antibody is used as a secondary antibody.

In the immunometric assay, an antigen in a test sample and a solid phased antigen are competitively reacted with a given amount of a labeled form of the antibody followed by separating the solid phase from the liquid phase; or an antigen in a test sample and an excess amount of labeled form of the antibody are reacted, then a solid phased antigen is added to allow an unreacted labeled form of the antibody to bind to the solid phase, and the solid phase is then separated from the liquid phase. Thereafter, the labeled amount in any of the phases is measured to determine the antigen level in the test sample.

In the nephrometry, the amount of insoluble sediment, which is produced as a result of the antigen-antibody reaction in a gel or in a solution, is measured. Even when the amount of antigen in a test sample is small and only a small amount of the sediment is obtained, a laser nephrometry utilizing laser scattering and the like can be suitably used.

Using the antibody II of the present invention, GDNF can be quantified, and can also be detected by tissue staining and the like. For these purposes, the antibody molecule itself may be used, and the F(ab')₂, Fab', or Fab fraction of the antibody molecule may also be used.

For applying each of these immunological methods to the quantification method of the present invention, any setting of particular conditions or procedures is not required. The system for assaying GDNF may be established by applying the usual technical concern of those skilled in the art, in addition to the usual conditions and operating method for the respective methods. For the details of these general technical means, reference can be made to the following reviews and texts.

For example, see, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, GDNF can be quantified with high sensitivity using the antibody II of the present invention.

The antibody II of the present invention can be used for preparing an antibody column for purification of GDNF, detecting GDNF in each fraction during purification, analyzing the behavior of GDNF in test cells and for other purposes.

Because a nucleic acid comprising the base sequence that encodes GDNF or a portion thereof (hereinafter, also referred to as 'sense GDNF'), or a nucleic acid comprising a base sequence complementary to the base sequence or a portion thereof (antisense GDNF) is capable of detecting an abnormality in the GDNF-encoding DNA or mRNA (gene abnormality) in a human or other warm-blooded animal (for example, rats, mice, hamsters, rabbits, sheep, goat, pigs, bovine, horses, cats, dogs, monkeys, chimpanzees, birds and the like) when used as a probe and the like, the same is useful as, for example, a gene diagnostic reagent for damage or mutation in the DNA, splicing abnormality or decreased expression in mRNA, or amplification in the DNA, increased expression in mRNA and the like. The nucleic acid comprising a part of the base sequence that encodes GDNF is not particularly limited as long as it has a length required as a probe or primer (for example, about 15 bases or more), and is not required to encode a partial peptide of GDNF.
The above-described gene diagnosis using the sense or antisense GDNF can be performed by, for example, the publicly known Northern hybridization, quantitative RT-PCR, PCR-SSCP assay, allele-specific PCR, PCR-SSOP assay, DGGE assay, RNase protection assay, PCR-RFLP assay, etc.
As shown in the Example below, cancer cells (e.g., breast cancer cells) undergo the action of GDNF, whereby the cell growth thereof is promoted. Therefore, by detecting and quantifying GDNF in a test sample such as cells, tissue, or body fluid using sense or antisense GDNF, whether or not the subject is in a state wherein cancers (e.g., breast cancer), particularly cancers that are highly sensitive to GDNF (e.g., breast cancer) are likely to grow, can be detected. Hence, sense or antisense GDNF is useful as a diagnostic reagent for cancers (e.g., breast cancer). For example, by quantifying the expression of GDNF in the sample using sense or antisense GDNF, when an increase in the expression of GDNF is detected, the subject can be diagnosed to be in a state wherein cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc., particularly breast cancer) is likely to grow.

### (III. Anti-GFRα1 antibody and the like)

A protein comprising the same or substantially the same amino acid sequence as the amino acid sequence shown by SEQ ID NO:11 (hereinafter, sometimes abbreviated 'GFRα1 protein isoform a') or a protein comprising the same or substantially the same amino acid sequence as the amino acid sequence shown by SEQ ID NO:13 (hereinafter, sometimes abbreviated 'GFRα1 protein isoform b') (hereinafter, both are sometimes together referred to as 'GFRa1' or 'the protein III of the present invention') may be a protein derived from human or warm-blooded animal (e.g., guinea pigs, rats, mice, chicken, rabbits, pigs, sheep, bovine, monkeys and the like) cells [e.g., hepatocytes, splenocytes, nerve cells, glial cells, P cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells (e.g., breast cancer cells) and the like]; or any tissues where such cells are present, for example, brain and various parts of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, and the like, and may be a synthetic protein.

As substantially the same amino acid sequence as the amino acid sequence shown by SEQ ID NO:11 or SEQ ID NO:13, an amino acid sequence having a homology of about 50% or more, preferably about 60% or more, more preferably about 70% or more, more preferably about 80% or more, particularly preferably about 90% or more, most preferably about 95% or more, to the amino acid sequence shown by SEQ ID NO:11 or SEQ ID NO:13 and the like can be mentioned.

Preferable proteins comprising substantially the same amino acid sequence as the amino acid sequence shown by SEQ ID NO:11 or SEQ ID NO:13 include, for example, the above-described protein comprising substantially the same amino acid sequence as the amino acid sequence shown by SEQ ID NO:11 or SEQ ID NO:13, and having substantially the same quality of activity as a protein comprising the amino acid sequence shown by SEQ ID NO:11 or SEQ ID NO:13, SEQ ID NO:11 or SEQ ID NO:13 and the like.

Here, 'a homology' means a ratio (%) of identical amino acid residues and similar amino acid residues to all overlapping amino acid residues in the best alignment (preferably, the algorithm considers introduction of gaps on one or both sides of the sequence for the best alignment) where two amino acid sequences are aligned using a mathematical algorithm known in the technical field. 'A similar amino acid' means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected to give no change in the phenotype of proteins (i.e., constitutive amino acid substitution). Specific examples of constitutive amino acid substitution are obvious in the relevant technical field, and are described in various documents (for example, refer Bowie et al, Science, 247: 1306-1310 (1990)). Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

As examples of substantially the same quality of activity, an activity to transmit GDNF stimulation into cells to thereby promote the growth of a cancer cells (e.g., breast cancer cells) and the like can be mentioned. 'Substantially the same quality of' means that the activities are qualitatively (e.g., physiologically or pharmacologically) equivalent to each other. Therefore, it is preferable that the degree of activity of the protein III of the present invention be equivalent to that of a protein comprising the amino acid sequence shown by SEQ ID NO:11 or SEQ ID NO:13 (e.g., about 0.01 to 100 times, preferably about 0.1 to 10 times, more preferably about 0.5 to 2 times), but the quantitative factors, such as the extent of activity and the molecular weight of the protein, may be different.
A measurement of the activity of GFRα1 can be performed in accordance with a method known per se. For example, as described in an Example below, by measuring cell growth of a cancer cells (e.g., breast cancer cells) that are co-expressing RET and GFRα1 when stimulated with GDNF, the activity can be evaluated.

Examples of GFRα1 include what are called muteins of proteins comprising (i) an amino acid sequence having 1 or 2 or more (e.g., about 1 to 50, preferably about 1 to 30, more preferably about 1 to 10, still more preferably several (1 to 5)) amino acids deleted from the amino acid sequence shown by SEQ ID NO:11 or SEQ ID NO:13, (ii) an amino acid sequence having 1 or 2 or more (e.g., about 1 to 50, preferably about 1 to 30, more preferably about 1 to 10, still more preferably several (1 to 5)) amino acids added to the amino acid sequence shown by SEQ ID NO:11 or SEQ ID NO:13, (iii) an amino acid sequence having 1 or 2 or more (e.g., about 1 to 50, preferably about 1 to 30, more preferably about 1 to 10, still more preferably several (1 to 5)) amino acids inserted in the amino acid sequence shown by SEQ ID NO:11 or SEQ ID NO:13, (iv) an amino acid sequence having 1 or 2 or more (e.g., about 1 to 50, preferably about 1 to 30, more preferably about 1 to 10, still more preferably several (1 to 5)) amino acids substituted by other amino acids in the amino acid sequence shown by SEQ ID NO:11 or SEQ ID NO:13, or (v) an amino acid sequence comprising a combination thereof., and the like. The protein preferably has substantially the same quality of activity as a protein having the amino acid sequence shown by SEQ ID NO:11 or SEQ ID NO:13.
When an amino acid sequence is inserted, deleted or substituted as described above, the position of the insertion, deletion or substitution is not particularly limited.

For the proteins in the present specification, the left end indicates the N-terminus (amino terminus) and the right end indicates the C-terminus (carboxyl terminus), according to the common practice of peptide designation. Any protein used in the present invention, including a protein comprising the amino acid sequence shown by SEQ ID NO:11 may have any of a carboxyl group (-COOH), a carboxylate (-COO⁻), an amide (-CONH₂), and an ester (-COOR) at the C-terminus thereof.

Here, as R in the ester, a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl, a phenyl-C₁₋₂ alkyl group such as benzyl and phenethyl, a C₇₋₁₄ aralkyl group such as an α-naphthyl-C₁₋₂ alkyl group such as α-naphthylmethyl, a pivaloyloxymethyl group; and the like can be used.

When GFRα1 has a carboxyl group (or a carboxylate) at a position other than the C-terminus, a protein wherein the carboxyl group is amidated or esterified is also included in the GFRα1 used in the present invention. In this case, useful esters include the above-described ester at the C-terminus and the like.

Furthermore, GFRα1 also includes those having the amino group of the amino acid residue (e.g., methionine residue) at the N-terminus protected by a protecting group (e.g., C₁₋₆ acyl groups such as C₁₋₆ alkanoyls such as formyl group and acetyl group, and the like); those having the glutamine residue resulting from cleavage at the N-terminus in vivo pyroglutamated; those having a substituent (e.g., -OH, -SH, amino group, imidazole group, indole group, guanidino group and the like) on the side chain of an amino acid in the molecule protected by an appropriate protecting group (e.g., C₁₋₆ acyl groups such as C₁₋₆ alkanoyl groups such as formyl group and acetyl group, and the like), or complex peptides having a sugar chain bound thereto, such as what is called a glycopeptide, and the like.

As specific examples of GFRα1, a protein comprising the amino acid sequence shown by SEQ ID NO:11 (human GFRα1 protein isoform a), a protein comprising the amino acid sequence shown by SEQ ID NO:13 (human GFRα1 protein isoform b) and the like can be mentioned.

The partial peptide of GFRα1 may be any partial peptide of GFRα1 described above, preferably having substantially the same quality of activity as GFRα1 described above. Here, 'substantially the same quality of activity' is as defined above. A determination of "substantially the same quality of activity' can be performed as described above. The partial peptide of GFRα1 preferably has immunogenicity.

For example, a peptide having at least 20 or more, preferably 50 or more, more preferably 70 or more, still more preferably 100 or more, most preferably 200 or more, amino acids of the constituent amino acid sequence of GFRα1 and the like are used.

In addition, the partial peptide of the GFRα1 used in the present invention may have (1) 1 or 2 or more (preferably about 1 to 20, more preferably about 1 to 10, still more preferably several (1 to 5)) amino acids deleted from the amino acid sequence thereof, or (2) 1 or 2 or more (preferably about 1 to 20, more preferably about 1 to 10, still more preferably several (1 to 5)) amino acids added to the amino acid sequence thereof, or (3) 1 or 2 or more (preferably about 1 to 20, more preferably about 1 to 10, still more preferably several (1 to 5)) amino acids inserted in the amino acid sequence thereof, or (4) 1 or 2 or more (preferably about 1 to 20, more preferably about 1 to 10, still more preferably several, still yet more preferably about 1 to 5) amino acids substituted by other amino acids in the amino acid sequence thereof, or (5) a combination thereof.

For the partial peptide of the GFRα1, the C-terminus may be any of a carboxyl group (-COOH), a carboxylate (-COO⁻) , an amide (-CONH₂) or an ester (-COOR).
Furthermore, the partial peptide of GFRα1, like the above-described GFRα1, includes one having a carboxyl group (or a carboxylate) at a position other than the C-terminus, those having the amino group of the amino acid residue (e.g., methionine residue) at the N-terminus protected by a protecting group; those having the glutamine residue resulting from cleavage on the N-terminus side *in vivo* pyroglutamated; those having a substituent on the side chain of an amino acid in the molecule protected by an appropriate protecting group, or complex peptides having a sugar chain bound thereto, such as what is called a glycopeptide, and the like.

The length of such an immunogenic peptide is not particularly limited, as long as the peptide has immunogenicity; for example, one having 8, preferably 10, more preferably 12, continuous amino acid residues can be mentioned.

As a salt of GFRα1 or a partial peptide thereof, salts with physiologically acceptable acids (e.g., inorganic acids, organic acids), bases (e.g., alkali metal salts) and the like are used, and physiologically acceptable acid addition salts are particularly preferable. Such salts include, for example, salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Useful substances that inhibits a function of GFRα1 or a partial peptide thereof or a salt thereof include, for example,
(1) an antibody against GFRα1 or a partial peptide thereof or a salt thereof,
(2) a low-molecular compound that inhibits a function of GFRα1 or a partial peptide thereof or a salt thereof or a salt thereof,
(3) an antisense nucleic acid against a nucleic acid that encodes GFRα1 or a partial peptide thereof, or
(4) an siRNA against an RNA that encodes GFRα1 or a partial peptide thereof, and the like.

Although the antibody against GFRα1 or a partial peptide thereof or a salt thereof (hereinafter, sometimes abbreviated 'the antibody III of the present invention') may be a polyclonal antibody or a monoclonal antibody, as long as it is an antibody capable of recognizing GFRα1 or a partial peptide thereof or a salt thereof, the antibody is preferably a monoclonal antibody. Although the isotype of the antibody is not particularly limited, it is preferably IgG, IgM or IgA.
The antibody III of the present invention may be any of a mouse antibody, rat antibody, rabbit antibody, human antibody, humanized antibody, chimeric antibody thereof and the like. Alternatively, antibodies obtained by an antibody display method, such as the phage display method, using a non-human warm-blooded animal (e.g., rabbits, goat, bovine, chicken, mice, rats, sheep, pigs, horses, cats, dogs, monkeys, chimpanzees and the like) or human antibody gene library and the like can also be included in the antibody III of the present invention. The antibody III of the present invention is preferably a human monoclonal antibody.

The antibody III of the present invention is not particularly limited with respect to molecular morphology, as long as it has at least a complementarity determining region (CDR) for specifically recognizing and binding to GFRα1 or a partial peptide thereof or a salt thereof; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like.

As the antibody III of the present invention, an antibody that recognizes an extracellular region of GFRα1 is preferable. GFRα1 is normally expressed on the cell surface via the GPI anchor. Therefore, normally, the full length of the peptide moiety of GFRα1 corresponds to the extracellular region.

An antibody against GFRα1 or a partial peptide thereof or a salt thereof (hereinafter, in the explanation of antibodies, these are sometimes comprehensively abbreviated 'GFRα1s') can be produced in accordance with a method of antibody or antiserum production known per se.

Described below are a method of preparing an antigen for the antibody III of the present invention, and a method of producing the antibody.

### (1) Preparation of antigen

As the antigen used to prepare the antibody III of the present invention, any of the above-described GFRα1s (e.g., a protein comprising the amino acid sequence shown by SEQ ID NO:11 or SEQ ID NO:13 (GFRα1) or a partial peptide thereof or a salt thereof), a fusion protein between an extracellular region protein of GFRα1 and another protein (peptide) or a salt thereof, or a (synthetic) peptide having 1 kind or 2 or more kinds of the same antigen determinant as GFRα1 and the like can be used (hereinafter, these are also simply referred to as the antigen III of the present invention).

As specific examples of the antigen III of the present invention, a cell line that naturally or artificially highly expresses GFRα1s or a membrane fraction thereof, an extracellular region protein of GFRα1 or a salt thereof, a fusion protein between an extracellular region of GFRα1 and another protein (peptide), or a (synthetic) peptide having 1 kind or 2 or more kinds of the same antigen determinant as GFRα1 and the like can be mentioned.

As examples of other proteins or peptides, FLAG-tag, His-tag, Myc-tag, V5-tag, GST-tag, S-tag, T7-tag, the Fc region of an antibody (human antibody, mouse antibody and the like) and the like can be mentioned.

The length of such a (synthetic) peptide is not particularly limited, as long as the peptide has immunogenicity; for example, one having 8, preferably 10, more preferably 12, continuous amino acid residues can be mentioned.

GFRα1 or a partial peptide thereof or a salt thereof can also be produced from the above-described human or warm-blooded animal cells or tissue by a method of protein purification known per se or a method based thereon, and can also be produced by culturing a transformant comprising a nucleic acid that encodes the protein (DNA, RNA and the like). GFRα1 or a partial peptide thereof or a salt thereof can also be produced in accordance with the method of peptide synthesis described below. A fusion protein between an extracellular region of GFRα1 and another protein (peptide) can be produced by culturing a transformant comprising a nucleic acid (DNA, RNA and the like) that encodes the fusion protein.

(a) When the antigen III of the present invention is prepared from a human or warm-blooded animal (e.g., guinea pigs, rats, mice, chicken, rabbits, pigs, sheep, bovine, monkeys and the like) tissue or cells, the tissue or cells may be homogenized to yield a crude fraction (e.g., membrane fraction, soluble fraction) that can be used as the antigen as is. Alternatively, the antigen can be purified and isolated by extraction with an acid, surfactant or alcohol and the like, and treating the extract by a combination of salting-out, dialysis, chromatographies such as gel filtration, reversed-phase chromatography, ion exchange chromatography, and affinity chromatography. The antigen obtained may be used as the immunogen as is, and may be subjected to limited degradation using peptidase and the like to yield a partial peptide that can be used as the immunogen.

(b) When a GFRα1s or a fusion protein between an extracellular region of GFRα1 and another protein (peptide) or a salt thereof is produced using a transformant comprising a nucleic acid that encodes the antigen III of the present invention, the nucleic acid can be prepared by a commonly known method of cloning [for example, a method described in Molecular Cloning (2nd ed.; J. Sambrook et al., Cold Spring Harbor Lab. Press, 1989) and the like].

The nucleic acid that encodes GFRα1 or a partial peptide thereof may be any one comprising the above-described base sequence that encodes the amino acid sequence of GFRα1 or a partial amino acid sequence thereof, used in the present invention. The nucleic acid may be DNA or RNA, or a DNA/RNA chimera, and is preferably DNA. In addition, the nucleic acid may be a double-strand, or single-strand. The double-strand may be a double-stranded DNA, a double-stranded RNA, or a DNA:RNA hybrid.

The DNA that encodes GFRα1 or a partial peptide thereof can be exemplified by genomic DNA, cDNA derived from human or other warm-blooded animal (e.g., simian, bovine, horses, swine, sheep, goat, rabbits, mice, rats, guinea pigs, hamsters, chicken and the like) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells (e.g., breast cancer cells) and the like]; or any tissues or organs where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., brown adipose tissue, white adipose tissue), skeletal muscle and the like], synthetic DNA, and the like. As the RNA that encodes GFRα1 or a partial peptide thereof, mRNA (mature mRNA) or early transcription product and the like can be mentioned.

As the method of cloning a DNA that fully encodes GFRα1 or a partial peptide thereof, a method wherein the DNA is amplified by a PCR method using a synthetic DNA primer having a portion of the base sequence that encodes GFRα1 or a partial peptide thereof, a method wherein the desired DNA is selected from a cDNA library by a hybridization method using a DNA fragment or synthetic DNA that encodes a portion or entire region of GFRα1 as the probe, and the like can be mentioned. The template polynucleotide used for the PCR may be any one comprising the base sequence that encodes GFRα1 or a partial peptide thereof; for example, genomic DNA, genomic DNA library, cDNA derived from the above-described cell/tissue, a cDNA library derived from the above-described cell/tissue, synthetic DNA and the like can be used. The hybridization can be carried out, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out more preferably under high stringent conditions.

High-stringent conditions refer to, for example, conditions involving a sodium concentration of about 19 to 40mM, preferably about 19 to 20mM, and a temperature of about 50 to 70°C, preferably about 60 to 65°C. In particular, a case wherein the sodium concentration is about 19mM and the temperature is about 65°C is most preferred. Those skilled in the art can simply regulate the condition to a desired stringency by appropriately changing a concentration of hybridization solution, temperature of hybridization reaction, probe concentration, length of probe, number of mismatch, time for hybridization reaction, salt concentration of washing solution, temperature for washing, etc.

More specifically, useful nucleic acids (DNA and the like) that encode GFRα1 include (i) a nucleic acid comprising the base sequence shown by SEQ ID NO:12 (the nucleic acid encodes a protein comprising the amino acid sequence shown by SEQ ID NO:11 (human GFRα1 protein isoform a)), or a nucleic acid comprising a base sequence that hybridizes with the base sequence shown by SEQ ID NO:12 under high stringent conditions, and encoding a protein or peptide having substantially the same quality of activity as the above-described protein comprising the amino acid sequence shown by SEQ ID NO:11 and the like, (ii) a nucleic acid comprising the base sequence shown by SEQ ID NO:14 (the nucleic acid encodes a protein comprising the amino acid sequence shown by SEQ ID NO:13 (human GFRα1 protein isoform b)), or a nucleic acid comprising a base sequence that hybridizes with the base sequence shown by SEQ ID NO:14 under high stringent conditions, and encoding a protein or peptide having substantially the same quality of activity as the above-described protein comprising the amino acid sequence shown by SEQ ID NO:13 and the like.
Useful nucleic acids capable of hybridizing with the base sequence shown by SEQ ID NO:12 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:12.
Useful nucleic acids capable of hybridizing with the base sequence shown by SEQ ID NO:14 under high stringent conditions include, for example, a nucleic acid comprising a base sequence having a homology of about 60% or more, preferably about 70% or more, more preferably about 80% or more, particularly preferably about 90% or more, to the base sequence shown by SEQ ID NO:14.
Homology of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool).

The base sequence of the DNA can be converted according to a method known per se, such as the ODA-LA PCR method, the Gapped duplex method, or the Kunkel method, or a method based thereon, using PCR, a commonly known kit, for example, Mutan^{™}-super Express Km (Takara Bio Inc.), Mutan^{™}-K (Takara Bio Inc.) and the like.

The cloned DNA that encodes the GFRα1 or the partial peptide thereof can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codons can be added using an appropriate synthetic DNA adapter. To obtain a DNA that encodes a fusion protein between an extracellular region of GFRα1 and another protein (peptide) or a salt thereof, a DNA that encodes a GFRα1 extracellular region cloned or synthesized in the same manner as the above-described method and a DNA that encodes another protein (peptide) can be joined by a method known per se or a method based thereon.

By transforming the host with an expression vector comprising a DNA that encodes the antigen III of the present invention, acquired as described above, and culturing the transformant obtained, the antigen III of the present invention can be produced.
An expression vector for the antigen III of the present invention can be produced by, for example, (a) cutting out a desired DNA fragment from the DNA that encodes the antigen III of the present invention, and (b) joining the DNA fragment downstream of a promoter in an appropriate expression vector.

Useful vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter used in the present invention may be any promoter appropriate for the host used to express the gene. For example, when an animal cell is used as the host, the SRα promoter, the SV40 promoter, the LTR promoter, the CMV promoter, the HSV-TK promoter and the like can be mentioned. Of these promoters, the CMV (cytomegalovirus) promoter, the SRα promoter and the like are preferably used.

When the host is a bacterium of the genus Escherichia, the trp promoter, the lac promoter, the recA promoter, the λP_{L} promoter, the lpp promoter, the T7 promoter and the like are preferred. When the host is a bacterium of the genus Bacillus, the SPO1 promoter, the SPO2 promoter, the penP promoter and the like are preferred. When the host is yeast, the PHO5 promoter, the PGK promoter, the GAP promoter, the ADH promoter and the like are preferred. When the host is an insect cell, the polyhedrin promoter, the P10 promoter and the like are preferred.

Useful expression vectors include, in addition to the above, expression vectors that optionally comprise an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene-defective Chinese hamster cell is used and the dhfr gene is used as the selection marker, a target gene can also be selected using a thymidine-free medium.

In addition, as required, a signal sequence that matches with the host may be added to the 5'-terminal side of the DNA encoding antigen III of the present invention. Useful signal sequences include a PhoA signal sequence, an OmpA signal sequence and the like when the host is a bacterium of the genus Escherichia; an α-amylase signal sequence, a subtilisin signal sequence and the like when the host is a bacterium of the genus Bacillus; an MFα signal sequence, an SUC2 signal sequence and the like when the host is yeast; and an insulin signal sequence, an α-interferon signal sequence, an antibody molecule signal sequence and the like when the host is an animal cell.

Using the thus-constructed vector comprising a DNA that encodes the antigen III of the present invention, a transformant can be produced.

As useful examples of the host, a bacterium of the genus Escherichia, a bacterium of the genus Bacillus, yeast, an insect cell, an insect, an animal cell, and the like can be mentioned.

As specific examples of the bacterium of the genus *Escherichia, Escherichia* coli K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)), and the like can be mentioned.

As useful examples of the bacterium of the genus Bacillus, Bacillus subtilis MI114 (Gene, Vol. 24, 255 (1983)), 207-21 (Journal of Biochemistry, Vol. 95, 87 (1984)) and the like can be mentioned.

As useful examples of the yeast, Saccharomyces cerevisiae AH22, AH22R⁻, NA87-11A, DKD-5D and 20B-12, Schizosaccharomyces pombe NCYC1913 and NCYC2036, Pichia pastoris KM71 and the like can be mentioned.

As useful examples of the insect cell, Spodoptera frugiperda cell (Sf cell), MG1 cell derived from the mid-intestine of Trichoplusia ni, High Five^{™} cell derived from an egg of Trichoplusia ni, cell derived from Mamestra brassicae, cell derived from Estigmena acrea, and the like can be mentioned when the virus is AcNPV. When the virus is BmNPV, Bombyx mori N cell (BmN cell) and the like can be used. As useful examples of the Sf cell, Sf9 cell (ATCC CRL1711), Sf21 cell (both in Vaughn, J. L. et al., In Vivo, 13, 213-217 (1977)), and the like can be mentioned.

As useful examples of the insect, a larva of Bombyx mori (Maeda et al., Nature, Vol. 315, 592 (1985)), and the like can be mentioned.

As useful examples of the animal cell, monkey cell COS-7, Vero cell, Chinese hamster cell CHO (hereinafter abbreviated as CHO cell), Chinese hamster cell (CHO) lacking the dhfr gene (hereinafter abbreviated as CHO(dhfr⁻) cell), mouse L cell, mouse AtT-20 cell, mouse myeloma cell, mouse ATDC5 cell, mouse NS0 cell, mouse FM3A cell, rat GH3 cells, human FL cell, human fetal HEK293 cell, human fetal cell 293F cell and the like can be mentioned.

Transformation can be performed according to the choice of host by a commonly known method.
A bacterium of the genus *Escherichia* can be transformed, for example, in accordance with a method described in Proc. Natl. Acad. Sci. USA, Vol.69, 2110 (1972), Gene, Vol.17, 107 (1982) and the like.

A bacterium of the genus *Bacillus* can be transformed, for example, according to a method described in Molecular & General Genetics, Vol.168, 111 (1979) and the like.

Yeast can be transformed, for example, in accordance with a method described in Methods in Enzymology, Vol.194, 182-187 (1991), Proc. Natl. Acad. Sci. USA, Vol.75, 1929 (1978) and the like.

An insect cell or insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, Vol.52, 456 (1973).

Thus, a transformant transformed with an expression vector comprising a DNA that encodes the antigen III of the present invention can be obtained.

Transformation can be performed according to the choice of host by a commonly known method.
When a transformant whose host is a bacterium of the genus *Escherichia* or a bacterium of the genus *Bacillus* is cultured, the culture medium used is preferably a liquid medium, in which a carbon source, a nitrogen source, an inorganic substance and others necessary for the growth of the transformant are contained. As examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, and potato extract can be mentioned; as examples of the inorganic substance, calcium chloride, sodium dihydrogen phosphate, magnesium chloride and the like can be mentioned.
In addition, yeast extract, vitamins, a growth promoting factor and the like may be added. The pH of the medium is desirably about 5 to 8.

As an example of the medium used to culture a bacterium of the genus *Escherichia,* an M9 medium comprising glucose and casamino acid [Miller, Journal of Experiments in Molecular Genetics, 431-433, Cold Spring Harbor Laboratory, New York, 1972] is preferable. As required, in order to increase promoter efficiency, a chemical agent, for example, 3β-indolylacrylic acid, may be added to the medium.

When the host is a bacterium of the genus *Escherichia,* cultivation is normally performed at about 15 to 43°C for about 3 to 24 hours, and the culture may be aerated or agitated as necessary.

When the host is a bacterium of the genus *Bacillus,* cultivation is normally performed at about 30 to 40°C for about 6 to 24 hours, and the culture may be aerated or agitated as necessary.

When a transformant whose host is yeast is cultured, as examples of the medium, Burkholder's minimal medium [Proc. Natl. Acad. Sci. USA, Vol.77, 4505(1980)] and an SD medium supplemented with 0.5% casamino acid [Proc. Natl. Acad. Sci. USA, Vol.81, 5330(1984)] can be mentioned. The pH of the medium is preferably adjusted to about 5 to 8. Cultivation is normally performed at about 20°C to 35°C for about 24 to 72 hours, and the culture may be aerated or agitated as necessary.

When a transformant whose host is an insect cell or insect is cultured, as the medium, Grace's Insect Medium (Nature, 195, 788(1962)) supplemented with inactivated 10% bovine serum and other additives as appropriate and the like are used. The pH of the medium is preferably adjusted to about 6.2 to 6.4. Cultivation is normally performed at about 27°C for about 3 to 5 days, and the culture may be aerated or agitated as necessary.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally performed at about 30 to 40°C for about 15 to 60 hours, and the culture may be aerated or agitated as necessary.

Thus, the antigen III of the present invention can be produced in the cells, on the cell membrane or out of the cells of the transformant.

Separation and purification of the GFRα1 from the above-described culture can be performed by, for example, the method described below.

When the antigen III of the present invention is extracted from a cultured bacterium or cells, a method is used as appropriate wherein the bacterium or cells are collected by a commonly known method after cultivation, suspended in an appropriate buffer solution, and disrupted by means of sonication, lysozyme and/or freeze-thawing and the like, after which a crude extract of the protein is obtained by centrifugation or filtration. The buffer solution may contain a protein denaturant such as urea or guanidine hydrochloride and a surfactant such as Triton X-100^{™}. When the antigen III of the present invention is secreted in the culture broth, the bacterium or cells are separated from the supernatant by a method known per se, and the supernatant is collected, after completion of the cultivation.

Purification of the antigen III of the present invention contained in the thus-obtained culture supernatant or extract can be performed by an appropriate combination of methods of separation/purification known per se. These commonly known methods of separation/purification include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like.

When the antigen III of the present invention thus obtained is a free form, the free form can be converted to a salt by a method known per se or a method based thereon; conversely, when the protein is obtained in the form of a salt, the salt can be converted to a free form or another salt by a method known per se or a method based thereon.

The antigen III of the present invention produced by the transformant can be optionally modified or partially deprived of a polypeptide by allowing an appropriate protein-modifying enzyme to act thereon before the purification or after the purification. As the protein-modifying enzyme used, for example, trypsin, chymotrypsin, arginyl endopeptidase, protein kinase, glycosidase and the like are used.

The presence of the antigen III of the present invention thus produced can be measured by an enzyme immunoassay, Western blotting and the like using a specific antibody.

(c) GFRα1-expressing mammalian cells themselves can be used directly as the antigen III of the present invention. As the mammalian cells, natural cells as described in section (a) above, cells transformed by a method as described in section (b) above and the like can be used. The host used for the transformation may be any cells collected from humans, monkeys, rats, mice, hamsters and the like; HEK293 cells, COS7 cells, CHO-K1 cells, NIH3T3 cells, Balb3T3 cells, FM3A cells, L929 cells, SP2/0 cells, P3U1 cells, NSO cells, B16 cells, or P388 cells and the like are preferably used.
(d) A peptide having 1 kind or 2 kinds or more of the same antigen determinant as that of a GFRα1 can be produced according to a commonly known method of peptide synthesis, or by cleaving a GFRα1 with an appropriate peptidase. The method of peptide synthesis may be any of, for example, a solid phase synthesis process and a liquid phase synthesis process. That is, a desired peptide can be produced by condensing a partial peptide or amino acids capable of constituting the peptide and the remaining portion, and eliminating any protecting group the resultant product may have. As examples of the commonly known methods of condensation and elimination of the protecting group, the methods described below can be mentioned.
   (i) M. Bodanszky and M.A. Ondetti, Peptide Synthesis, Interscience Publishers, New York (1966)
   (ii) Schroeder and Luebke, The Peptide, Academic Press, New York (1965)
   (iii) Nobuo Izumiya, et al.: Peptide Gosei-no-Kiso to Jikken, published by Maruzen Co. (1975);
   (iv) Haruaki Yajima and Shunpei Sakakibara: Seikagaku Jikken Koza 1, Tanpakushitsu no Kagaku IV, 205 (1977)
   (v) Haruaki Yajima, ed.: Zoku Iyakuhin no Kaihatsu, Vol. 14, Peptide Synthesis, published by Hirokawa Shoten.

After the reaction, the partial peptide used in the present invention can be purified and isolated by a combination of ordinary methods of purification, for example, solvent extraction, distillation, column chromatography, liquid chromatography, recrystallization and the like. When the peptide obtained by the above-described method is a free form, the free form can be converted to an appropriate salt by a commonly known method; conversely, when the peptide is obtained in the form of a salt, the salt can be converted to a free form by a commonly known method.

### (2) Preparation of monoclonal antibody

### (a) Preparation of monoclonal antibody producing cell by hybridoma method

The antigen III of the present invention is administered to a warm-blooded animal. The method of immunization may be any method allowing promotion of antibody production; intravenous injection, intraperitoneal injection, intramuscular injection or subcutaneous injection and the like are preferably used.
Natural mammalian cells or transformed mammalian cells that express the protein III of the present invention can be injected to an immunized animal in a state suspended in a medium used for tissue culture (e.g., RPMI1640) or a buffer solution (e.g., Hanks' Balanced Salt Solution).

The antigen III of the present invention permits direct use for immunization in an insolubilized form. The antigen III of the present invention may be used for immunization in the form of a conjugate thereof bound or adsorbed to a suitable carrier. Regarding the mixing ratio of the carrier and the antigen III of the present invention (hapten), any carrier can be bound or adsorbed in any ratio, as long as an antibody against the antigen III of the present invention bound or adsorbed to the carrier is efficiently produced; usually, a natural or synthetic polymeric carrier in common use for preparation of an antibody against a hapten antigen, bound or adsorbed in a ratio of 0.1 to 100 parts by weight to 1 part by weight of the hapten, can be used. As examples of the natural polymeric carrier, the serum albumin of a mammal such as cattle, rabbit, or human, the thyroglobulin of a mammal such as cattle or rabbit, the hemoglobin of a mammal such as cattle, rabbit, human, or sheep, keyhole limpet hemocyanin and the like are used. As examples of the synthetic polymeric carrier, various latexes of polymers or copolymers of polyamino acids, polystyrenes, polyacryls, polyvinyls, polypropylenes and the like, and the like can be used.

Various condensing agents can be used for crosslinking the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like are conveniently used. When amino groups are crosslinked together, it is also possible to react one amino group with an activated ester reagent having a dithiopyridyl group (for example, 3-(2-pyridyldithio)propionic acid N-succinimidyl (SPDP) and the like), followed by reduction, to introduce the thiol group, and to introduce a maleimide group into the other amino group using a maleimide activated ester reagent, followed by a reaction of both.

To increase antibody productivity in this administration, complete Freund's adjuvant or incomplete Freund's adjuvant may be administered. The administration is normally performed every 2 to 6 weeks, in a total of about 2 to 10 times. In preparing the monoclonal antibody III of the present invention, DNA immunization may be utilized (see, for example, Nature, Vol.356, terms 152-154). As examples of the warm-blooded animal used, monkeys, rabbits, dogs, guinea pigs, mice, rats, sheep, goat, and chicken can be mentioned, and mice and rats are preferably used.

In preparing monoclonal antibody-producing cells, a monoclonal antibody-producing hybridoma can be prepared by selecting an individual showing an antibody titer from among antigen-immunized warm-blooded animals, for example, mice, collecting the spleen or lymph nodes 2 to 5 days after final immunization, and fusing antibody-producing cells contained therein with myeloma cells of the same or different animal species. A measurement of antibody titer in antiserum may be made by, for example, reacting the labeled protein described below with the antiserum, and thereafter determining the activity of the labeling agent bound to the antibody. The fusion may be operated by a known method, for example, the method of Koehler and Milstein [Nature, 256, 495 (1975)]. As examples of fusogen, polyethylene glycol (PEG), Sendai virus and the like can be mentioned, and PEG is preferably used.

As examples of the myeloma cell, NS-1, P3U1, SP2/0, AP-1 and the like can be mentioned, and SP2/0 or P3U1 and the like are preferably used. A preferable ratio of the number of antibody-producing cells (splenocytes) and number of myeloma cells used is generally about 1:1 to 20:1; cell fusion can be efficiently performed by adding a PEG (preferably PEG1000 to PEG6000) at concentrations of about 10 to 80%, and conducting incubation generally at 20 to 40°C, preferably at 30 to 37°C, generally for 1 to 10 minutes.

Electrofusion may be used for cell fusion to prepare monoclonal antibody-producing cells.

Selection of a hybridoma can be performed according to a method known per se or a method based thereon. This selection can normally be performed using an animal cell culture medium supplemented with HAT (hypoxanthine, aminopterin, thymidine). As the medium for selection and breeding, any medium can be used, as long as the hybridoma can grow therein. For example, an RPMI 1640 medium containing 1 to 20%, preferably 10 to 20%, fetal bovine serum, a GIT medium (Wako Pure Chemical Industries, Ltd.) containing 1 to 10% fetal bovine serum or a serum-free medium for hybridoma culture (SFM-101, Nissui Pharmaceutical Co., Ltd.) and the like can be used. Cultivation temperature is normally 20 to 40°C, preferably about 37°C. Cultivation time is normally 5 days to 3 weeks, preferably 1 week to 2 weeks. Cultivation can normally be performed in the presence of 5% gaseous carbon dioxide.

For screening monoclonal antibody-producing hybridomas, various methods can be used; for example, a method wherein a hybridoma culture supernatant is added to a solid phase (e.g., microplates) having a protein antigen or protein-expressing cells adsorbed directly thereto or along with a carrier, then an anti-immunoglobulin antibody (for example, anti-mouse immunoglobulin antibody is used in cases where the splenocytes used for cell fusion are from a mouse) or protein A, labeled with a radioactive substance, enzyme or the like, is added, and the monoclonal antibody bound to the solid phase is detected, a method wherein a hybridoma culture supernatant is added to a solid phase having an anti-immunoglobulin antibody or protein A adsorbed thereto, a protein labeled with a radioactive substance, enzyme or the like is added, and the monoclonal antibody bound to the solid phase is detected, and the like can be mentioned.

### (b) Preparation of monoclonal antibody by other methods

The method of preparing the antibody III of the present invention is not limited to the method described in (a); for example, what is called the antibody display technique, wherein an antibody gene library prepared from human or warm-blooded animal (e.g., monkeys, rabbits, dogs, guinea pigs, mice, rats, sheep, goat, camel, chicken and the like) B lymphocytes by a commonly known method, presented on the cell surfaces of bacteriophages, Escherichia coli, yeast, animal cells and the like, on ribosome and the like, can be used [Nature Biotechnology 23, 1105 (2005)]. The human or warm-blooded animal may be naive, and may also be a cancer patient with high expression of the antigen III of the present invention or a warm-blooded animal immunized with the antigen III of the present invention by the method described in (a). The form of the antibody to be presented to the cell surface is exemplified by, but not limited to, the IgG molecule, IgM molecule, Fab fragment, single-chain Fv (scFv) fragment and the like.

The gene for a monoclonal antibody (fragment) that specifically binds to the antigen III of the present invention is obtained by reacting antibody (fragment)-presenting cells or antibody (fragment) presenting ribosomes that are carrying the above-described antibody gene library with the antigen III of the present invention for a given time, washing away the non-specifically binding portion, thereafter eluting and recovering the portion that specifically binds to the antigen III of the present invention, allowing the antibody (fragment)-presenting cells or antibody (fragment)-presenting ribosomes to grow by a commonly known method, thereafter repeating this procedure several times, and isolating the desired product from finally cloned antibody (fragment)-presenting cells or antibody (fragment)-presenting ribosomes by a commonly known method. The monoclonal antibody fragment gene thus obtained can be recombined with the corresponding region of the IgG antibody gene by a commonly known method to obtain a monoclonal IgG antibody gene.

The antibody III of the present invention can also be obtained by immunizing antibody-producing cells isolated from a human or the above-described warm-blooded animal with the antigen III of the present invention by a method known per se in vitro, and thereafter preparing a hybridoma in the same manner as (a).

### (c) Production of monoclonal antibody

The monoclonal antibody III of the present invention can be produced by culturing a monoclonal antibody-producing hybridoma obtained in (a), or a recombinant cell line wherein an antibody gene isolated by a commonly known method from a monoclonal antibody-producing hybridoma obtained in (a) or a monoclonal antibody gene obtained in (b) is artificially expressed. The monoclonal antibody III of the present invention can also be produced by inserting the antibody gene in a warm-blooded animal or plant chromosome by a commonly known method, and allowing the antibody III to be produced in warm-blooded animal blood, milk, or eggs, plants, fungi and the like [Curr. Opin. Biotevhnol. 7, 536 (1996), Nature Rev. Genet 4, 794 (2003), Appl. Environ. Microbiol. 70, 2567 (2004)]. Useful warm-blooded animals include, for example, bovine, goat, sheep, pigs, chicken, mice, rabbits and the like. Useful plants include tobacco, corn, potato, duckweed and the like.

The monoclonal antibody III of the present invention can be purified from the above-described monoclonal antibody-containing material by a method known per se, for example, a method of immunoglobulin separation and purification [e.g., salting-out, alcohol precipitation, isoelectric point precipitation, electrophoresis, absorption-desorption using an ion exchanger (e.g., DEAE) or a hydrophobicity column, ultracentrifugation, gel filtration, affinity purification for separating and purifying only an antibody by means of a carrier wherein an antigen or a substance with affinity for the antibody, such as protein A or protein G, has been immobilized].

### (3) Preparation of polyclonal antibody

The polyclonal antibody III of the present invention can be produced by a method known per se or a method based thereon. For example, the polyclonal antibody III of the present invention can be produced by preparing a complex of the above-described the antigen III of the present invention and a carrier protein, immunizing a warm-blooded animal with the complex in the same manner as the above-described method of monoclonal antibody production, collecting the product containing an antibody against the antigen from the immunized animal, and separating and purifying the antibody.
Regarding the complex of an antigen and carrier protein used to immunize a warm-blooded animal, any kind of carrier protein can be crosslinked at any mixing ratio of carrier and hapten, as long as an antibody against the carrier-crosslinked immunized antigen is efficiently produced; for example, a method wherein bovine serum albumin, bovine thyroglobulin, KLH or the like is coupled at a ratio of about 0.1 to 20, preferably about 1 to 5, parts by weight per 1 part by weight of hapten, can be used.
For coupling of an antigen and a carrier protein, various condensing agents can be used; active ester reagents containing glutaraldehyde, carbodiimide, a maleimide active ester, a thiol group or a dithiopyridyl group, and the like are used.
The condensation product, as is or along with a carrier or a diluent, is administered to a warm-blooded animal at a site permitting antibody production. To increase antibody productivity in this administration, complete Freund's adjuvant or incomplete Freund's adjuvant may be administered. The administration can normally be performed every 2 to 6 weeks, in a total of about 3 to 10 times.
A polyclonal antibody can be collected from blood, ascites fluid, breast milk, egg and the like of a warm-blooded animal immunized by the above-described method.
Polyclonal antibody titer in antiserum can be determined in the same manner as the above-described determination of antibody titer in serum. Separation and purification of a polyclonal antibody can be performed by the same method of immunoglobulin separation and purification as the above-described separation and purification of monoclonal antibody.

A nucleic acid comprising a base sequence complementary to the target region of the desired nucleic acid, i.e., a nucleic acid capable of hybridizing with the desired nucleic acid, can be described as being 'antisense' against the desired nucleic acid. Meanwhile, a nucleic acid comprising a base sequence having a homology to the target region of the desired nucleic acid can be described as being 'sense' against the desired nucleic acid. Here, 'having a homology' or 'being complementary' refers to having an indentity or complementarity of about 70% or more, preferably about 80% or more, more preferably about 90% or more, most preferably about 95% or more between base sequences.

A nucleic acid comprising a base sequence complementary to the base sequence that encodes GFRα1 or a portion thereof (hereinafter, also referred to as 'antisense GFRα1' or 'the antisense nucleic acid III of the present invention') can be designed and synthesized on the basis of the base sequence information on the cloned or determined nucleic acid that encodes GFRα1. Such a nucleic acid is capable of inhibiting the replication or expression of the gene that encodes GFRα1. Specifically, antisense GFRα1 is capable of hybridizing with the RNA transcribed from the gene that encodes GFRα1, and inhibiting the synthesis (processing) or function (translation into protein) of mRNA.

The target region of antisense GFRα1 is not particularly limited in its length as long as the translation into GFRα1 protein is inhibited as a result of hybridization of an antisense nucleic acid, and it may be the whole sequence or a partial sequence of the mRNA that encodes the protein, which can be exemplified by a short strand of about 15 bases and a long strand of the whole mRNA or early transcription product. In consideration of the ease of synthesis and the issue of antigenicity, an oligonucleotide consisting of about 15 to 30 bases is preferable, but this is not to be construed as limiting. Specifically, for example, the 5'-end hairpin loop, 5'-end 6-base-pair repeat, 5'-end untranslated region, translation initiation codon, protein coding region, translation termination codon, 3'-end untranslated region, 3'-end palindrome region, and 3'-end hairpin loop of the nucleic acid that encodes GFRα1 can be chosen as the target region, and any region in the gene that encodes GFRα1 can be chosen as the target. For example, the intron portion of the gene is preferably used as the target region.
Furthermore, antisense GFRα1 may be one that not only capable of hybridizing with mRNA or early transcription product that encodes GFRα1 to inhibit the translation into protein, but also capable of binding to the gene that encodes GFRα1 which is a double-stranded DNA to form a triplex and inhibit the transcription of RNA.

Examples of the antisense nucleic acid include deoxyribonucleotides containing 2-deoxy-D-ribose, ribonucleotides containing D-ribose, other types of nucleotides which are N-glycosides of the purine or pyrimidine base, or other polymers having non-nucleotide backbones (e.g., commercially available protein nucleic acids and synthetic sequence-specific nucleic acid polymers) or other polymers containing special linkages (provided that the polymers contain nucleotides having such a configuration that allows base pairing or base stacking, as is found in DNA or RNA) and the like. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates and the like) and those with charged linkages or sulfur-containing linkages (e.g., phosphorothioates, phosphorodithioates and the like), those having side chain groups such as proteins (nucleases, nuclease inhibitors, toxins, antibodies, signal peptides, poly-L-lysine and the like), saccharides (e.g., monosaccharides, and the like), those with intercalators (e.g., acridine, psoralen and the like), those containing chelators (e.g., metals, radioactive metals, boron, oxidative metals and the like), those containing alkylating agents, those with modified linkages (e.g., α anomeric nucleic acids and the like), and the like. Herein the terms 'nucleoside' , 'nucleotide' and 'nucleic acid' are used to refer to moieties that contain not only the purine and pyrimidine bases, but also other heterocyclic bases, which have been modified. These modifications may include methylated purines and pyrimidines, acylated purines and pyrimidines or other heterocyclic rings. Modified nucleotides and modified nucleotides may also have modifications on the sugar moiety thereof, wherein, for example, one or more hydroxyl groups may optionally be substituted with a halogen atom, an aliphatic group, and the like, or may be converted to functional groups such as ether or amine.

Preferably, the antisense nucleic acid is an optionally modified RNA or DNA. Specific examples of the modified nucleic acid (RNA, DNA) include, but are not limited to, those resistant to degradation such as sulfur derivatives, thiophosphate derivatives of nucleic acids, polynucleosideamide and oligonucleosideamide. Antisense GFRα1 can preferably be designed with the following aims. Specifically, antisense nucleic acid in cells is further stabilized, the cell permeability of antisense nucleic acid is increased, affinity for target sense strand is increased, and, the toxicity, if any, of antisense nucleic acid is reduced. Many such modifications are known in the art, and are disclosed in, for example, J. Kawakami et al., Pharm Tech Japan, Vol.8, pp.247, 1992; Vol.8, pp.395, 1992; S.T. Crooke et al. ed., Antisense Research and Applications, CRC Press, 1993 and elsewhere.

The antisense nucleic acid may contain altered or modified sugars, bases or linkages, and can be provided in a specialized form such as liposomes or microspheres, or can be applied to gene therapy, or can be provided in combination with attached moieties. Such attached moieties include polycations such as polylysine that act as charge neutralizers of the phosphate backbone, or hydrophobic moieties such as lipids (e.g., phospholipids, cholesterols and the like) that enhance the interaction with cell membranes or increase uptake of the nucleic acid. Preferred lipids to be attached are cholesterols or derivatives thereof (e.g., cholesteryl chloroformate, cholic acid and the like). These moieties can be attached to the nucleic acid at the 3' or 5'-end thereof and can also be attached thereto via a base, sugar, or intramolecular nucleoside linkage. Other moieties may be capping groups specifically placed at the 3' or 5'-end of the nucleic acid to prevent degradation by nucleases such as exonuclease and RNase. Such capping groups include, but are not limited to, hydroxyl protecting groups known in the art, including glycols such as polyethylene glycol and tetraethylene glycol and the like.

A ribozyme capable of specifically cleaving an RNA (mRNA or early transcription product and the like) that encodes GFRα1 in the coding region (in case of early transcription product, the intron portion is included) can also be included in the antisense GFRα1. 'Ribozyme' refers to an RNA having an enzyme activity for nucleic acid cleavage; however, since it has recently been demonstrated that an oligo-DNA having the base sequence of the enzyme activity site also has such nucleic acid cleavage activity, this term is used herein as including DNA, as long as it has sequence-specific nucleic acid cleavage activity. The most versatile ribozyme is self-splicing RNA, which is found in infectious RNAs such as viroid and virusoid, and is known in the hammerhead type, hairpin type and the like. The hammerhead type exhibits enzyme activity with about 40 bases, and it is possible to specifically cleave only a target mRNA by rendering several bases at both ends adjacent to the hammerhead structure portion (about 10 bases in total) complementary to the desired cleavage site of mRNA. Because this type of ribozyme has RNA as the only substrate, the same has a further advantage that genomic DNA is never targeted. When RET mRNA assumes a double-stranded structure per se, the target sequence can be rendered single-stranded by using a hybrid ribozyme coupled with an RNA motif derived from a viral nucleic acid capable of specifically binding to RNA helicase [Proc. Natl. Acad. Sci. USA, 98(10): 5572-5577 (2001)]. Furthermore, when ribozyme is used in the form of an expression vector comprising the DNA that encodes the same, the ribozyme may be a hybrid ribozyme further coupled with a sequence of altered tRNA to promote the transfer of the transcription product to cytoplasm [Nucleic Acids Res., 29(13): 2780-2788 (2001)].

A double-stranded oligo-RNA (siRNA) (siRNA against an RNA that encodes GFRα1) having a base sequence complementary to a partial sequence in the coding region of an RNA (mRNA or early transcription product and the like) that encodes GFRα1 (in case of early transcription product, the intron portion is included) can also be included in antisense GFRα1. The phenomenon of so-called RNA interference (RNAi), in which introducing short double-stranded RNA into a cell results in the degradation of a mRNA complementary to one of the chains of the RNA, is known to occur in nematodes, insects, plants, and the like, but since it was confirmed that this phenomenon also occurs in mammalian cells [Nature, 411 (6836): 494-498 (2001)], it has been widely used as an alternative to ribozyme.

The antisense nucleic acid III of the present invention can be prepared by determining a target region of mRNA or early transcription product on the basis of the information of a cDNA sequence or a genomic DNA sequence that encodes GFRα1, and synthesizing a sequence complementary thereto using a commercially available DNA/RNA synthesizer (Applied Biosystems, Beckman and the like). siRNA having an RNAi activity can be prepared by synthesizing a sense strand and an antisense strand respectively with the DNA/RNA automatic synthesizer, denaturing in a suitable annealing-buffer solution at, for example, about 90°C to 95°C for about 1 minute, and annealing at about 30°C to 70°C for about 1 to 8 hours. In addition, a longer double-stranded polynucleotide can be prepared by synthesizing complementary oligonucleotide strands in an alternately overlapping manner, annealing the oligonucleotides, and ligating with ligase.

The gene expression inhibitory activity of antisense GFRα1 can be examined using a transformant containing a nucleic acid that encodes GFRα1, an in vivo or in vitro GFRα1-encoding-gene expression system or an in vivo or in vitro GFRα1 translation system.

The above-described substances that inhibit a function (for example, GFRα1 activity and expression) of GFRα1, such as the antibody III of the present invention and the antisense nucleic acid III of the present invention, have, for example, the following uses.

As shown in an Example below, by allowing GDNF to act on cancer cells (for example, breast cancer cells), cell growth is promoted, and this cell growth is suppressed by siRNA against GFRα1. This fact shows that the growth of a cancer cells (for example, breast cancer cells) is promoted by GDNF/GFRα1/RET signal activation, and that a substance capable of inhibiting an activity or expression of GFRα1 inhibits the growth of a cancer cells (for example, breast cancer cells), and is effective in the prophylaxis/treatment of cancers (for example, breast cancer).

Because the antibody III of the present invention is capable of inhibiting GFRα1 activity by binding specifically to GFRα1, and also because the antisense nucleic acid III of the present invention is capable of inhibiting GFRα1 expression, it is possible to inhibit an activity or expression of GFRα1 in cancer cells by administering the antibody III of the present invention to a cancer (for example, breast cancer) patient, or administering the antisense nucleic acid III of the present invention to a patient to introduce (and express) the same into target cells, to thereby inhibit the growth of the cancer cells, and prevent/treat cancers.

As shown in an Example below, GFRα1 is highly expressed on the surface of cancer cells (for example, breast cancer cells). Therefore, the antibody I of the present invention is capable of killing cancer cells and preventing/treating cancers by binding to RET on the cancer cell surface, and inducing antibody-dependent cellular cytotoxicity (ADCC) or complement-dependent cytotoxicity (CDC).

Therefore, a pharmaceutical comprising the above-described substance that inhibits a function of GFRα1, such as a) the antibody III of the present invention or b) the antisense nucleic acid III of the present invention, can be used as, for example, a prophylactic/therapeutic agent for cancers (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like) (preferably, a prophylactic/therapeutic agent breast cancer), cancer cell apoptosis promoter, cancer cell (preferably, breast cancer cells) growth inhibitor, cancer cell cycle alteration inducer, cancer metastasis suppressant, cancer cell adhesion inhibitor and the like.

When the antibody III of the present invention is used as the above-described prophylactic/therapeutic agent and the like, the antibody can be prepared as a pharmaceutical preparation in accordance with a conventional method.
When the antisense nucleic acid III of the present invention is used as the above-described prophylactic/therapeutic agent and the like, the nucleic acid, as is or after being inserted into an appropriate expression vector such as retrovirus vector, adenovirus vector, or adenovirus associated virus vector in a functional way, can be prepared as a pharmaceutical preparation in accordance with a conventional method. The nucleic acid can be administered as is, or along with an auxiliary for promoting its ingestion, using a gene gun or a catheter such as a hydrogel catheter.

A pharmaceutical comprising a substance that inhibits a function of GFRal such as the antibody III of the present invention or the antisense nucleic acid III of the present invention, is of low toxicity and can be administered in the form of liquid preparations as they are, or as pharmaceutical compositions in suitable dosage forms, to human or non-human mammals (e.g., rats, rabbits, sheep, pigs, bovine, cats, dogs, monkeys and the like), orally or parenterally (e.g., intravascularly, subcutaneously and the like).

The substance inhibiting the function of GFRα1 such as the antibody III or antisense nucleic acid III of the present invention may be administered as is, or may be administered as an appropriate pharmaceutical composition. The pharmaceutical composition used for the administration may comprise the antibody III of the present invention or the antisense nucleic acid III of the present invention and a pharmacologically acceptable carrier, diluent or filler. Such a pharmaceutical composition is provided as a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. The injection can be prepared by, for example, dissolving, suspending or emulsifying the antibody III of the present invention or the antisense nucleic acid III of the present invention in a sterile aqueous or oily solution normally used for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50mol) adduct of hydrogenated castor oil)] and the like. As examples of oily solutions, sesame oil, soybean oil and the like can be used, which may be used in combination with solubilizers such as benzyl benzoate, benzyl alcohol. The injectable preparation prepared is preferably filled in an appropriate ampoule. A suppository used for rectal administration may also be prepared by mixing the above-described antibody or the antisense nucleic acid in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a commonly known method, and may contain a carrier, diluent or filler normally used in the field of pharmaceutical making. As the carrier or filler for tablets, for example, lactose, starch, sucrose, and magnesium stearate are used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. As the content amount of the antibody, it is preferable that normally 5 to 500 mg, particularly 5 to 100 mg for injections or 10 to 250 mg for other dosage forms, per medication unit dosage form, of the above-described antibody be contained. Regarding the content of antisense nucleic acid, it is preferable that the above-described antisense nucleic acid be contained at normally 5 to 500 mg, particularly 5 to 100 mg for an injection, or 10 to 250 mg for other dosage forms, per unit dosage form.

The dosage of the above-described prophylactic/therapeutic agents and the like comprising the antibody III of the present invention varies also depending on the subject of administration, target disease, symptoms, route of administration and the like; for example, when the agent is used for the treatment/prevention of breast cancer in an adult, the antibody III of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

The dosage of the above-described prophylactic/therapeutic agents and the like comprising the antisense nucleic acid III of the present inventions varies also depending on the subject of administration, target disease, symptoms, route of administration and the like; for example, when the agent is used for the treatment/prevention of breast cancer in an adult, the antisense nucleic acid III of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

Each of the above-described compositions may comprise other active ingredients, as long as they do not produce an unwanted interaction when blended with the above-described antibody or antisense nucleic acid.

Furthermore, the substance inhibiting the function of GFRα1 such as the antibody III or the antisense nucleic acid III may be used in combination with other drugs, for example, alkylating agents (e.g., cyclophosphamide, ifosfamide and the like), metabolic antagonists (e.g., methotrexate, 5-fluorouracil and the like), anticancer antibiotics (e.g., mitomycin, adriamycin and the like), plant-derived anticancer agents (e.g., vincristine, vindesine, Taxol and the like), cisplatin, carboplatin, ethopoxide, irinotecan and the like.
The antibody III or antisense nucleic acid III and the above-mentioned drugs may be administered to a patient simultaneously or at different times.

Because the antibody III of the present invention specifically recognizes GFRα1, and can be used for quantitation of GFRα1 in a test liquid, particularly for quantitation by sandwich immunoassay and the like, the same is useful as, for example, a diagnostic reagent for decreased expression or increased expression of the protein and the like. As shown in an Example below, cancer cells (for example, breast cancer cells) express GFRα1, and undergo the action of GDNF, whereby cell growth is promoted; when cancer cells are treated with siRNA against GFRα1 to suppress the amount expressed, the growth of the cancer cells is suppressed. Therefore, by detecting and quantifying GFRα1 in a test sample such as cells, tissue, or body fluid using the antibody III of the present invention, cancers (for example, breast cancer), particularly cancers that are highly sensitive to GDNF (for example, breast cancer) can be detected. Hence, the antibody III of the present invention is useful as a diagnostic reagent for cancers (for example, breast cancer). For example, by quantifying GFRα1 in the sample using the antibody III of the present invention, when an increase in the expression of GFRα1 is detected, the subject can be diagnosed as having a cancer (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like, particularly breast cancer), or as being likely to suffer from a cancer in the future. Furthermore, by quantifying the expression of GFRα1 in cancer cells, the GDNF sensitivity of the cancer can be determined. If an increase in the expression of GFRα1 in cancer cells is detected, the cancer can be judged to be a cancer that is highly sensitive to GDNF, and grows vigorously GDNF-dependently.

As examples of the method of quantifying GFRα1 using the antibody III of the present invention,
(i) a method of quantifying GFRα1 in a test liquid, comprising competitively reacting the antibody III of the present invention, a test liquid and a labeled form of GFRα1, and determining the ratio of labeled GFRα1 bound to the antibody,
(ii) a method of quantifying GFRα1 in a test liquid, comprising simultaneously or sequentially reacting a test liquid, the antibody III of the present invention insolubilized on a carrier and another antibody III of the present invention which has been labeled, and thereafter determining the activity of the labeling agent on the insolubilizing carrier and the like can be mentioned.

In the above-described method of quantitation (ii), the two kinds of antibodies are desirably ones that specifically recognize different portions of GFRα1. For example, provided that one antibody is an antibody that recognizes the N-terminus of GFRα1, the other antibody can be an antibody that reacts with the C-terminus of GFRα1.

As the labeling agent used for the assay methods using a labeled substance, a radioisotope, an enzyme, a fluorescent substance, a luminescent substance and the like are used. As the radioisotope, for example, [¹²⁵I], [¹³¹I], [³H], [¹⁴C] and the like are used; as the above-described enzyme, stable enzymes with a high specific activity are preferable; for example, β-galactosidase, β-glucosidase, alkaline phosphatase, peroxidase, malate dehydrogenase and the like are used; as examples of the fluorescent substance, cyanine fluorescent dyes (e.g., Cy2, Cy3, Cy5, Cy5.5, Cy7 (manufactured by Amersham Biosciences K.K.) and the like), fluorescamine, fluorescein isothiocyanate and the like are used; as examples of the luminescent substance, luminol, luminol derivatives, luciferin, lucigenin and the like are used. Furthermore, a biotin-avidin system can also be used for the binding of the antibody or antigen and the labeling agent.

As the test liquid, when GFRα1 is localized in cells, a cell homogenate obtained by suspending the cells in an appropriate buffer, and then breaking the cells by ultrasonication, freeze-thaw cycling and the like, is used, and when GFRα1 is secreted extracellularly, a cell culture supernatant or a body fluid (blood, serum, plasma, urine, sweat, breast milk and the like) is used. If necessary, the quantification may be carried out after separating and purifying RET from a homogenate, a cell-culture supernatant or a body fluid and the like. In addition, intact cells can be used as the sample, as long as label detection is possible.

The quantification method of GFRα1 using the antibody III of the present invention is not particularly limited. Any quantification method may be used, so long as the amount of an antibody, antigen or antibody-antigen complex corresponding to the amount of antigen (e.g., protein amount) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use, for example, the sandwich method described later.

For insolubilization of the antigen or antibody, physical adsorption may be used, and chemical binding methods conventionally used to insolubilize or immobilize proteins, enzymes and the like may be used as well. As examples of the carrier, insoluble polysaccharides such as agarose, dextran, and cellulose; synthetic resins, for example, polystyrene, polyacrylamide, silicon and the like, or glass and the like can be mentioned.

In the sandwich method, the antibody III of the present invention insolubilized is reacted with a test liquid (primary reaction), then reacted with the antibody III of the present invention labeled (secondary reaction), after which the activity of the labeling agent on the insolubilizing carrier is measured, whereby the amount of the protein III used in the present invention in the test liquid can be quantified. The primary and secondary reactions may be performed simultaneously or with a time lag. The labeling agent and the method for insolubilization can be the same as those described above. In the immunoassay by the sandwich method, the antibody used for the solid phase or the antibody for labeling is not necessarily from one kind, but a mixture of two or more kinds of antibodies may be used for increasing the measurement sensitivity and other purposes.

In the method of measuring GFRα1 by the sandwich method, the antibody III of the present invention used in the primary reaction and that used in the secondary reaction are preferably antibodies having different sites for GFRα1 binding. Hence, for example, provided that the antibody used in the secondary reaction recognizes the C-terminus of GFRα1, the antibody used in the primary reaction is preferably an antibody that recognizes a site other than the C-terminus, for example, the N-terminus.

The antibody III of the present invention can be used in measuring systems other than the sandwich method, for example, the competitive method, the immunometric method, nephelometry, and the like.

In the competitive method, an antigen in a test liquid and a labeled form of antigen are reacted competitively against an antibody, an unreacted labeled antigen (F) is separated from an antibody-bound labeled antigen (B) (B/F separation), and the labeled amount of B or F is determined, thereby to quantify the antigen in the test liquid. The present reaction method includes a liquid phase method in which B/F separation is performed using a soluble antibody as the antibody and using polyethylene glycol or a secondary antibody against the antibody and the like; and a solid phase method in which a solid-phased antibody is used as a primary antibody or a soluble antibody is used as a primary antibody and a solid-phased antibody is used as a secondary antibody.

In the immunometric method, the antigen in a test liquid and a solid-phase-immobilized antigen are competitively reacted with a given amount of the antibody of the present invention labeled, after which the solid phase and the liquid phase are separated, or the antigen in the test liquid and an excess amount of the antibody of the present invention labeled are reacted, and then a solid-phase-immobilized antigen is added to bind the unreacted portion of the antibody of the present invention labeled to the solid phase, after which the solid phase and the liquid phase are separated. Next, the amount of labeling agent in either phase is measured to quantify the amount of antigen in the test liquid.

Also, in nephelometry, the amount of insoluble precipitate resulting from an antigen-antibody reaction in the gel or in the solution is measured. Even when the amount of antigen in the test solution is small and only a small amount of precipitate is obtained, laser nephelometry, which utilizes laser scattering, and the like are preferably used.

Using the antibody III of the present invention, GFRα1 can be quantified, and can also be detected by tissue staining and the like. For these purposes, the antibody molecule itself may be used, and the F(ab')₂, Fab', or Fab fraction of the antibody molecule may also be used.

In applying these individual immunological measurement methods to the method III of the present invention, it is unnecessary to set special conditions, procedures and the like. Making ordinary technical considerations for those skilled in the art to the ordinary conditions and procedures in each method, a measurement system of GFRα1 can be constructed. For details of these general technical means, compendia, books and the like can be referred to.

For example, see edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.

As described above, GFRα1 can be quantified with high sensitivity using the antibody III of the present invention.

The antibody III of the present invention can be used for preparing an antibody column for purification of GFRα1, detecting GFRα1 in each fraction during purification, analyzing the behavior of GFRα1 in test cells and for other purposes.

Because a nucleic acid comprising the base sequence that encodes GFRα1 or a portion thereof (hereinafter, also referred to as 'sense GFRα1'), or a nucleic acid comprising a base sequence complementary to the base sequence or a portion thereof (antisense GFRα1) is capable of detecting an abnormality in the GFRα1-encoding DNA or mRNA (gene abnormality) in a human or other warm-blooded animal (for example, rats, mice, hamsters, rabbits, sheep, goat, pigs, bovine, horses, cats, dogs, monkeys, chimpanzees, birds and the like) when used as a probe and the like, the same is useful as, for example, a gene diagnostic reagent for damage or mutation in the DNA, splicing abnormality or decreased expression in mRNA, or amplification in the DNA, increased expression in mRNA and the like. The nucleic acid comprising a portion of the base sequence that encodes GFRα1 is not particularly limited, as long as it has a length sufficient for a probe (for example, about 15 bases or more), and does not need to encode a partial peptide of GFRα1.
The above-described gene diagnosis using sense or antisense GFRα1 can be performed by, for example, Northern hybridization, quantitative RT-PCR, PCR-SSCP assay, allele-specific PCR, PCR-SSOP assay, DGGE assay, RNase protection assay, PCR-RFLP assay and the like that are known per se.
As shown in an Example below, cancer cells (for example, breast cancer cells) express GFRα1, and undergo the action of GDNF, whereby cell growth is promoted; by treating cancer cells with siRNA against GFRα1 to suppress the amount expressed, the growth of a cancer cells can be suppressed. Therefore, by detecting and quantifying GFRα1 in a test sample such as cells, tissue, or body fluid using the antibody III of the present invention, cancers (for example, breast cancer), particularly cancers that are highly sensitive to GDNF (for example, breast cancer) can be detected. Hence, sense or antisense GFRα1 is useful as a diagnostic reagent for cancers (for example, breast cancer). For example, by quantifying the expression of GFRα1 in the sample using sense or antisense GFRα1, when an increase in the expression of GFRα1 is detected, the subject can be diagnosed as having, for example, a cancer (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like, particularly breast cancer), or as being likely to suffer from a cancer in the future. Furthermore, by quantifying the expression of GFRα1 in cancer cells, the GDNF sensitivity of the cancer can be determined. If an increase in the expression of GFRα1 in cancer cells is detected, the cancer can be judged to be a cancer that is highly sensitive to GDNF, and grows vigorously GDNF-dependently.

### (IV. Substances that inhibit a function of GDNF, GFRα1 or RET (herein, also referred to as GDNF/GFRα1/RET))

The present invention provides a prophylactic/therapeutic agent for cancers (for example, breast cancer) comprising a substance that inhibits a function of GDNF, GFRα1 or RET.

'A function of GDNF, GFRα1 or RET' refers to a function of activating RET by GDNF via interactions of GDNF, GFRα1 and GFRα1. As the 'a function of GDNF, GFRα1 or RET', activation of RET signal by GDNF, promotion of cell growth of cancer (for example, breast cancer) cells and the like by GDNF and the like can be mentioned. Here, in some cases of thyroid cancer and the like, it has been reported that due to an activated mutation of RET or GFRα1, a signal downstream thereof becomes activated GDNF-non-dependently to promote cell growth (non-patent document 1, non-patent document 5), but this is not included in 'a function of GDNF, GFRα1 or RET'.

Substances that inhibit a function of GDNF, GFRα1 or RET include,
(1) an antibody against RET or a partial peptide thereof or a salt thereof, an antibody against GDNF or a partial peptide thereof or a salt thereof, an antibody against GFRα1 or a partial peptide thereof or a salt thereof
   (an antibody against a protein comprising the same or substantially the same amino acid sequence as the amino acid sequence shown by SEQ ID NO:1, SEQ ID NO:3, SEQ ID NO:5, SEQ ID NO:7, SEQ ID NO:9, SEQ ID NO:11 or SEQ ID NO:13 or a partial peptide thereof or a salt thereof),
(2) a low-molecular compound or a salt thereof,
(3) an antisense nucleic acid against a nucleic acid that encodes a protein comprising the same or substantially the same amino acid sequence as the amino acid sequence shown by SEQ ID NO:1, SEQ ID NO:3, SEQ ID NO:5, SEQ ID NO:7, SEQ ID NO:9, SEQ ID NO:11 or SEQ ID NO:13, and
(4) an siRNA against an RNA that encodes a protein comprising the same or substantially the same amino acid sequence as the amino acid sequence shown by SEQ ID NO:1, SEQ ID NO:3, SEQ ID NO:5, SEQ ID NO:7, SEQ ID NO:9, SEQ ID NO:11 or SEQ ID NO:13, which have an activity to inhibit a function of GDNF, GFRα1 or RET (signal transduction inhibitory activity of GDNF, cell growth inhibitory activity of GDNF on cancer (for example, breast cancer) cells and the like).

As the antibody (1), the above-described antibody I, antibody II, and antibody III of the present invention and the like can be used. As the antisense nucleic acid (3), the above-described the antisense nucleic acid I of the present invention, antisense nucleic acid II, antisense nucleic acid III and the like can be used. As the siRNA (4), a double-stranded oligo-RNA having a base sequence complementary to a partial sequence in the coding region of the above-described RNA that encodes GDNF, GFRα1 or RET (mRNA or early transcription product and the like) (in case of early transcription product, the intron portion is included) can be mentioned.

The substance that inhibits a function of GDNF, GFRα1 or RET is preferably the antibody (1). The antibody (1) is capable of inhibiting signal activation downstream of RET by binding specifically to GDNF, GFRα1 or RET to inhibit, for example, the interaction between GDNF and RET, the interaction between GDNF and GFRα1, the interaction between GFRα1 and RET and the like.

Substances that inhibit a function of GDNF, GFRα1 or RET have, for example, the following uses.

As shown in an Example below, RET and GDFα1 are expressed on the cell surfaces of cancer cells (for example, breast cancer cells), applying GDNF to cancer cells (for example, breast cancer cells) activates signals downstream of RET and promotes cell growth, and this cell growth is suppressed by siRNA against RET or GDFα1. This fact shows that in cancer cells (for example, breast cancer cells), functional GDFα1 and RET are expressed, and the growth of a cancer cells (for example, breast cancer cells) is promoted due to activation of the GDNF/GDFα1/RET signal, and that a substance capable of inhibiting this GDNF/GDFα1/RET signal activation inhibits the growth of a cancer cells (for example, breast cancer cells), and is effective in the prophylaxis/treatment of cancers (for example, breast cancer).

Therefore, by administering a substance that inhibits a function of GDNF, GFRα1 or RET to a cancer (for example, breast cancer) patient, it is possible to inhibit GDNF/GDFα1/RET signal activation in cancer cells to thereby inhibit the growth of the cancer cells, and prevent/treat cancers.

Therefore, a pharmaceutical comprising the above-described substance that inhibits a function of GDNF, GFRα1 or RET, can be used as, for example, a prophylactic/therapeutic agent for cancers (e.g., colorectal cancer, breast cancer, lung cancer, prostatic cancer, esophageal cancer, gastric cancer, liver cancer, biliary tract cancer, spleen cancer, renal cancer, urinary bladder cancer, uterine cancer, ovarian cancer, testicular cancer, thyroid cancer, pancreatic cancer, brain tumor, blood tumors and the like) (preferably, a prophylactic/therapeutic agent for breast cancer), cancer cell apoptosis promoter, cancer cell (preferably, breast cancer cells) growth inhibitor, cancer cell cycle alteration inducer, cancer metastasis suppressant, cancer cell adhesion inhibitor and the like.

Particularly, a substance that inhibits a function of GDNF, GFRα1 or RET is useful in the treatment of cancers wherein GFRα1 protein and RET protein are expressed (for example, breast cancer) and cancers wherein GDNF protein, GFRα1 protein and RET protein are expressed (for example, breast cancer). This is because these cancer cells are capable of becoming activated GDNF-dependently and growing. The expression of the GDNF protein, GFRα1 protein and RET protein in cancer cells can be confirmed by flowcytometry and immunohistological analysis using antibodies that are specific for these proteins.

When a substance that inhibits a function of GDNF, GFRα1 or RET is used as the above-described prophylactic/therapeutic agent and the like, the substance can be prepared as a pharmaceutical preparation in accordance with a conventional method.
When the antisense nucleic acid (3) being a substance that inhibits a function of GDNF, GFRα1 or RET is used as the above-described prophylactic/therapeutic agent and the like, the nucleic acid, as is or after being inserted into an appropriate expression vector such as retrovirus vector, adenovirus vector, or adenovirus associated virus vector in a functional way, can be prepared as a pharmaceutical preparation in accordance with a conventional method. The nucleic acid can be administered as is, or along with an auxiliary for promoting its ingestion, using a gene gun or a catheter such as a hydrogel catheter.

A pharmaceutical comprising a substance that inhibits a function of GDNF, GFRα1 or RET is of low toxicity and can be administered in the form of liquid preparations as they are, or as pharmaceutical compositions in suitable dosage forms, to human or non-human mammals (e.g., rats, rabbits, sheep, pigs, bovine, cats, dogs, monkeys and the like), orally or parenterally (e.g., intravascularly, subcutaneously and the like).

The substance inhibiting the function of GDNF, GFRα1 or RET of the present invention may be administered as is, or may be administered as an appropriate pharmaceutical composition. The pharmaceutical composition used for the administration may comprise the substance inhibiting the function of GDNF, GFRα1 or RET and a pharmacologically acceptable carrier, diluent or filler. Such a pharmaceutical composition is provided as a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. The injection can be prepared by, for example, dissolving, suspending or emulsifying the substance inhibiting the function of GDNF, GFRα1 or RET in a sterile aqueous or oily solution normally used for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50mol) adduct of hydrogenated castor oil)] and the like. As examples of oily solutions, sesame oil, soybean oil and the like can be used, which may be used in combination with solubilizers such as benzyl benzoate, benzyl alcohol. The injectable preparation prepared is preferably filled in an appropriate ampoule. A suppository used for rectal administration may also be prepared by mixing the above-described substance inhibiting the function of GDNF, GFRα1 or RETin an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a commonly known method, and may contain a carrier, diluent or filler normally used in the field of pharmaceutical making. As the carrier or filler for tablets, for example, lactose, starch, sucrose, and magnesium stearate are used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. As the content amount of the antibody, it is preferable that normally 5 to 500 mg, particularly 5 to 100 mg for injections or 10 to 250 mg for other dosage forms, per medication unit dosage form, antibody of the above-described (1) be contained. Regarding the content of antisense nucleic acid, it is preferable that the antisense nucleic acid of the above-described (3) be contained at normally 5 to 500 mg, particularly 5 to 100 mg for an injection, or 10 to 250 mg for other dosage forms, per unit dosage form.

The dosage of the above-described prophylactic/therapeutic agents and the like comprising the antibody of the above-described (1) varies also depending on the subject of administration, target disease, symptoms, route of administration and the like; for example, when the agent is used for the treatment/prevention of breast cancer in an adult, the antibody is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

The dosage of the above-described prophylactic/therapeutic agents and the like comprising the antisense nucleic acid of the above-described (3) varies also depending on the subject of administration, target disease, symptoms, route of administration and the like; for example, when the agent is used for the treatment/prevention of breast cancer in an adult, the antibody is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

Each of the foregoing compositions may contain another active ingredient, as long as no undesirable interaction is produced when blended with the substance inhibiting the function of GDNF, GFRα1 or RET.

Furthermore, the substance inhibiting the function of GDNF, GFRα1 or RET may be used in combination with other drugs, for example, alkylating agents (e.g., cyclophosphamide, ifosfamide and the like), metabolic antagonists (e.g., methotrexate, 5-fluorouracil and the like), anticancer antibiotics (e.g., mitomycin, adriamycin and the like), plant-derived anticancer agents (e.g., vincristine, vindesine, Taxol and the like), cisplatin, carboplatin, ethopoxide, irinotecan and the like. The substance inhibiting the function of GDNF, GFRα1 or RET and the above-mentioned drugs may be administered to a patient simultaneously or at different times.

Abbreviations for bases, amino acids and the like used in the present description are based on abbreviations specified by the IUPAC-IUB Commission on Biochemical Nomenclature or abbreviations in common use in relevant fields, some examples of which are given below. When an optical isomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Sec: Selenocysteine

Substituents, protecting groups and reagents frequently mentioned herein are represented by the symbols shown below. Me: Methyl group
Et: Ethyl group
Bu: Butyl group
Ph: Phenyl group
TC: Thiazolidine-4(R)-carboxamide group
Tos: p-Toluenesulfonyl
CHO: Formyl
Bzl: Benzyl
Cl₂-Bzl: 2, 6-Dichlorobenzyl
Bom: Benzyloxymethyl
Z: Benzyloxycarbonyl
Cl-Z: 2-Chlorobenzyloxycarbonyl
Br-Z: 2-Bromobenzyloxycarbonyl
Boc: t-Butoxycarbonyl
DNP: Dinitrophenyl
Trt: Trityl
Bum: t-Butoxymethyl
Fmoc: N-9-Fluorenylmethoxycarbonyl
HOBt: 1-Hydroxybenztriazole
HOOBt: 3,4-Dihydro-3-hydroxy-4-oxo-1,2,3-benzotriazine
HONB:1-Hydroxy-5-norbornene-2,3-dicarboximide
DCC: N, N'-Dicyclohexylcarbodiimide

Each SEQ ID NO in the Sequence Listing shows the following sequence.
[SEQ ID NO: 1]
   SEQ ID NO: 1 shows an amino acid sequence of RET protein·isoform a.
[SEQ ID NO: 2]
   SEQ ID NO: 2 shows a base sequence of DNA encoding RET protein·isoform a having the amino acid sequence shown in SEQ ID NO: 1.
[SEQ ID NO: 3]
   SEQ ID NO: 3 shows an amino acid sequence of RET protein·isoform c.
[SEQ ID NO: 4]
   SEQ ID NO: 4 shows a base sequence of DNA encoding RET protein· isoform c having the amino acid sequence shown in SEQ ID NO: 3.
[SEQ ID NO: 5]
   SEQ ID NO: 5 shows an amino acid sequence of GDNF protein·isoform 1.
[SEQ ID NO: 6]
   SEQ ID NO: 6 shows a base sequence of DNA encoding GDNF protein·isoform 1 having the amino acid sequence shown in SEQ ID NO: 5.
[SEQ ID NO: 7]
   SEQ ID NO: 7 shows an amino acid sequence of GDNF protein·isoform 2.
[SEQ ID NO: 8]
   SEQ ID NO: 8 shows a base sequence of DNA encoding GDNF protein· isoform 2 having the amino acid sequence shown in SEQ ID NO: 7.
[SEQ ID NO: 9]
   SEQ ID NO: 9 shows an amino acid sequence of GDNF protein·isoform 3.
[SEQ ID NO: 10]
   SEQ ID NO: 10 shows a base sequence of DNA encoding GDNF protein· isoform 3 having the amino acid sequence shown in SEQ ID NO: 9.
[SEQ ID NO: 11]
   SEQ ID NO: 11 shows an amino acid sequence of GFRα1 protein· isoform a.
[SEQ ID NO: 12]
   SEQ ID NO: 12 shows a base sequence of DNA encoding GFRα1 protein·isoform a having the amino acid sequence shown in SEQ ID NO: 11.
[SEQ ID NO: 13]
   SEQ ID NO: 13 shows an amino acid sequence of GFRα1 protein·isoform b.
[SEQ ID NO: 14]
   SEQ ID NO: 14 shows a base sequence of DNA encoding GFRα1 protein·isoform b having the amino acid sequence shown in SEQ ID NO: 13.

The present invention is explained in detail in the following by referring to Examples, which are not to be construed as limitative.

### [Example 1]

### Enhancement of cell growth of human breast cancer cell line by GDNF administration

Human breast cancer-derived cell line MCF 7 purchased from American Type Culture Collection (ATCC) was suspended in MEM (Invitrogen) containing 10% fetal calf serum (Hyclone), 1 x MEM nonessential amino acid (Dainippon Pharmaceutical Co., Ltd.), 1 mM MEM sodium pyruvate (Invitrogen), 100 units/mL penicillin G (Wako Pure Chemical Industries, Ltd.) and 100 µg/mL streptomycin sulfate (Wako Pure Chemical Industries, Ltd.) and plated on a 96-well flat-bottomed tissue culture plate at a cell density of 3000 cells in 100 µL per a well.. After culture in a 5% carbon dioxide gas stream at 37°C for 1 day, a suspension of human recombinant GDNF (R&D systems) in 0.1% bovine serum albumin was administered. After culture for 3 more days, a Cell-Counting Kit-8 solution (Wako Pure Chemical Industries, Ltd.) was added at 10 µL per well, and the cells were cultured in a 5% carbon dioxide gas stream at 37°C for 3 more hours. The absorbance at 450 nm was measured to count the cells. As a result, the cells administered with GDNF showed an about 40% increase in the absorbance as compared to the cells without administration (Fig. 1). This result suggests that GDNF induces cell growth of human breast cancer-derived cell line MCF 7.

### [Example 2]

### Suppression of cell growth of human breast cancer cell line by administration of siRNAs against RET gene

Human breast cancer-derived cell line MCF 7 purchased from American Type Culture Collection (ATCC) was suspended in MEM (Invitrogen) containing 10% fetal calf serum (Hyclone), 1 x MEM nonessential amino acid (Dainippon Pharmaceutical Co., Ltd.), 1 mM MEM sodium pyruvate (Invitrogen), 100 units/mL penicillin G (Wako Pure Chemical Industries, Ltd.) and 100 µg/mL streptomycin sulfate (Wako Pure Chemical Industries, Ltd.), cultured in a 5% carbon dioxide stream at 37°C, and transfected with siRNAs.
Specifically, the following 3 kinds of siRNAs (B-Bridge) having an activity to cleave mRNA of RET gene were blended and the blend was subjected to transfection. As a control, a mixture of the following 3 kinds of siRNAs guaranteed not to show no non-specific RNAi effect (B-Bridge, hereinafter to be abbreviated as non-silencing dsRNA) on the cells was used.
<siRNA for RET>
   (SHF27A-0609-1)
      Sense strand : gaccauagcuccugggagaTT (SEQ ID NO: 15)
      Antisense strand: ucucccaggagcuauggucTT (SEQ ID NO: 16)
   (SHF27A-0609-2)
      Sense strand : gaacuugguucuuggaaaaTT (SEQ ID NO: 17)
      Antisense strand: uuuuccaagaaccaaguucTT (SEQ ID NO: 18)
   (SHF27A-0609-3)
      Sense strand : ccacauggauugaaaacaaTT (SEQ ID NO: 19)
      Antisense strand: uuguuuucaauccauguggTT (SEQ ID NO: 20)
<Non-silencing dsRNA>
   (C6A-0126-1)
      Sense strand : auccgcgcgauaguacguaTT (SEQ ID NO: 21)
      Antisense strand: uacguacuaucgcgcggauTT (SEQ ID NO: 22)
   (C6A-0126-2)
      Sense strand : uuacgcguagcguaauacgTT (SEQ ID NO: 23)
      Antisense strand: cguauuacgcuacgcguaaTT (SEQ ID NO: 24)
   (C6A-0126-3)
      Sense strand : uauucgcgcguauagcgguTT (SEQ ID NO: 25)
      Antisense strand: accgcuauacgcgcgaauaTT (SEQ ID NO: 26)
A solution (3 µL) containing the siRNAs for 300 pmol of the RET gene or 300 pmol of the non-silencing dsRNAs was mixed with 100 µL of a suspension of 1 million MCF 7 cells in Nucleofector Solution V (Amaxa biosystems), and the mixture was transfected by Nucleofector program P-020. The entire amount of this mixture was added to 4 mL of an MCF 7 cell culture medium and the cells were cultured for 24 hr andharvested. After the harvested cells were plated on a 96-well flat-bottomed tissue culture plate at a cell density of 3500 cells per well, a suspension of human recombinant GDNF (R&D systems) in 0.1% bovine serum albumin was administered. The cells were cultured for 3 more days, and a Cell-Counting Kit-8 solution (Wako Pure Chemical Industries, Ltd.) was added at 10 µL per well. The cells were stood in a 5% carbon dioxide gas stream at 37°C for 3 more hours, and the absorbance at 450 nm was measured to count the cells.
As a result, the cells administered with siRNAs against the RET gene showed suppression of an increase in the absorbance by administration of GDNF as compared to the cells administered with the non-silencing dsRNAs (Fig. 2). This result suggests that in the absence of the siRNA RET protein activated by GDNF induces cell growth in human breast cancer-derived cell line MCF 7, and the inhibition of the function of RET by siRNA and the like can suppress the growth of a cancer cells.

### [Example 3]

### Suppression of cell growth of human breast cancer cell line by administration of siRNAs against GFRα1 gene

Human breast cancer-derived cell line MCF 7 purchased from American Type Culture Collection (ATCC) was suspended in MEM (Invitrogen) containing 10% fetal calf serum (Hyclone), 1 x MEM nonessential amino acid (Dainippon Pharmaceutical Co., Ltd.), 1 mM MEM sodium pyruvate (Invitrogen), 100 units/mL penicillin G (Wako Pure Chemical Industries, Ltd.) and 100 µg/mL streptomycin sulfate (Wako Pure Chemical Industries, Ltd.), cultured in a 5% carbon dioxide stream at 37°C, and transfected with siRNAs.
Specifically, the following 3 kinds of siRNAs (B-Bridge) having an activity to cleave mRNA of GFRα1 gene were blended and the blend was subjected to transfection. As a control, a mixture of the following 3 kinds of siRNAs guaranteed not to show no non-specific RNAi effect (B-Bridge, hereinafter to be abbreviated as non-silencing dsRNA) on the cells was used.
<siRNA for GFRα1>
   (SHF27A-0610-1)
      Sense strand : gagcagagcugcagcaccaTT (SEQ ID NO: 27)
      Antisense strand: uggugcugcagcucugcucTT (SEQ ID NO: 28)
   (SHF27A-0610-2)
      Sense strand : gcagcugucuaaaggaaaaTT (SEQ ID NO: 29)
      Antisense strand: uuuuccuuuagacagcugcTT (SEQ ID NO: 30)
   (SHF27A-0610-3)
      Sense strand : cucagaaggcuuugggauaTT (SEQ ID NO: 31)
      Antisense strand: uaucccaaagccuucugagTT (SEQ ID NO: 32)
<Non-silencing dsRNA>
   (C6A-0126-1)
      Sense strand : auccgcgcgauaguacguaTT (SEQ ID NO: 21)
      Antisense strand: uacguacuaucgcgcggauTT (SEQ ID NO: 22)
   (C6A-0126-2)
      Sense strand : uuacgcguagcguaauacgTT (SEQ ID NO: 23)
      Antisense strand: cguauuacgcuacgcguaaTT (SEQ ID NO: 24)
   (C6A-0126-3)
      Sense strand : uauucgcgcguauagcgguTT (SEQ ID NO: 25)
      Antisense strand: accgcuauacgcgcgaauaTT (SEQ ID NO: 26)
A solution (4 µL) containing the siRNAs for 25 pmol of the GFRα1 gene or 25 pmol of the non-silencing dsRNAs was mixed with 100 µL of a suspension of 1 million MCF 7 cells in Nucleofector Solution V (Amaxa biosystems), and the mixture was transfected by Nucleofector program P-020. The entire amount of this mixture was added to 4 mL of an MCF 7 cell culture medium and the cells were cultured for 24 hr and harvested. After the harvested cells were plated on a 96-well flat-bottomed tissue culture plate at a cell density of 3500 cells per well, a suspension of human recombinant GDNF (R&D systems) in 0.1% bovine serum albumin was administered. The cells were cultured for 3 more days, and a Cell-Counting Kit-8 solution (Wako Pure Chemical Industries, Ltd.) was added at 10 µL per well. The cells were stood in a 5% carbon dioxide gas stream at 37°C for 3 more hours, and the absorbance at 450 nm was measured to count the cells.
As a result, the cells administered with siRNAs against the GFRα1 gene showed suppression of an increase in the absorbance by administration of GDNF as compared to the cells administered with the non-silencing dsRNAs (Fig. 3). This result suggests that in the absence of the siRNA GFRα1 protein activated by GDNF induces cell growth in human breast cancer-derived cell line MCF 7, and the inhibition of the function of GFRα1 by siRNA and the like can suppress the growth of a cancer cells.

### [Example 4]

### Expression of RET protein in human breast cancer cell line

A lysate of human breast cancer-derived cell line MCF 7 cultured by the method of Example 1 was prepared, separated by SDS-polyacrylamide electrophoresis, and transferred onto a PVDF membrane. Using an anti-RET goat polyclonal antibody (R&D systems) as the primary antibody and an HRP (Horse Radish Peroxidase)-added anti-goat IgG antibody (SIGMA) as the secondary antibody, Western blot analysis was performed. As a result, a band was detected near the molecular weight of 170 kDa (Fig. 4a). Based on the size of the molecular weight, this band was considered to have derived from the RET protein, suggesting expression of the RET protein in MCF 7. Furthermore, flow cytometry analysis was performed using MCF 7 viable cells, the same antibody as the primary antibody and an AlexaFluor488-added anti-goat IgG antibody (Invitrogen) as the secondary antibody. As a result, a peak shift was observed when the anti-RET antibody was used (Fig. 4b). These results, including the above-mentioned Western blot analysis, suggest expression of the RET protein on the surface of the cellular membrane of MCF 7.

### [Example 5]

### Expression of GFRα1 protein in human breast cancer cell line

A lysate of human breast cancer-derived cell line MCF 7 cultured by the method of Example 1 was prepared, separated by SDS-polyacrylamide electrophoresis, and transferred onto a PVDF membrane. Using an anti-GFRα1 goat polyclonal antibody (R&D systems) as the primary antibody and an HRP-added anti-goat IgG antibody (SIGMA) as the secondary antibody, Western blot analysis was performed. As a result, a band was detected near the molecular weight of 55 kDa (Fig. 5a). Based on the size of the molecular weight, this band was considered to have derived from the GFRα1 protein, suggesting expression of the GFRα1 protein in MCF 7. Furthermore, flow cytometry analysis of MCF 7 was performed using MCF 7 viable cells, the same antibody as the primary antibody and an AlexaFluor488-added anti-goat IgG antibody (Invitrogen) as the secondary antibody. As a result, a peak shift was observed when the anti-GFRα1 antibody was used (Fig. 5b). These results, including the above-mentioned Western blot analysis, suggest expression of the GFRα1 protein on the surface of the cellular membrane of MCF 7.

### [Example 6]

### Phosphorylation of intracellular signal transduction protein of human breast cancer cell line by GDNF administration

Human breast cancer-derived cell line MCF 7 cultured by the method of Example 1 was plated on a 6-well flat-bottomed tissue culture plate at a cell density of 1.5 million cells in 100 µL per well. The cells were cultured in a 5% carbon dioxide gas stream at 37°C for one day, and a suspension of human recombinant GDNF (R&D systems) in 0.1% bovine serum albumin was administered at a final concentration of 10 ug/mL. Lysates were prepared after culturing for 0, 10, 20 and 30 min. A part of the lysate was analyzed by a PathScan Sandwich ELISA kit (Cell Signaling). As a result, induction of phosphorylation of ERK1/2, Akt, MEK1/2 due to the addition of GDNF was detected (Fig. 6a). Furthermore, a part of the lysate was separated by SDS-polyacrylamide electrophoresis, and transferred onto a PVDF membrane. Using an anti-phosphorylation ERK1/2 antibody, an anti-phosphorylation JNK antibody, an anti-phosphorylation p38-MAPK antibody, an anti-phosphorylation Akt antibody and an anti-Akt antibody (Cell Signaling) as primary antibodies and an HRP-added anti-rabbit IgG antibody (Cell Signaling) as the secondary antibody, Western blot analysis was performed. As a result, induction of phosphorylation of ERK1/2 and Akt due to the addition of GDNF was observed (Fig. 6b). These experiments have indicated that MCF 7 responds to the addition of GDNF and activates a protein in the intracellular signal transduction pathway.

### [Example 7]

### Enhancement of cell growth of human breast cancer cell line by rat GDNF administration

Human breast cancer-derived cell line MCF 7 cultured by the method of Example 1 was plated on a 96-well flat-bottomed tissue culture plate at a cell density of 3000 cells in 100 µL per well. After culture in a 5% carbon dioxide gas stream at 37°C for 1 day, a suspension of rat recombinant GDNF (R&D systems) in 0.1% bovine serum albumin was administered. After culture for 3 more days, a Cell-Counting Kit-8 solution (Wako Pure Chemical Industries, Ltd.) was added at 10 µL per well, and the cells were cultured in a 5% carbon dioxide gas stream at 37°C for 3 more hours. The absorbance at 450 nm was measured to count the cells. As a result, the cells administered with GDNF showed an about 40% increase in the absorbance as compared to the cells without administration (Fig. 7). This result shows that GDNF of rodents such as rat etc. induces cell growth of human breast cancer-derived cell line MCF 7 and a possibility that a tumor-bearing experiment can be performed using rodents.

### [Example 8]

### Expression of RET protein in human breast cancer tissue

A human breast cancer tissue section (SuperBioChip) was boiled in 10 mM citrate buffer (ph 6.0) for 15 min to activate an antigen. Using the anti-RET antibody (R&D systems) used in Example 4 as the primary antibody, an anti-goat Ig·biotin-labeled rabbit polyclonal antibody as the secondary antibody (DAKO) and an HRP-labeled streptavidin (DAKO) as the tertiary reactant, an immunohistological staining analysis was performed based on color development with DAB. As a result, a stained image was detected in breast cancer tissues, thus suggesting expression of RET protein in the breast cancer tissues (Fig. 8). In addition, the results of Example 9 using serial sections show a possibility that the RET protein and GFRα1 protein are expressed in the same cell.

### [Example 9]

### Expression of GFRα1 protein in human breast cancer tissue

A human breast cancer tissue section (SuperBioChip) was boiled in 10 mM citrate buffer (ph 6.0) for 15 min to activate an antigen. Using the anti-GFRα1 antibody (R&D systems) used in Example 5 as the primary antibody, an anti-goat Ig·biotin-labeled rabbit polyclonal antibody as the secondary antibody (DAKO) and an HRP-labeled streptavidin (DAKO) as the tertiary reactant, an immunohistological staining analysis was performed based on color development with DAB. As a result, a stained image was detected in breast cancer tissues, thus suggesting expression of GFRα1 protein in the breast cancer tissues (Fig. 9). In addition, the results of Example 8 using serial sections show a possibility that the RET protein and GFRα1 protein are expressed in the same cell.

### [Example 10]

### Inhibition of cell growth of human breast cancer cell line by anti-GDNF antibody administration

Human breast cancer-derived cell line MCF 7 cultured by the method of Example 1 was plated on a 96-well flat-bottomed tissue culture plate at a cell density of 3000 cells in 100 µL per well. After culture in a 5% carbon dioxide gas stream at 37°C for 1 day, a suspension of an anti-GDNF polyclonal antibody (R&D systems) or an anti-GDNF monoclonal antibody (R&D systems) in PBS was administered and human recombinant GDNF (R&D systems) was administered at a final concentration of 1 ng/mL. After culture for 3 more days, a Cell-Counting Kit-8 solution (Wako Pure Chemical Industries, Ltd.) was added at 10 µL per well, and the cells were cultured in a 5% carbon dioxide gas stream at 37°C for 3 more hours. The absorbance at 450 nm was measured to count the cells. As a result, the cells administered with anti-GDNF antibody showed an about 30% decrease in the absorbance as compared to the cells without administration (Fig. 10). This result suggests that GDNF induces cell growth in human breast cancer-derived cell line MCF 7 in the absence of the antibody, and the inhibition of the function of GDNF by the antibody and the like can supress the growth of a cancer cells.

### Industrial Applicability

An antibody against a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof or a salt thereof can be safely used as, for example, an agent for the prophylaxis or treatment of breast cancer and the like, an apoptosis promoter of cancer cells, an inhibitor of cancer cell growth and the like.

This application is based on patent application Nos. 2005-308589 filed in Japan (filing date: October 24, 2005) and 2006-045994 filed in Japan (filing date: February 22, 2006), the contents of which are incorporated in full herein by this reference.

## Claims

1. A pharmaceutical agent comprising a substance that inhibits the function of a protein comprising the same or substantially the same amino acid sequence as that shown in SEQ ID NO.: 1 or SEQ ID NO: 3, or a partial peptide thereof or a salt thereof.

2. The pharmaceutical agent of claim 1, wherein the substance is
(1) an antibody to a protein comprising the same or substantially the same amino acid sequence as that shown in SEQ ID NO: 1 or SEQ ID NO: 3, or a partial peptide thereof or a salt thereof,
(2) a low-molecular-weight compound that inhibits the function of a protein comprising the same or substantially the same amino acid sequence as that shown in SEQ ID NO: 1 or SEQ ID NO: 3, or a partial peptide thereof or a salt thereof, or a salt thereof,
(3) an antisense nucleic acid to a nucleic acid encoding a protein comprising the same or substantially the same amino acid sequence as that shown in SEQ ID NO: 1 or SEQ ID NO: 3, or a partial peptide thereof, or
(4) siRNA to RNA encoding a protein comprising the same or substantially the same amino acid sequence as that shown in SEQ ID NO: 1 or SEQ ID NO: 3, or a partial peptide thereof.

3. A pharmaceutical agent comprising an antibody to a protein comprising the same or substantially the same amino acid sequence as that shown in SEQ ID NO: 1 or SEQ ID NO: 3, or a partial peptide thereof or a salt thereof.

4. The pharmaceutical agent of claim 2 or 3, wherein the antibody is a monoclonal antibody.

5. The pharmaceutical agent of claims 1 to 4, which is an agent for the prophylaxis or treatment of cancer.

6. The pharmaceutical agent of claims 1 to 4, which is an agent for the prophylaxis or treatment of breast cancer.

7. The pharmaceutical agent of claims 1 to 4, which is a growth inhibitor of a cancer cell.

8. The pharmaceutical agent of claims 1 to 4, which is a growth inhibitor of a breast cancer cell.

9. A diagnostic reagent for breast cancer comprising an antibody to a protein comprising the same or substantially the same amino acid sequence as that shown in SEQ ID NO: 1 or SEQ ID NO: 3, or a partial peptide thereof or a salt thereof.

10. A method for the prophylaxis or treatment of cancer, comprising administering, to a mammal, an effective amount of a substance that inhibits the function of a protein comprising the same or substantially the same amino acid sequence as that shown in SEQ ID NO: 1 or SEQ ID NO: 3, or a partial peptide thereof or a salt thereof.

11. A method for the prophylaxis or treatment of breast cancer, comprising administering, to a mammal, an effective amount of a substance that inhibits the function of a protein comprising the same or substantially the same amino acid sequence as that shown in SEQ ID NO: 1 or SEQ ID NO: 3, or a partial peptide thereof or a salt thereof.

12. A method for inhibiting growth of a cancer cell, comprising administering, to a mammal, an effective amount of a substance that inhibits the function of a protein comprising the same or substantially the same amino acid sequence as that shown in SEQ ID NO: 1 or SEQ ID NO: 3, or a partial peptide thereof or a salt thereof.

13. A method for inhibiting growth of a breast cancer cell, comprising administering, to a mammal, an effective amount of a substance that inhibits the function of a protein comprising the same or substantially the same amino acid sequence as that shown in SEQ ID NO: 1 or SEQ ID NO: 3, or a partial peptide thereof or a salt thereof.

14. The method of claims 10 to 13, wherein the substance is
(1) an antibody to a protein comprising the same or substantially the same amino acid sequence as that shown in SEQ ID NO: 1 or SEQ ID NO: 3, or a partial peptide thereof or a salt thereof,
(2) a low-molecular-weight compound that inhibits the function of a protein comprising the same or substantially the same amino acid sequence as that shown in SEQ ID NO: 1 or SEQ ID NO: 3, or a partial peptide thereof or a salt thereof, or a salt thereof,
(3) an antisense nucleic acid to a nucleic acid encoding a protein comprising the same or substantially the same amino acid sequence as that shown in SEQ ID NO: 1 or SEQ ID NO: 3, or a partial peptide thereof, or
(4) siRNA to RNA encoding a protein comprising the same or substantially the same amino acid sequence as that shown in SEQ ID NO: 1 or SEQ ID NO: 3, or a partial peptide thereof.

15. The method of claims 10 to 13, wherein the substance is an antibody to a protein comprising the same or substantially the same amino acid sequence as that shown in SEQ ID NO: 1 or SEQ ID NO: 3, or a partial peptide thereof or a salt thereof.

16. The method of claim 15, wherein the antibody is a monoclonal antibody.

17. Use of a substance that inhibits the function of a protein comprising the same or substantially the same amino acid sequence as that shown in SEQ ID NO: 1 or SEQ ID NO: 3, or a partial peptide thereof or a salt thereof, for producing an agent for the prophylaxis or treatment of cancer.

18. Use of a substance that inhibits the function of a protein comprising the same or substantially the same amino acid sequence as that shown in SEQ ID NO: 1 or SEQ ID NO: 3, or a partial peptide thereof or a salt thereof, for producing an agent for the prophylaxis or treatment of breast cancer.

19. Use of a substance that inhibits the function of a protein comprising the same or substantially the same amino acid sequence as that shown in SEQ ID NO: 1 or SEQ ID NO: 3, or a partial peptide thereof or a salt thereof, for producing a growth inhibitor of a cancer cell.

20. Use of a substance that inhibits the function of a protein comprising the same or substantially the same amino acid sequence as that shown in SEQ ID NO: 1 or SEQ ID NO: 3, or a partial peptide thereof or a salt thereof, for producing a growth inhibitor of a breast cancer cell.

21. The use of claims 17 to 20, wherein the substance is an antibody to a protein comprising the same or substantially the same amino acid sequence as that shown in SEQ ID NO: 1 or SEQ ID NO: 3, or a partial peptide thereof or a salt thereof.

22. The use of claim 21, wherein the antibody is a monoclonal antibody.

23. A pharmaceutical agent comprising a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.

24. The pharmaceutical agent of claim 23, wherein the substance is
(1) an antibody to a protein comprising the same or substantially the same amino acid sequence as that shown in SEQ ID NO: 5, SEQ ID NO: 7 or SEQ ID NO: 9, or a partial peptide thereof or a salt thereof,
(2) a low-molecular-weight compound that inhibits the function of a protein comprising the same or substantially the same amino acid sequence as that shown in SEQ ID NO: 5, SEQ ID NO: 7 or SEQ ID NO: 9, or a partial peptide thereof or a salt thereof, or a salt thereof,
(3) an antisense nucleic acid to a nucleic acid encoding a protein comprising the same or substantially the same amino acid sequence as that shown in SEQ ID NO: 5, SEQ ID NO: 7 or SEQ ID NO: 9, or a partial peptide thereof, or.
(4) siRNA to RNA encoding a protein comprising the same or substantially the same amino acid sequence as that shown in SEQ ID NO: 5, SEQ ID NO: 7 or SEQ ID NO: 9, or a partial peptide thereof.

25. A pharmaceutical agent comprising an antibody to a protein comprising the same or substantially the same amino acid sequence as that shown in SEQ ID NO: 5, SEQ ID NO: 7 or SEQ ID NO: 9, or a partial peptide thereof or a salt thereof.

26. The pharmaceutical agent of claim 24 or 25, wherein the antibody is a monoclonal antibody.

27. The pharmaceutical agent of claims 23 to 26, which is an agent for the prophylaxis or treatment of cancer.

28. The pharmaceutical agent of claims 23 to 26, which is an agent for the prophylaxis or treatment of breast cancer.

29. The pharmaceutical agent of claims 23 to 26, which is a growth inhibitor of a cancer cell.

30. The pharmaceutical agent of claims 23 to 26, which is a growth inhibitor of a breast cancer cell.

31. A diagnostic reagent of breast cancer comprising an antibody to a protein comprising the same or substantially the same amino acid sequence as that shown in SEQ ID NO: 5, SEQ ID NO: 7 or SEQ ID NO: 9, or a partial peptide thereof or a salt thereof.

32. A diagnostic reagent of breast cancer comprising a nucleic acid encoding a protein comprising the same or substantially the same amino acid sequence as that shown in SEQ ID NO: 5, SEQ ID NO: 7 or SEQ ID NO: 9, or a partial peptide thereof.

33. A method for the prophylaxis or treatment of cancer, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.

34. A method for the prophylaxis or treatment of breast cancer, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.

35. A method for inhibiting growth of a cancer cell, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.

36. A method for inhibiting growth of a breast cancer cell, comprising administering, to a mammal, an effective amount of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof.

37. The method of claims 33 to 36, wherein the substance is
(1) an antibody to a protein comprising the same or substantially the same amino acid sequence as that shown in SEQ ID NO: 5, SEQ ID NO: 7 or SEQ ID NO:9, or a partial peptide thereof or a salt thereof,
(2) a low-molecular-weight compound that inhibits the function of a protein comprising the same or substantially the same amino acid sequence as that shown in SEQ ID NO: 5, SEQ ID NO: 7 or SEQ ID NO:9, or a partial peptide thereof or a salt thereof, or a salt thereof,
(3) an antisense nucleic acid to a nucleic acid encoding a protein comprising the same or substantially the same amino acid sequence as that shown in SEQ ID NO: 5, SEQ ID NO: 7 or SEQ ID NO:9, or a partial peptide thereof, or
(4) siRNA to RNA encoding a protein comprising the same or substantially the same amino acid sequence as that shown in SEQ ID NO: 5, SEQ ID NO: 7 or SEQ ID NO:9, or a partial peptide thereof.

38. The method of claims 33 to 36, wherein the substance is an antibody to a protein comprising the same or substantially the same amino acid sequence as that shown in SEQ ID NO: 5, SEQ ID NO: 7 or SEQ ID NO: 9, or a partial peptide thereof or a salt thereof.

39. The method of claim 38, wherein the antibody is a monoclonal antibody.

40. Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing an agent for the prophylaxis or treatment of cancer.

41. Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing an agent for the prophylaxis or treatment of breast cancer.

42. Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing a growth inhibitor of a cancer cell.

43. Use of a substance that inhibits the function of a protein comprising the same or substantially the same amino acid sequence as that shown in SEQ ID NO: 5, SEQ ID NO: 7 or SEQ ID NO: 9, or a partial peptide thereof or a salt thereof, for producing a growth inhibitor of a breast cancer cell.

44. The use of claims 40 to 43, wherein the substance is an antibody to a protein comprising the same or substantially the same amino acid sequence as that shown in SEQ ID NO: 5, SEQ ID NO: 7 or SEQ ID NO: 9, or a partial peptide thereof or a salt thereof.

45. The use of claim 44, wherein the antibody is a monoclonal antibody.

46. A pharmaceutical agent comprising a substance that inhibits the function of a protein comprising the same or substantially the same amino acid sequence as that shown in SEQ ID NO: 11 or SEQ ID NO: 13, or a partial peptide thereof or a salt thereof.

47. The pharmaceutical agent of claim 46, wherein the substance is
(1) an antibody to a protein comprising the same or substantially the same amino acid sequence as that shown in SEQ ID NO: 11 or SEQ ID NO: 13, or a partial peptide thereof or a salt thereof,
(2) a low-molecular-weight compound that inhibits the function of a protein comprising the same or substantially the same amino acid sequence as that shown in SEQ ID NO: 11 or SEQ ID NO: 13, or a partial peptide thereof or a salt thereof, or a salt thereof,
(3) an antisense nucleic acid to a nucleic acid encoding a protein comprising the same or substantially the same amino acid sequence as that shown in SEQ ID NO: 11 or SEQ ID NO: 13, or a partial peptide thereof, or
(4) siRNA to RNA encoding a protein comprising the same or substantially the same amino acid sequence as that shown in SEQ ID NO: 11 or SEQ ID NO: 13, or a partial peptide thereof.

48. A pharmaceutical agent comprising an antibody to a protein comprising the same or substantially the same amino acid sequence as that shown in SEQ ID NO: 11 or SEQ ID NO: 13, or a partial peptide thereof or a salt thereof.

49. The pharmaceutical agent of claim 47 or 48, wherein the antibody is a monoclonal antibody.

50. The pharmaceutical agent of claims 46 to 49, which is an agent for the prophylaxis or treatment of cancer.

51. The pharmaceutical agent of claims 46 to 49, which is an agent for the prophylaxis or treatment of breast cancer.

52. The pharmaceutical agent of claims 46 to 49, which is a growth inhibitor of a cancer cell.

53. The pharmaceutical agent of claims 46 - 49, which is a growth inhibitor of a breast cancer cell.

54. A diagnostic reagent of breast cancer comprising an antibody to a protein comprising the same or substantially the same amino acid sequence as that shown in SEQ ID NO: 11 or SEQ ID NO: 13, or a partial peptide thereof or a salt thereof.

55. A diagnostic reagent of breast cancer comprising a nucleic acid encoding a protein comprising the same or substantially the same amino acid sequence as that shown in SEQ ID NO: 11 or SEQ ID NO: 13, or a partial peptide thereof.

56. A method for the prophylaxis or treatment of cancer, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.

57. A method for the prophylaxis or treatment of breast cancer, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.

58. A method for inhibiting growth of a cancer cell, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.

59. A method for inhibiting growth of a breast cancer cell, comprising administering, to a mammal, an effective amount of a substance that inhibits the function of a protein comprising the same or substantially the same amino acid sequence as that shown in SEQ ID NO: 11 or SEQ ID NO: 13, or a partial peptide thereof or a salt thereof.

60. The method of claims 56 to 59, wherein the substance is
(1) an antibody to a protein comprising the same or substantially the same amino acid sequence as that shown in SEQ ID NO: 11 or SEQ ID NO: 13, or a partial peptide thereof or a salt thereof,
(2) a low-molecular-weight compound that inhibits the function of a protein comprising the same or substantially the same amino acid sequence as that shown in SEQ ID NO: 11 or SEQ ID NO: 13, or a partial peptide thereof or a salt thereof, or a salt thereof,
(3) an antisense nucleic acid to a nucleic acid encoding a protein comprising the same or substantially the same amino acid sequence as that shown in SEQ ID NO: 11 or SEQ ID NO: 13, or a partial peptide thereof, or
(4) siRNA to RNA encoding a protein comprising the same or substantially the same amino acid sequence as that shown in SEQ ID NO: 11 or SEQ ID NO: 13, or a partial peptide thereof.

61. The method of claims 56 to 59, wherein the substance is an antibody to a protein comprising the same or substantially the same amino acid sequence as that shown in SEQ ID NO: 11 or SEQ ID NO: 13, or a partial peptide thereof or a salt thereof.

62. The method of claim 61, wherein the antibody is a monoclonal antibody.

63. Use of a substance that inhibits the function of a protein comprising the same or substantially the same amino acid sequence as that shown in SEQ ID NO: 11 or SEQ ID NO: 13, or a partial peptide thereof or a salt thereof, for producing an agent for the prophylaxis or treatment of cancer.

64. Use of a substance that inhibits the function of a protein comprising the same or substantially the same amino acid sequence as that shown in SEQ ID NO: 11 or SEQ ID NO: 13, or a partial peptide thereof or a salt thereof, for producing an agent for the prophylaxis or treatment of breast cancer.

65. Use of a substance that inhibits the function of a protein comprising the same or substantially the same amino acid sequence as that shown in SEQ ID NO: 11 or SEQ ID NO: 13, or a partial peptide thereof or a salt thereof, for producing a growth inhibitor of a cancer cell.

66. Use of a substance that inhibits the function of a protein comprising the same or substantially the same amino acid sequence as that shown in SEQ ID NO: 11 or SEQ ID NO: 13, or a partial peptide thereof or a salt thereof, for producing a growth inhibitor of a breast cancer cell.

67. The use of claims 63 - 66, wherein the substance is an antibody to a protein comprising the same or substantially the same amino acid sequence as that shown in SEQ ID NO: 11 or SEQ ID NO: 13, or a partial peptide thereof or a salt thereof.

68. The use of claim 67, wherein the antibody is a monoclonal antibody.

69. A diagnostic reagent of breast cancer comprising a nucleic acid encoding a protein comprising the same or substantially the same amino acid sequence as that shown in SEQ ID NO: 1 or SEQ ID NO: 3, or a partial peptide thereof.

70. An agent for the prophylaxis or treatment of breast cancer comprising a substance that inhibits the function of GDNF, GFRα1 and RET.

71. The agent of claim 70, wherein the function of GDNF, GFRα1 and RET is an activation of RET by GDNF, or promotion of breast cancer cell growth by GDNF.

72. The agent of claim 70, wherein the substance is
(1) an antibody to a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof or a salt thereof,
(2) a low-molecular-weight compound that inhibits the function of GDNF, GFRα1 and RET, or a salt thereof,
(3) an antisense nucleic acid to a nucleic acid encoding a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof, or
(4) siRNA to RNA encoding a protein comprising the same or substantially the same amino acid sequence as that shown in SEQ ID NO: 1, SEQ ID NO: 3, SEQ ID NO: 5, SEQ ID NO: 7, SEQ ID NO: 9, SEQ ID NO: 11 or SEQ ID NO: 13, or a partial peptide thereof, and having a signal transduction inhibitory activity by GDNF, or a breast cancer cell growth inhibitory activity by GDNF.

73. The agent of claim 70, wherein the substance is
(1) an antibody to a protein comprising the same or substantially the same amino acid sequence as that shown in SEQ ID NO: 5, SEQ ID NO: 7 or SEQ ID NO: 9, or a partial peptide thereof or a salt thereof,
(2) an antibody to a protein comprising the same or substantially the same amino acid sequence as that shown in SEQ ID NO: 11 or SEQ ID NO: 13, or a partial peptide thereof or a salt thereof, or
(3) an antibody to a protein comprising the same or substantially the same amino acid sequence as that shown in SEQ ID NO: 1 or SEQ ID NO: 3, or a partial peptide thereof or a salt thereof.

74. The agent of claim 72 or 73, wherein the antibody is a monoclonal antibody.

75. The agent of claim 70, which is used for the treatment of breast cancer expressing GFRα1 protein and RET protein.

76. The agent of claim 70, which is used for the treatment of breast cancer expressing GDNF protein, GFRα1 protein and RET protein.

77. A method for the prophylaxis or treatment of breast cancer, comprising administering, to a mammal, an effective amount of a substance that inhibits the function of GDNF, GFRα1 and RET.

78. A method for the prophylaxis or treatment of breast cancer, comprising administering, to a patient with breast cancer expressing GFRα1 protein and RET protein, an effective amount of a substance that inhibits the function of GDNF, GFRα1 and RET.

79. A method for the prophylaxis or treatment of breast cancer, comprising administering, to a patient with breast cancer expressing GDNF protein, GFRα1 protein and RET protein, an effective amount of a substance that inhibits the function of GDNF, GFRα1 and RET.

80. The method of any of claims 77 to 79, wherein the substance is
(1) an antibody to a protein comprising the same or substantially the same amino acid sequence as that shown in SEQ ID NO: 5, SEQ ID NO: 7 or SEQ ID NO: 9, or a partial peptide thereof or a salt thereof,
(2) an antibody to a protein comprising the same or substantially the same amino acid sequence as that shown in SEQ ID NO: 11 or SEQ ID NO: 13, or a partial peptide or a salt thereof, or
(3) an antibody to a protein comprising the same or substantially the same amino acid sequence as that shown in SEQ ID NO: 1 or SEQ ID NO: 3, or a partial peptide thereof or a salt thereof.

81. Use of a substance that inhibits the function of GDNF, GFRα1 and RET, for producing an agent for the prophylaxis or treatment of breast cancer.

82. Use of a substance that inhibits the function of GDNF, GFRα1 and RET, for producing an agent for the prophylaxis or treatment of breast cancer to patients of breast cancer expressing GFRα1 protein and RET protein.

83. Use of a substance that inhibits the function of GDNF, GFRα1 and RET, for producing an agent for the prophylaxis or treatment of breast cancer to patients of breast cancer expressing GDNF protein, GFRα1 protein and RET protein.

84. The use of any of claims 81 to 83, wherein the substance is
(1) an antibody to a protein comprising the same or substantially the same amino acid sequence as that shown in SEQ ID NO: 5, SEQ ID NO: 7 or SEQ ID NO: 9, or a partial peptide or a salt thereof,
(2) an antibody to a protein comprising the same or substantially the same amino acid sequence as that shown in SEQ ID NO: 11 or SEQ ID NO: 13, or a partial peptide or a salt thereof, or
(3) an antibody to a protein comprising the same or substantially the same amino acid sequence as that shown in SEQ ID NO: 1 or SEQ ID NO: 3, or a partial peptide or a salt thereof.
